# FASCICULE DE BREVET EUROPEEN

(11) **EP 1 484 328 B1**
(45) Date de publication et mention de la délivrance du brevet: **02.05.2007**
(21) Numéro de dépôt: 04019136.3
(22) Date de dépôt: 14.09.2000
(51) Int. Cl.: C07D 401/04, A61K 31/47, A61P 31/04, C07D 409/14, C07D 417/14

(54) **Dérivés de la quinolyl propyl piperidine et leur utilisation en tant qu' agents antimicrobiens**
Chinolyl-propyl-piperidinderivate und deren Verwendung als antimikrobielle Wirkstoffe
Derivatives of quinolyl-propyl-piperidine and their use as antimicrobial agents

(30) Priorité: 17.09.1999 FR 9911679
(43) Date de publication de la demande: 08.12.2004
(62) Demande divisionnaire de: 00962637.5
(73) Titulaire: Aventis Pharma S.A., 92160 Antony (FR)
(72) Inventeur: Carry, Jean-Christophe, 94100 Saint Maur des Fosses (FR); Cheve, Michel, 91450 Soisy sur Seine (FR); El Ahmad, Youssef, 9400 Creteil (FR); Evers, Michel, 94510 La Queue en Brie (FR); Malleron, Jean-Luc, 91460 Marcoussis (FR); Mignani, Serge, 92290 Chatenay Malabry (FR); Tabart, Michel, 91290 La Norville (FR); Viviani, Fabrice, 95380 Louvres (FR)
(74) Mandataire: Hirsch & Associés

(56) Documents cités:
- WO-A-00/43383
- WO-A-99/37635

## Description

La présente invention concerne des dérivés de quinolyl propyl pipéridine de formule générale : 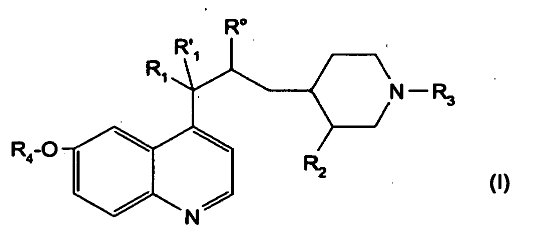 qui sont actifs comme antimicrobiens. L'invention concerne également leur préparation et les compositions les contenant.

Dans la demande de brevet WO 99/37635 ont été décrits des dérivés de quinolyl propyl pipéridine antimicrobiens, de formule générale : 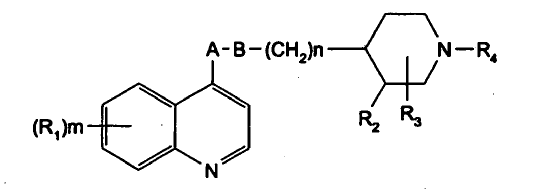 dans laquelle le radical R₁ est notamment alcoxy (C1-6), R₂ est hydrogène, R₃ est en position -2 ou -3 et représente alcoyle (C1-6) pouvant être éventuellement substitué par 1 à 3 substituants choisis parmi thiol, halogène, alcoylthio, trifluorométhyl, alcoyloxycarbonyle, alcoylcarbonyle, alcènyloxycarbonyle, alcènylcarbonyle, hydroxy éventuellement substitué par alcoyle ..., R₄ est un groupe -CH₂-R₅ pour lequel R₅ est selectionné parmi alcoyle hydroxyalcoyle, alcènyle, alcynyle, tétrahydrofuryle, phénylalcoyle éventuellement substitué, phénylalcényle éventuellement substitué, hétéroarylalcoyle éventuellement substitué, hétéroaroyle éventuellement substitué ..., n est 0 à 2, m est 1 ou 2 et A et B sont notamment oxygène, soufre, sulfinyle, sulfonyle, CR₆R₇ pour lequel R₆ et R₇ représentent H, thiol, alcoylthio, halo, trifluorométhyle, alcènyle, alcènylcarbonyle, hydroxy, amino ...

Dans la demande de brevet européen EP30044 ont été décrits des dérivés de quinoléine utiles comme cardiovasculaires, répondant à la formule générale : 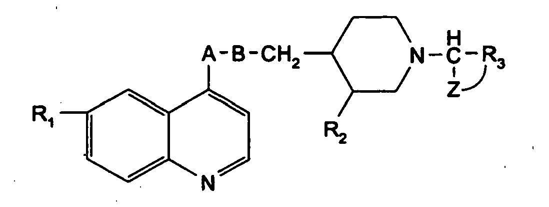 dans laquelle R₁ est notamment alcoyloxy, A-B est -CH₂-CH₂-, -CHOH-CH₂-, -CH₂-CHOH-, -CH₂-CO- ou -CO-CH₂-, R₁ est H, OH ou alcoyloxy, R₂ est éthyle ou vinyle, R₃ est notamment alcoyle, hydroxyalcoyle, cycloalcoyle, hydroxy, alcènyle, alcynyle, tétrahydrofuryle, phénylalcoyle, diphénylalcoyle éventuellement substitué, phénylalcényle éventuellement substitué, benzoyl ou benzoylalcoyle éventuellement substitué, hétéroaryle ou hétéroarylalcoyle éventuellement substitué et Z est H ou alcoyle ou forme avec R₃ un radical cycloalcoyle.

Il a maintenant été trouvé, et c'est ce qui fait l'objet de la présente invention, que les produits de formule générale (I) pour lesquels :
- R₁: est un atome d'hydrogène ou d'halogène, ou un radical hydroxy,
- R'₁: est un atome d'hydrogène, ou peut représenter halogène lorsque R₁ est également un atome d'halogène, et
- R°: est un atome d'hydrogène, ou bien
- R₁ et R°: forment ensemble une liaison et
- R'₁: est un atome d'hydrogène,
- R₂: représente un radical carboxy, carboxyméthyle, carboxy-2 éthyle, hydroxyméthyle, alcoyloxycarbonyle, alcoyloxycarbonylméthyle ou alcoyloxycarbonyl-2-éthyle (dont les parties alcoyle contiennent de 1 à 6 C),
- R₃: représente un radical propargyle substitué par un radical phényle pouvant lui même porter 1 à 4 s ubstituants [ choisis parmi halogène, hydroxy, alcoyle, alcoyloxy, trifluorométhyle, trifluorométhoxy, carboxy, alcoyloxycarbonyle, cyano ou amino], ou substitué par un radical cycloalcoyle contenant 3 à 7 chaînons ou substitué par un radical hétérocyclyle aromatique de 5 à 6 chaînons comprenant 1 à 4 hétéroatomes choisis parmi l'azote, l'oxygène et le soufre et éventuellement lui-même substitué [par halogène, hydroxy, alcoyle, alcoyloxy, trifluorométhyle, trifluorométhoxy, oxo, carboxy, alcoyloxycarbonyle, cyano ou amino],
- et R₄: représente un radical alcoyle (contenant de 1 à 6 atomes de carbone), alcényl-CH₂- ou alcynyl-CH₂- dont les parties alcényle ou alcynyle contiennent 2 à 6 atomes de carbone,
étant entendu que les radicaux et portions alcoyle sont en en chaîne droite ou ramifiée et contiennent, sauf mention spéciale, 1 à 4 atomes de carbone,
sous leurs formes diastéréoisomères ou leurs mélanges, ainsi que leurs sels, sont de puissants agents antibactériens.

Il est entendu que les radicaux et portions alcoyle sont en chaîne droite ou ramifiée et contiennent (sauf mention spéciale) 1 à 4 atomes de carbone, et que dans l'alternative où R₁ ou R'₁ repésentent un atome d'halogène ou lorsque R₃ porte un substituant halogène, celui-ci peut être choisi parmi fluor, chlore, brome ou iode, de préférence le fluor.

Dans la formule générale ci-dessus, lorsque R₃ porte un substituant hétérocyclyle aromatique, ce dernier peut être choisi (à titre non limitatif) parmi thiényle, furyle, pyrrolyle, imidazolyle, thiazolyle, oxazolyle, thiadiazolyle, oxadiazolyle, tétrazolyle, pyridyle, pyridazinyle, pyrazinyle, pyrimidinyle. Il est également entendu que dans la définition de R₃ le radical alcoyle substitué ne porte simultanément qu'un seul radical cyclique.

Selon l'invention, les produits de formule générale (I) peuvent être obtenus par condensation de la chaîne R₃ sur le dérivé de quinolyl propyl pipéridine de formule générale : 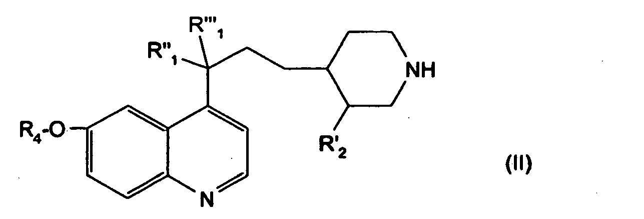 dans laquelle R₄ est défini comme précédemment, R"₁ et R"'₁ représentent des atomes d'hydrogène ou forment ensemble un radical oxo et R'₂ représente un radical carboxy, carboxyméthyle ou carboxy-2 éthyle protégés, ou un radical alcoyloxycarbonyle, alcoyloxycarbonylméthyle ou alcoyloxycarbonyl-2-éthyle, pour obtenir un dérivé de quinolyl propyl pipéridine de formule générale : 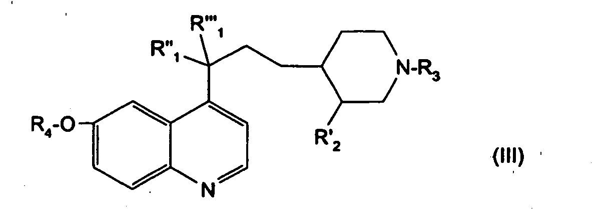 pour lequel R"₁, R"'₁, R'₂ et R₄ sont définis comme ci-dessus et R₃ est défini comme précédemment,
suivie le cas échéant de l'élimination du radical protecteur d'acide,
puis le cas échéant suivie de la réduction du radical oxo représenté par R"₁ et R'''₁ en un alcool pour lequel R₁ représente hydroxy puis éventuellement de l'halogénation si l'on veut obtenir un dérivé de quinolyl propyl pipéridine pour lequel R₁ est un atome d'halogène, et éventuellement de la déhydrohalogénation du dérivé halogéné correspondant, pour obtenir un dérivé de quinolyl propyl pipéridine pour lequel R₁ et R° forment ensemble une liaison, ou bien de la dihalogénation du produit de formule générale (III) pour lequel R"₁ et R"'₁ forment ensemble un radical oxo pour obtenir un dérivé de quinolyl propyl pipéridine pour lequel R₁ et R'₁ sont des atomes d'halogène,
et/ou le cas échéant suivie de la réduction de l'acide protégé sous forme d'un radical R'₂ en position -3 de la pipéridine, en un radical hydroxyméthyle et éventuellement de la transformation en un radical carboxyméthyle ou carboxy-2 éthyle selon les méthodes habituelles,
puis éventuellement suivie de l'élimination du radical protecteur d'acide et éventuellement de la transformation du produit obtenu en un sel.

La condensation de la chaîne R₃ sur la pipéridine s'effectue avantageusement par action d'un dérivé de formule générale :

R₃-X (IV)

dans laquelle R₃ est défini comme précédemment et X représente un atome d'halogène, un radical méthylsulfonyle, un radical trifluorométhylsulfonyle ou p.toluènesulfonyle, en opérant en milieu anhydre, de préférence inerte (azote ou argon par exemple) dans un solvant organique tel qu'un amide (diméthylformamide par exemple), une cétone (acétone par exemple) ou un nitrile (acétonitrile par exemple) en présence d'une base telle qu'une base organique azotée (par exemple triéthylamine) ou une base minérale (carbonate alcalin : carbonate de potassium par exemple) à une température comprise entre 20°C et la température de reflux du solvant.
De préférence, on fait agir un dérivé pour lequel X est un atome de brome ou d'iode.

Il est souvent préférable de condenser un halogénure de propargyle, puis de substituer la chaîne par un radical phényle, cycloalcoyle ou hétérocyclyle.
Dans cette alternative, l'addition de la chaîne propargylique s'effectue au moyen de bromure de propargyle, dans les conditions énoncées ci-dessus pour R₃ en présence ou non d'un iodure de métal alcalin comme par exemple l'iodure de potassium ou de sodium.
Lorsqu'il s'agit de la substitution par un radical phényle ou hétérocyclyle, la réaction s'effectue par action d'un halogénure dérivé du radical cyclique à substituer, en présence de triéthylamine, en milieu anhydre dans un solvant tel qu'un amide (diméthylformamide par exemple) ou un nitrile (acétonitrile par exemple) et en présence d'un sel de palladium comme par exemple le tétrakis triphénylphosphine palladium et d'iodure cuivreux, à une température comprise entre 20°C et la température de reflux du solvant.
Lorsqu'il s'agit de la substution par un groupement cycloalkyle, la réaction s'effectue par action d'un organolithien comme le n.butyllithium ou le tert-butyllithium sur le dérivé propargylique obtenu précédemment, en milieu anhydre dans un éther comme par exemple le tétrahydrofurane à une température comprise entre -78 et 0°C, puis action d'une cycloalcanone suivi de la désoxygénation de l'alcool intermédiaire selon les méthodes classiques.

Il est entendu que, lorsque les radicaux alcoyle représentés par R₃ portent des substituants carboxy ou amino, ces derniers sont préalablement protégés, puis libérés après la réaction. On opère selon les méthodes habituelles qui n'altèrent pas le reste de la molécule, notamment selon les méthodes décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973).

Le radical carboxy protégé représenté par R'₂ peut être choisi parmi les esters facilement hydrolysables. A titre d'exemple peuvent être cités les esters méthylique, benzylique, tertiobutylique, ou bien les esters de phénylpropyle ou de propargyle. Eventuellement la protection du radical carboxy s'effectue simultanément à la réaction. Dans ce cas le produit de formule générale (II) mis en oeuvre porte un radical R'₂ = carboxy.

La réduction du radical oxo en un alcool s'effectue selon les méthodes habituelles qui n'altèrent pas le reste de la molécule, notamment par action d'un agent réducteur comme par exemple un hydrure (borohydrure alcalin : borohydrure de sodium ou de potassium, triacétoxy-borohydrure de sodium, cyanoborohydrure de sodium par exemple, hydrure d'aluminium et de lithium ou hydrure de diisobutyl aluminium) en opérant de préférence dans sous atmosphère inerte, dans un solvant organique comme un alcool (méthanol, éthanol, isopropanol par exemple), ou un éther (par exemple tétrahydrofuranne) ou un solvant chloré (par exemple dichorométhane) à une température comprise entre 20°C et la température de reflux du solvant.

L'halogénation destinée à obtenir un dérivé de quinolyl propyl quinoléine pour lequel R'₁ est un atome d'halogène, à partir du dérivé pour lequel R'₁ est hydroxy, peut être mise en oeuvre en présence d'un trifluorure d'aminosoufre (trifluorure de diéthylamino soufre, trifluorure de bis(2-méthoxyéthyl)amino soufre (Deoxofluor^{®}), trifluorure de morpholino soufre par exemple) ou alternativement en présence de tétrafluorure de soufre, au moyen d'un réactif comme un halogénure de tétra alkylammonium, de tri alkyl benzylammonium ou de tri alkyl phénylammonium ou au moyen d'un halogénure de métal alcalin additionné éventuellement d'un éther couronne. La réaction de fluoration peut être également mise en oeuvre paraction d'un agent de fluoration comme un fluorure de soufre [par exemple trifluorure de morpholino soufre, tétrafluorure de soufre (J. Org. Chem., 40 3808 (1975)), trifluorure de diéthylamino soufre (Tetrahedron, 44, 2875 (1988)), trifluorure de bis(2-méthoxyéthyl)amino soufre (Deoxofluor^{®}). Alternativement la réaction de fluoration peut aussi s'effectuer au moyen d'un agent de fluoration comme l'hexafluoropropyl diéthylamine (JP 2 039 546) ou la N-(chloro-2 trifluoro-1,1,2 éthyl) diéthylamine.
Lorsque l'on met en oeuvre un halogénure de tétra alkylammonium, ce dernier peut être choisi, à titre d'exemple, parmi les halogénures de tétra méthylammonium, de tétra éthylammonium, de tétra propylammonium, de tétra butylammonium (tétra n-butylammonium par exemple), de tétra pentylammonium, de tétra cyclohexylammonium, de tri éthyl méthylammonium de tri butyl méthylammonium, ou de tri méthyl propylammonium.

On opère dans un solvant organique tel qu'un solvant chloré (par exemple dichlorométhane, dichloréthane, chloroforme) ou dans un éther (tétrahydrofurane, dioxanne par exemple) à une température comprise entre -78 et 40°C (de préférence entre 0 et 30°C). Il est avantageux d'opérer en milieu inerte (argon ou azote notamment).
Il est également possible d'opérer par traitement par un agent d'halogénation comme le chlorure de thionyle ou trichlorure de phosphore dans un solvant organique tel qu'un solvant chloré (dichlorométhane, chloroforme par exemple), à une température comprise entre 0°C et la température de reflux du mélange réactionnel.

La dihalogénation du produit de formule générale (III) pour lequel R"₁ et R"'₁ forment ensemble un radical oxo pour obtenir un dérivé de quinolyl propyl pipéridine pour lequel R₁ et R'₁ sont des atomes d'halogène peut être mise en oeuvre dans des conditions analogues à celles de l'halogénation ci-dessus.

La déhydrohalogénation du dérivé halogéné obtenu à partir du dérivé pour lequel R₁ est hydroxy peut être mise en oeuvre notamment par traitement par le diazabicyclo[5,4,0]undéc-7-ène dans un solvant organique aromatique (toluène par exemple), à une température comprise entre 20°C et la température de reflux du mélange réactionnel.

La réduction de l'acide protégé sous forme d'un radical R'₂ en position -3 de la pipéridine, en un radical hydroxyméthyle s'effectue selon les méthodes habituelles qui n'altèrent pas le reste de la molécule, notamment on opère par action d'un hydrure (hydrure d'aluminium et de lithium ou hydrure de diisobutyl aluminium par exemple) dans un solvant tel qu'un éther (tétrahydrofurane par exemple) à une température comprise entre 20 et 60°C.

La transformation du radical hydroxyméthyle en position - 3 de la pipéridine en un radical carboxyméthyle s'effectue selon les méthodes habituelles qui n'altèrent pas le reste de la molécule, notamment elle peut être mise en oeuvre par action d'un agent d'halogénation comme par exemple le chlorure de thionyle ou le trichlorure de phosphore ou le tribromure de phosphore puis d'un cyanure alcalin (cyanure de potassium ou cyanure de sodium par exemple) pour préparer le dérivé cyanométhyle correspondant, suivie de l'hydrolyse du nitrile.

L'halogénation peut être effectuée dans un solvant chloré (dichlorométhane, chloroforme par exemple), à une température comprise entre 0°C et la température de reflux du solvant.
La réaction du cyanure alcalin peut être mise en oeuvre dans un solvant comme le diméthylsulfoxyde, un amide (diméthylformamide par exemple), une cétone (acétone par exemple), un éther comme par exemple le tétrahydrofurane ou un alcool comme par exemple le méthanol ou l'éthanol, à une température comprise entre 20°C et la température de reflux du mélange réactionnel.
L'hydrolyse du nitrile s'effectue selon les méthodes classiques qui n'altèrent pas le reste de la molécule, notamment par action de l'acide chlorhydrique en milieu méthanolique, à une température comprise entre 20 et 70°C, suivi de la saponification de l'ester obtenu (par exemple par l'hydroxyde de sodium dans un mélange de dioxane et d'eau), ou bien directement par action de l'acide sulfurique aqueux à une température comprise entre 50 et 80°C.
La transformation du radical hydroxyméthyle en position - 3 de la pipéridine en un radical carboxy-2 éthyle s'effectue par exemple à partir du dérivé halogéné, préparé comme décrit ci-dessus, par condensation du sel de sodium du malonate de diéthyle suivie de l'hydrolyse acide en milieu aqueux du produit obtenu.

L'élimination le cas échéant du radical protecteur d'acide pour obtenir un dérivé de quinolyl propyl pipéridine pour lequel R₂ est un radical carboxy, s'effectue selon les méthodes habituelles, notamment par hydrolyse acide ou saponification de l'ester R'₂. Notamment on fait agir la soude en milieu hydroorganique, par exemple dans un alcool comme le méthanol ou un éther comme le dioxanne, à une température comprise entre 20°C et la température de reflux du mélange réactionnel. On peut également mettre en oeuvre l'hydrolyse en milieu chlorhydrique aqueux à une température comprise entre 20 et 100°C.

Le dérivé de quinolyl propyl pipéridine de formule générale (II) ou l'acide correspondant pour lequel R'₂ représente un radical carboxy, peut être préparé selon ou par analogie avec les méthodes décrites ci-après dans les exemples ou selon ou par analogie avec les méthodes décrites dans la demande de brevet européen EP 30044 ou dans la demande internationale WO 99/37635. Les intermédiaires des dérivés de quinolyl propyl pipéridine pour lesquels R₄ représente alcényl-CH₂O- ou alcynyl-CH₂O- peuvent être obtenus par analogie avec la préparation des intermédiaires pour lesquels R₄ est alcoyloxy, par action du dérivé halogéné correspondant sur le dérivé de quinoléine hydroxylé en position -6.

Le dérivé carboxy-2 éthyle protégé de formule générale (II) peut être obtenu selon ou par analogie avec la méthode décrite dans la demande internationale WO 99/37635 suivie de l'hydrolyse du nitrile et de l'esterification de l'acide ainsi obtenu, ou peut être préparé selon ou par analogie avec les méthodes décrites ci-après dans les exemples.

Il est entendu que les dérivés de formule générale (I), (II), (III), ou leurs intermédiaires de départ peuvent exister sous forme cis ou trans au niveau des substituants en position -3 et -4 de la pipéridine. Les dérivés de configuration trans peuvent être obtenus à partir des dérivés de configuration cis selon ou par analogie avec la méthode décrite dans la demande internationale WO 99/37635.

Les dérivés de quinolyl propyl pipéridine de formule générale (I) peuvent être purifiés le cas échéant par des méthodes physiques telles que la cristallisation ou la chromatographie.

Par ailleurs il est entendu que lorsque R'₁ est un atome d'hydrogène et R₁ est hydroxy ou halogène, il existe des formes diastéréoisomères et que les formes diastéréoisomères et leurs mélanges entrent aussi dans le cadre de la présente invention. Ces derniers peuvent être notamment séparés par chromatographie sur silice ou par Chromatographie Liquide Haute Performance (CLHP).

Les dérivés de quinolyl propyl pipéridine de formule générale (I) peuvent être transformés en sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Certains des dérivés de quinolyl propyl pipéridine de formule générale (I) portant un radical carboxy peuvent être transformés à l'état de sels métalliques ou en sels d'addition avec les bases azotées selon les méthodes connues en soi. Ces sels entrent également dans le cadre de la présente invention. Les sels peuvent être obtenus par action d'une base métallique (par exemple alcaline ou alcalino terreuse), de ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN-dibenzyléthylènediamine, diphénylènediamine, benzhydrylamine, quinine, choline, arginine, lysine, leucine, dibenzylamine).

Les dérivés de quinolyl propyl pipéridine selon l'invention sont des agents antibactériens particulièrement intéressants.

In vitro, sur germes gram positifs les dérivés de quinolyl propyl pipéridine selon l'invention se sont montrés actifs à des concentrations comprises entre 0,015 et 4 µg/ml sur *Staphylococcus aureus* A S5155 résistante à la méticilline, ainsi que la majorité d'entre eux à des concentrations comprises entre 0,06 et 8 µg/ml sur *Streptococcus pneumoniae* IP53146 et à des concentrations comprises entre 0,12 et 64 µg/ml sur *Enterococcus faecium* ATCC19434 ou H983401 et sur germes gram négatifs, ils se sont montrés actifs à des concentrations comprises entre 0,12 et 32 µg/ml sur *Moraxella catharrhalis* IPA152 ; in vivo, ils se sont montrés actifs sur les infections expérimentales de la souris à *Staphylococcus aureus* IP8203 à des doses comprises entre 10 et 150 mg/kg par voie sous cutanée (DC₅₀) et pour certains d'entre eux à des doses comprises entre 20 et 150 mg/kg par voie orale.

Enfin, les produits selon l'invention sont particulièrement intéressants du fait de leur faible toxicité. Aucun des produits n'a manifesté de toxicité à la dose de 100 mg/kg par voie sous cutanée chez la souris (2 administrations).

Parmi les produits selon l'invention, plus particulièrement intéressants sont les dérivés de quinolyl propyl quinoléine de formule générale (I) pour lesquels :
R₁ est un atome d'hydrogène ou d'halogène, ou un radical hydroxy,
   R'₁ est un atome d'hydrogène, et
   R° est un atome d'hydrogène, ou bien
R₁ et R° forment ensemble une liaison et
   R'₁ est un atome d'hydrogène,
R₂ représente un radical carboxy ou carboxyméthyle, et
   R₃ représente un radical propargyle substitué par un radical phényle pouvant lui même porter 1 à 3 substituants halogène, ou propargyle substitué par un radical hétérocyclyle aromatique de 5 à 6 chaînons comprenant 1 à 4 hétéroatomes choisis parmi l'azote, l'oxygène et le soufre, ou bien
R₂ représente un radical hydroxyméthyle, alcoyloxycarbonyle ou alcoyloxycarbonylméthyle (dont les portions alcoyle contiennent 1 à 6 atomes de carbone) et
R₃ représente un radical propargyle substitué par un radical hétérocyclyle aromatique de 5 à 6 chaînons comprenant 1 à 4 hétéroatomes choisis parmi l'azote, l'oxygène et le soufre,
   et R₄ représente un radical alcoyle (contenant 1 à 6 atomes de carbone),
les radicaux et portions alcoyle étant en en chaîne droite ou ramifiée, ainsi que les formes diastéréoisomères ou leurs mélanges, ainsi que leurs sels,
et parmi ces produits, plus spécialement préféré est le produit suivant :
- acide (3R,4R)-4-[3-hydroxy-3-(6-méthoxyquinolin-4-yl)propyl]-1-[3-(2,3,5-trifluorophényl)prop-2-ynyl]pipéridine-3-carboxylique.

Les produits cités dans les exemples sont particulièrement préférés ; les dérivés de quinolyl propyl pipéridine ci-après sont également des produits intéressants :
Acide (3R,4R)-4-[3-(6-méthoxyquinolin-4-yl)propyl]-1-[3-(4-fluoro-phényl)-prop-2-ynyl]-pipéridine-3-carboxylique
Acide (3R,4R)-4-[3-(6-méthoxyquinolin-4-yl)propyl]-1-[3-(2-chloro-phényl)-prop-2-ynyl]-pipéridine-3-carboxylique
Acide (3R,4R)-4-[3-(6-méthoxyquinolin-4-yl)propyl]-1-[3-(3-chloro-phényl)-prop-2-ynyl]-pipéridine-3-carboxylique
Acide (3R,4R)-4-[3-(6-méthoxyquinolin-4-yl)propyl]-1-[3-(4-chloro-phényl)-prop-2-ynyl]-pipéridine-3-carboxylique
Acide (3R,4R)-4-[3-(6-méthoxyquinolin-4-yl)propyl]-1-[3-(2-méthyl-phényl)-prop-2-ynyl]-pipéridine-3-carboxylique
Acide (3R,4R)-4-[3-(6-méthoxyquinolin-4-yl)propyl]-1-[3-(3-méthyl-phényl)-prop-2-ynyl]-pipéridine-3-carboxylique
Acide (3R,4R)-4-[3-(6-méthoxyquinolin-4-yl)propyl]-1-[3-(4-méthyl-phényl)-prop-2-ynyl]-pipéridine-3-carboxylique
Acide (3R,4R)-4-[3-(6-méthoxyquinolin-4-yl)propyl]-1-[3-(2-trifluorométhyl-phényl)-prop-2-ynyl]-pipéridine-3-carboxylique
Acide (3R,4R)-4-[3-(6-méthoxyquinolin-4-yl)propyl]-1-[3-(3-trifluorométhyl-phényl)-prop-2-ynyl]-pipéridine-3-carboxylique
Acide (3R,4R)-4-[3-(6-méthoxyquinolin-4-yl)propyl]-1-[3-(4-trifluorométhyl-phényl)-prop-2-ynyl]-pipéridine-3-carboxylique
Acide (3R,4R)-4-[3-(6-méthoxyquinolin-4-yl)propyl]-1-[3-(2-méthoxy-phényl)-prop-2-ynyl]-pipéridine-3-carboxylique
Acide (3R,4R)-4-[3-(6-méthoxyquinolin-4-yl)propyl]-1-[3-(3-méthoxy-phényl)-prop-2-ynyl]-pipéridine-3-carboxylique
Acide (3R,4R)-4-[3-(6-méthoxyquinolin-4-yl)propyl]-1-[3-(4-méthoxy-phényl)-prop-2-ynyl]-pipéridine-3-carboxylique
Acide (3R,4R)-4-[3-(6-méthoxyquinolin-4-yl)propyl]-1-[3-(3,4-difluoro-phényl)-prop-2-ynyl]-pipéridine-3-carboxylique
Acide (3R,4R)-4-[3-(6-méthoxyquinolin-4-yl)propyl]-1-[3-(2,4-difluoro-phényl)-prop-2-ynyl]-pipéridine-3-carboxylique
Acide (3R,4R)-4-[3-(6-méthoxyquinolin-4-yl)propyl]-1-[3-(3,4-dichloro-phényl)-prop-2-ynyl]-pipéridine-3-carboxylique
Acide (3R,4R)-4-[3-(6-méthoxyquinolin-4-yl)propyl]-1-[3-(2,3-dichloro-phényl)-prop-2-ynyl]-pipéridine-3-carboxylique
Acide (3R,4R)-4-[3-(6-méthoxyquinolin-4-yl)propyl]-1-[3-(2,4-dichloro-phényl)-prop-2-ynyl]-pipéridine-3-carboxylique
Acide (3R,4R)-4-[3-(6-méthoxyquinolin-4-yl)propyl]-1-[3-(2,4,6-trichloro-phényl)-prop-2-ynyl]-pipéridine-3-carboxylique
Acide (3R,4R)-4-[3-(6-méthoxyquinolin-4-yl)propyl]-1-[3-(3,5-dichloro-phényl)-prop-2-ynyl]-pipéridine-3-carboxylique
Acide (3R,4R)-4-[3-(6-méthoxyquinolin-4-yl)propyl]-1-[3-(4-chloro-3-fluoro-phényl)-prop-2-ynyl]-pipéridine-3-carboxylique
Acide (3R,4R)-4-[3-(6-méthoxyquinolin-4-yl)propyl]-1-[3-(3-chloro-4-fluoro-phényl)-prop-2-ynyl]-pipéridine-3-carboxylique
Acide (3R,4R)-4-[3-(6-méthoxyquinolin-4-yl)propyl]-1-[3-(2-chloro-4-fluoro-phényl)-prop-2-ynyl]-pipéridine-3-carboxylique
Acide (3R,4R)-4-[3-(6-méthoxyquinolin-4-yl)propyl]-1-[3-(3-chloro-5-fluoro-phényl)-prop-2-ynyl]-pipéridine-3-carboxylique
Acide (3R,4R)-4-[3-(6-méthoxyquinolin-4-yl)propyl]-1-[3-(4-chloro-2-fluoro-phényl)-prop-2-ynyl]-pipéridine-3-carboxylique
Acide (3R,4R)-4-[3-(6-méthoxyquinolin-4-yl)propyl]-1-[3-(3-fluoro-4-méthyl-phényl)-prop-2-ynyl]-pipéridine-3-carboxylique
Acide (3R,4R)-4-[3-(6-méthoxyquinolin-4-yl)propyl]-1-[3-(4-chloro-3-trifluorométhylphényl)-prop-2-ynyl]-pipéridine-3-carboxylique
Acide (3R,4R)-4-[3-(6-méthoxyquinolin-4-yl)propyl]-1-[3-(2-chloro-4-trifluorométhylphényl)-prop-2-ynyl]-pipéridine-3-carboxylique
Acide (3R,4R)-4-[3-(6-méthoxyquinolin-4-yl)propyl]-1-[3-(2-chloro-5-trifluorométhylphényl)-prop-2-ynyl]-pipéridine-3-carboxylique
Acide (3R,4R)-4-[3-(6-méthoxyquinolin-4-yl)propyl]-1-[3-(5-chloro-2-méthoxy-phényl)-prop-2-ynyl]-pipéridine-3-carboxylique
Acide (3R,4R)-4-[3-(6-méthoxyquinolin-4-yl)propyl]-1-[3-(3,5-bis-trifluorométhyl-phényl)-prop-2-ynyl]-pipéridine-3-carboxylique
Acide (3R,4R)-4-[3-(6-méthoxyquinolin-4-yl)propyl]-1-[3-(3,5-diméthyl-phényl)-prop-2-ynyl]-pipéridine-3-carboxylique
Acide (3R,4R)-4-[3-(6-méthoxyquinolin-4-yl)propyl]-1-[3-(2,4-dichloro-6-méthyl-phényl)-prop-2-ynyl]-pipéridine-3-carboxylique
Acide (3R,4R)-4-[3-(6-méthoxyquinolin-4-yl)propyl]-1-[3-(5-chloro-thien-2-yl)-prop-2-ynyl]-pipéridine-3-carboxylique
Acide (3R,4R)-4-[3-(6-méthoxyquinolin-4-yl)propyl]-1-[3-(3-chloro-thien-2-yl)-prop-2-ynyl]-pipéridine-3-carboxylique
Acide (3R,4R)-4-[3-(6-méthoxyquinolin-4-yl)propyl]-1-[3-(5-méthyl-thien-2-yl)-prop-2-ynyl]-pipéridine-3-carboxylique
Acide (3R,4R)-4-[3-(6-méthoxyquinolin-4-yl)propyl]-1-[3-(3-méthyl-thien-2-yl)-prop-2-ynyl]-pipéridine-3-carboxylique
Acide (3R,4R)-4-[3-(6-méthoxyquinolin-4-yl)propyl]-1-[3-(thien-3-yl)-prop-2-ynyl]-pipéridine-3-carboxylique
Acide (3R,4R)-4-[3-(6-méthoxyquinolin-4-yl)propyl]-1-[3-(1-méthyl-pyrrol-2-yl)-prop-2-ynyl]-pipéridine-3-carboxylique
Acide (3R,4R)-4-[3-(6-méthoxyquinolin-4-yl)propyl]-1-[3-(1-méthyl-pyrrol-3-yl)-prop-2-ynyl]-pipéridine-3-carboxylique
Acide (3R,4R)-4-[3-(6-méthoxyquinolin-4-yl)propyl]-1-[3-(1,3-thiazol-2-yl)-prop-2-ynyl]-pipéridine-3-carboxylique
Acide (3R,4R)-4-[3-(6-méthoxyquinolin-4-yl)propyl]-1-[3-(1,3-thiazol-4-yl)-prop-2-ynyl]-pipéridine-3-carboxylique
Acide (3R,4R)-4-[3-(6-méthoxyquinolin-4-yl)propyl]-1-[3-(1,3-thiazol-5-yl)-prop-2-ynyl]-pipéridine-3-carboxylique
Acide (3R,4R)-4-[3-(6-méthoxyquinolin-4-yl)propyl]-1-[3-(1-méthyl-imidazol-2-yl)-prop-2-ynyl]-pipéridine-3-carboxylique
Acide (3R,4R)-4-[3-(6-méthoxyquinolin-4-yl)propyl]-1-[3-(3-méthyl-imidazol-4-yl)-prop-2-ynyl]-pipéridine-3-carboxylique
Acide (3R,4R)-4-[3-(6-méthoxyquinolin-4-yl)propyl]-1-[3-(3-méthyl-pyrazol-4-yl)-prop-2-ynyl]-pipéridine-3-carboxylique
Acide (3R,4R)-4-[3-(6-méthoxyquinolin-4-yl)propyl]-1-[3-(oxazol-2-yl)-prop-2-ynyl]-pipéridine-3-carboxylique
Acide (3R,4R)-4-[3-(6-méthoxyquinolin-4-yl)propyl]-1-[3-(oxazol-4-yl)-prop-2-ynyl]-pipéridine-3-carboxylique
Acide (3R,4R)-4-[3-(6-méthoxyquinolin-4-yl)propyl]-1-[3-(oxazol-5-yl)-prop-2-ynyl]-pipéridine-3-carboxylique
Acide (3R,4R)-4-[3-(6-méthoxyquinolin-4-yl)propyl]-1-[3-(pyridin-2-yl)-prop-2-ynyl]-pipéridine-3-carboxylique
Acide (3R,4R)-4-[3-(6-méthoxyquinolin-4-yl)propyl]-1-[3-(pyridin-3-yl)-prop-2-ynyl]-pipéridine-3-carboxylique
Acide (3R,4R)-4-[3-(6-méthoxyquinolin-4-yl)propyl]-1-[3-(pyridin-4-yl)-prop-2-ynyl]-pipéridine-3-carboxylique
Acide (3R,4R)-4-[3-(6-méthoxyquinolin-4-yl)propyl]-1-[3-(pyrimidin-2-yl)-prop-2-ynyl]-pipéridine-3-carboxylique
Acide (3R,4R)-4-[3-(6-méthoxyquinolin-4-yl)propyl]-1-[3-(pyrimidin-4-yl)-prop-2-ynyl]-pipéridine-3-carboxylique
Acide (3R,4R)-4-[3-(6-méthoxyquinolin-4-yl)propyl]-1-[3-(pyrimidin-5-yl)-prop-2-ynyl]-pipéridine-3-carboxylique
Acide (3R,4R)-4-[3-(6-méthoxyquinolin-4-yl)propyl]-1-[3-(pyrazin-2-yl)-prop-2-ynyl]-pipéridine-3-carboxylique
Acide (3R,4R)-4-[3-(6-méthoxyquinolin-4-yl)propyl]-1-(3-(pyridazin-3-yl)-prop-2-ynyl]-pipéridine-3-carboxylique
Acide (3R,4R)-4-[3-(6-méthoxyquinolin-4-yl)propyl]-1-[3-(pyridazin-4-yl)-prop-2-ynyl]-pipéridine-3-carboxylique
Acide (3R,4R)-4-[3-(R,S)-hydroxy-3-(6-méthoxyquinolin-4-yl)propyl]-1-[3-phényl-prop-2-ynyl]-pipéridine-3-carboxylique
Acide (3R,4R)-4-[3-(R,S)-hydroxy-3-(6-méthoxyquinolin-4-yl)propyl]-1-[3-(2-fluorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(4-fluorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(2-chlorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(3-chlorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(4-chlorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(2-méthylphényl)-prop-2-ynyl]-pipéridine-3-carboxylique
Acide (3R,4R)-4-[3-(R,S)-hydroxy-3-(6-méthoxyquinolin-4-yl)propyl]-1-[3-(3-méthylphényl)-prop-2-ynyl]-pipéridine-3-carboxylique
Acide (3R,4R)-4-[3-(R,S)-hydroxy-3-(6-méthoxyquinolin-4-yl)propyl]-1-[3-(4-méthylphényl)-prop-2-ynyl]-pipéridine-3-carboxylique
Acide (3R,4R)-4-[3-(R,S)-hydroxy-3-(6-méthoxyquinolin-4-yl)propyl]-1-[3-(2-trifluorométhyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(3-trifluorométhyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(4-trifluorométhyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(2-méthoxyphényl)-prop-2-ynyl]-pipéridine-3-carboxylique
Acide (3R,4R)-4-[3-(R,S)-hydroxy-3-(6-méthoxyquinolin-4-yl)propyl]-1-[3-(3-méthoxyphényl)-prop-2-ynyl]-pipéridine-3-carboxylique
Acide (3R,4R)-4-[3-(R,S)-hydroxy-3-(6-méthoxyquinolin-4-yl)propyl]-1-[3-(4-méthoxy phényl)-prop-2-ynyl]-pipéridine-3-carboxylique
Acide (3R,4R)-4-[3-(R,S)-hydroxy-3-(6-méthoxyquinolin-4-yl)propyl]-1-[3-(3,4-difluorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(2,4-difluorophényl)-prop-2-ynyl]-pipéridine-3-carboxylique
Acide (3R,4R)-4-[3-(R,S)-hydroxy-3-(6-methoxyquinolin-4-yl)propyl]-1-[3-(3,4-dichlorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(2,3-dichlorophényl)-prop-2-ynyl]-pipéridine-3-carboxylique
Acide (3R,4R)-4-[3-R,S)-hydroxy-3-(6-méthoxyquinolin-4-yl)propyl]-1-[3-(2,4-dichlorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(2,4,6-trichlorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(3,5-dichlorophényl)-prop-2-ynyl]-pipéridine-3-carboxylique
Acide (3R,4R)-4-[3-(R,S)-hydroxy-3-(6-méthoxyquinolin-4-yl)propyl]-1-[3-(4-chloro-3-fluoro-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(3-chloro-4-fluoro-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(2-chloro-4-fluoro-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(3-chloro-5-fluoro-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(4-chloro-2-fluoro-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(3-fluoro-4-méthyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(4-chloro-3-trifluorométhyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(2-chloro-4-trifluorométhyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(2-chloro-5-trifluorométhyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(5-chloro-2-méthoxy-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(3,5-bis-trifluorométhyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(3,5-diméthylphényl)-prop-2-ynyl]-pipéridine-3-carboxylique
Acide (3R,4R)-4-[3-(R,S)-hydroxy-3-(6-méthoxyquinolin-4-yl)propyl]-1-[3-(2,4-dichloro-6-méthyl-phényl)-prop-2-ynyl]-pipéridine-3-carboxylique
Acide (3R,4R)-4-[3-(R,S)-hydroxy-3-(6-méthoxyquinolin-4-yl)propyl]-1-[3-(5-chloro-thien-2-yl)-prop-2-ynyl]-pipéridine-3-carboxylique
Acide (3R,4R)-4-[3-(R,S)-hydroxy-3-(6-méthoxyquinolin-4-yl)propyl]-1-[3-(3-chloro-thien-2-yl)-prop-2-ynyl]-pipéridine-3-carboxylique
Acide (3R,4R)-4-[3-(R,S)-hydroxy-3-(6-méthoxyqumolm-4-yl)propyl]-1-[3-(5-méthyl-thien-2-yl)-prop-2-ynyl]-pipéridine-3-carboxylique
Acide (3R,4R)-4-[3-(R,S)-hydroxy-3-(6-méthoxyquinolin-4-yl)propyl]-1-[3-(3-méthyl-thien-2-yl)-prop-2-ynyl]-pipéridine-3-carboxylique
Acide (3R,4R)-4-[3-(R,S)-hydroxy-3-(6-méthoxyqumolm-4-yl)propyl]-1-[3-(thien-3-yl)-prop-2-ynyl]-pipéridine-3-carboxylique
Acide (3R,4R)-4-[3-(R,S)-hydroxy-3-(6-méthoxyquinolin-4-yl)propyl]-1-[3-(1-méthylpyrrol-2-yl)-prop-2-ynyl]-pipéridine-3-carboxylique
Acide (3R,4R)-4-[3-(R,S)-hydroxy-3-(6-méthoxyquinolin-4-yl)propyl]-1-[3-(1-méthylpyrrol-3-yl)-prop-2-ynyl]-pipéridine-3-carboxylique
Acide (3R,4R)-4-[3-(R,S)-hydroxy-3-(6-méthoxyquinolin-4-yl)propyl]-1-[3-(1,3-thiazol-2-yl)-prop-2-ynyl]-pipéridine-3-carboxylique
Acide (3R,4R)-4-[3-(R,S)-hydroxy-3-(6-méthoxyquinolin-4-yl)propyl]-1-[3-(1,3-thiazol-4-yl)-prop-2-ynyl]-pipéridine-3-carboxylique
Acide (3R,4R)-4-[3-(R,S)-hydroxy-3-(6-méthoxyquinolin-4-yl)propyl]-1-[3-(1,3-thiazol-5-yl)-prop-2-ynyl]-pipéridine-3-carboxylique
Acide (3R,4R)-4-[3-(R,S)-hydroxy-3-(6-méthoxyquinolin-4-yl)propyl]-1-[3-(1-méthylimidazol-2-yl)-prop-2-ynyl]-pipéridine-3-carboxylique
Acide (3R,4R)-4-[3-(R,S)-hydroxy-3-(6-méthoxyquinolin-4-yl)propyl]-1-[3-(3-méthylimidazol-4-yl)-prop-2-ynyl]-pipéridine-3-carboxylique
Acide (3R,4R)-4-[3-(R,S)-hydroxy-3-(6-méthoxyquinolin-4-yl)propyl]-1-[3-(3-méthylpyrazol-4-yl)-prop-2-ynyl]-pipéridine-3-carboxylique
Acide (3R,4R)-4-[3-(R,S)-hydroxy-3-(6-méthoxyquinolin4-yl)propyl]-1-[3-(oxazol-2-yl)-prop-2-ynyl]-pipéridine-3-carboxylique
Acide(3R,4R)-4-[3-(R,S)-hydroxy-3-(6-méthoxyquinolin-4-yl)propyl]-1-[3-(oxazol-4-yl)-prop-2-ynyl]-pipéridine-3-carboxylique
Acide (3R,4R)-4-[3-R,S)-hydroxy-3-(6-méthoxyquinolin-4-yl)propyl]-1-[3-(oxazol-5-yl)-prop-2-ynyl]-pipéridine-3-carboxylique
Acide (3R,4R)-4-[3-(R,S)-hydroxy-3-(6-méthoxyquinolin-4-yl)propyl]-1-[3-(pyridin-2-yl)-prop-2-ynyl]-pipéridine-3-carboxylique
Acide (3R,4R)-4-[3-(R,S)-hydroxy-3-(6-méthoxyquinolin-4-yl)propyl]-1-[3-(pyridin-3-yl)-prop-2-ynyl]-pipéridine-3-carboxylique
Acide (3R,4R)-4-[3-(R,S)-hydroxy-3-(6-méthoxyquinolin-4-yl)propyl]-1-[3-(pyridin-4-yl)-prop-2-ynyl]-pipéridine-3-carboxylique
Acide (3R,4R)-4-[3-(R,S)-hydroxy-3-(6-méthoxyquinolin-4-yl)propyl]-1-[3-(pyrimidin-2-yl)-prop-2-ynyl]-pipéndine-3-carboxylique
Acide (3R,4R)-4-[3-(R,S)-hydroxy-3-(6-méthoxyquinolin-4-yl)propyl]-1-[3-(pyrimidin-4-yl)-prop-2-ynyl]-pipéridine-3-carboxylique
Acide (3R,4R)-4-[3-(R,S)-hydroxy-3-(6-méthoxyqumolin-4-yl)propyl]-1-[3-(pyrimidin-5-yl)-prop-2-ynyl]-pipéridine-3-carboxylique
Acide (3R,4R)-4-[3-(R,S)-hydroxy-3-(6-méthoxyquinolin-4-yl)propyl]-1-[3-(pyrazin-2-yl)-prop-2-ynyl]-pipéridine-3-carboxylique
Acide (3R,4R)-4-[3-(R,S)-hydroxy-3-(6-méthoxyquinolin-4-yl)propyl]-1-[3-(pyridazin-3-yl)-prop-2-ynyl]-pipéridine-3-carboxylique
Acide (3R,4R)-4-[3-(R,S)-hydroxy-3-(6-méthoxyquinolin-4-yl)propyl]-1-[3-(pyridazin-4-yl)-prop-2-ynyl]-pipéridine-3-carboxylique
Acide (3R,4R)-4-[3-(R,S)-fluoro-3-(6-méthoxyquinolin-4-yl)propyl]-1-[3-phényl-prop-2-ynyl]-pipéridine-3-carboxylique
Acide (3R,4R)-4-[3-(R,S)-fluoro-3-(6-méthoxyquinolin-4-yl)propyl]-1-[3-(2-fluoro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(4-fluoro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(2-chloro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(3-chloro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(4-chloro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(2-méthylphényl)-prop-2-ynyl]-pipéridine-3-carboxylique
Acide (3R,4R)-4-[3-(R,S)-fluoro-3-(6-méthoxyquinolin-4-yl)propyl]-1-[3-(3-méthylphényl)-prop-2-ynyl]-pipéridine-3-carboxylique
Acide (3R,4R)-4-[3-(R,S)-fluoro-3-(6-méthoxyquinolin-4-yl)propyl]-1-[3-(4-méthylphényl)-prop-2-ynyl]-pipéridine-3-carboxylique
Acide (3R,4R)-4-[3-(RS)-fluoro-3-(6-méthoxyquinolin-4-yl)propyl]-1-[3-(2-trifluorométhyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(3-trifluorométhyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(4-trifluorométhyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(2-méthoxyphényl)-prop-2-ynyl]-pipéridine-3-carboxylique
Acide (3R,4R)-4-[3-(R,S)-fluoro-3-(6-méthoxyquinolin-4-yl)propyl]-1-[3-(3-méthoxyphényl)-prop-2-ynyl]-pipéridine-3-carboxylique
Acide (3R,4R)-4-[3-(R,S)-fluoro-3-(6-méthoxyquinolin-4-yl)propyl]-1-[3-(4-méthoxyphényl)-prop-2-ynyl]-pipéridine-3-carboxylique
Acide (3R,4R)-4-[3-(R,S)-fluoro-3-(6-méthoxyquinolin-4-yl)propyl]-1-[3-(3,4-difluorophényl)-prop-2-ynyl]-pipéndine-3-carboxylique
Acide (3R,4R)-4-[3-(R,S)-fluoro-3-(6-méthoxyquinolin-4-yl)propyl]-1-[3-(2,4-difluorophényl)-prop-2-ynyl]-pipéridine-3-carboxylique
Acide (3R,4R)-4-[3-(R,S)-fluoro-3-(6-méthoxyquinolin-4-yl)propyl]-1-[3-(3,4-dichlorophényl)-prop-2-ynyl]-pipéridine-3-carboxylique
Acide (3R,4R)-4-[3-(R,S)-fluoro-3-(6-méthoxyquinolin-4-yl)propyl]-1-[3-(2,3-dichlorophényl)-prop-2-ynyl]-pipéridine-3-carboxylique
Acide (3R,4R)-4-[3-(R,S)-fluoro-3-(6-méthoxyquinolin-4-yl)propyl]-1-[3-(2,4-dichlorophényl)-prop-2-ynyl]-pipéridine-3-carboxylique
Acide (3R,4R)-4-[3-(R,S)-fluoro-3-(6-méthoxyquinolin-4-yl)propyl]-1-[3-(2,4,6-trichlorophényl)-prop-2-ynyl]-pipéridine-3-carboxylique
Acide (3R,4R)-4-[3-(R,S)-fluoro-3-(6-méthoxyquinolin-4-yl)propyl]-1-[3-(3,5-dichlorophényl)-prop-2-ynyl]-pipéridine-3-carboxylique
Acide (3R,4R)-4-[3-(R,S)-fluoro-3-(6-méthoxyquinolin-4-yl)propyl]-1-[3-(4-chloro-3-fluoro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(3-chloro-4-fluoro-phényl)-prop-2-ynyl]-pipéridine-3-carhoxylique
Acide (3R,4R)-4-[3-RS)-fluoro-3-(6-méthoxyquinolin-4-yl)propyl]-1-[3-(2-chloro-4-fluoro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(3-chloro-5-fluoro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(4-chloro-2-fluoro-phényl)-prop-2-ynyl]-pipéridine-3-carboxylique
Acide (3R,4R)-4-[3-(R,S)-fluoro-3-(6-méthoxyquinolin-4-yl)propyl]-1-[3-(3-fluoro-4-méthyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(4-chloro-3-trifluorométhyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(2-chloro-4-trifluorométhyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(2-chloro-5-trifluorométhyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(5-chloro-2-méthoxy-phényl)-prop-2-ynyl]-pipéridine-3-carboxylique
Acide (3R,4R)-4-[3-(R,S)-fluoro-3-(6-méthoxyquinolin-4-yl)propyl]-1-[3-(3,5-bis-trifluorométhyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(3,5-diméthylphényl)-prop-2-ynyl]-pipéridine-3-carboxylique
Acide (3R,4R)-4-[3-(R,S)-fluoro-3-(6-méthoxyquinolin-4-yl)propyl]-1-[3-(2,4-dichloro-6-méthyl-phényl)-prop-2-ynyl]-pipéridine-3-carboxylique
Acide (3R,4R)-4-[3-(R,S)-fluoro-3-(6-méthoxyquinolin-4-yl)propyl]-1-[3-(5-chloro-thien-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(3-chloro-thien-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(5-méthyl-thien-2-yl)-prop-2-ynyl]-pipéridine-3-carboxylique
Acide (3R,4R)-4-[3-(R,S)-fluoro-3-(6-méthoxyqumolin-4-yl)propyl]-1-[3-(3-méthyl-thien-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(thien-3-yl)-prop-2-ynyl]-pipéridine-3-carboxylique
Acide (3R,4R)-4-[3-(R,S)-fluoro-3-(6-méthoxyquinolin-4-yl)propyl]-1-[3-(1-méthyl-pyrrol-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(1-méthyl-pyrrol-3-yl)-prop-2-ynyl]-pipéridine-3-carboxylique
Acide (3R,4R)-4-[3-(R,S)-fluoro-3-(6-méthoxyquinolin-4-yl)propyl]-1-[3-(1,3-thiazol-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(1,3-thiazol-4-yl)-prop-2-ynyl]-pipéridine-3-carboxylique
Acide (3R,4R)-4-[3-(R,S)-fluoro-3-(6-méthoxyquinolin-4-yl)propyl]-1-[3-(1,3-thiazol-5-yl)-prop-2-ynyl]-pipéridine-3-carboxylique
Acide (3R,4R)-4-[3-(R,S)-fluoro-3-(6-méthoxyquinolin-4-yl)propyl]-1-[3-(1-méthylimidazol-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(3-méthylimidazol-4-yl)-prop-2-ynyl]-pipéridine-3-carboxylique
Acide (3R,4R)-4-[3-(R,S)-fluoro-3-(6-méthoxyquinolin-4-yl)propyl]-1-[3-(3-méthylpyrazol-4-yl)-prop-2-ynyl]-pipéridine-3-carboxylique
Acide (3R,4R)-4-[3-(R,S)-fluoro-3-(6-méthoxyquinolin-4-yl)propyl]-1-[3-(oxazol-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(oxazol-4-yl)-prop-2-ynyl]-pipéridine-3-carboxylique
Acide (3R,4R)-4-[3-(R,S)-fluoro-3-(6-méthoxyquinolin-4-yl)propyl]-1-[3-(oxazol-5-yl)-prop-2-ynyl]-pipéridine-3-carboxylique
Acide (3R,4R)-4-[3-(R,S)-auoro-3-(6-méthoxyquinolin-4-yl)propyl]-1-[3-(pyridin-2-yl)-prop-2-ynyl]-pipéridine-3-carboxylique
Acide (3R,4R)-4-[3-(RS)-fluoro-3-(6-méthoxyquinolin-4-yl)propyl]-1-[3-(pyridin-3-yl)-prop-2-ynyl]-pipéridine-3-carboxylique
Acide (3R,4R)-4-[3-(R,S)-fluoro-3-(6-méthoxyquinolin-4-yl)propyl]-1-[3-(pyridin-4-yl)-prop-2-ynyl]-pipéridine-3-carboxylique
Acide (3R,4R)-4-[3-(R,S)-fluoro-3-(6-méthoxyquinolin-4-yl)propyl]-1-[3-(pyrimidin-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(pyrimidin-4-yl)-prop-2-ynyl]-pipéridine-3-carboxylique
Acide (3R,4R)-4-[3-(R,S)-fluoro-3-(6-méthoxyquinolin-4-yl)propyl]-1-[3-(pyrimidin-5-yl)-prop-2-ynyl]-pipéridine-3-carboxylique
Acide (3R,4R)-4-[3-(R,S)-fluoro-3-(6-méthoxyquinolin-4-yl)propyl]-1-[3-(pyrazin-2-yl)-prop-2-ynyl]-pipéridine-3-carboxylique
Acide (3R,4R)-4-[3-(R,S)-fluoro-3-(6-méthoxyquinolin-4-yl)propyl]-1-[3-(pyridazin-3-yl)-prop-2-ynyl]-pipéridine-3-carboxylique
Acide (3R,4R)-4-[3-(R,S)-fluoro-3-(6-méthoxyquinolin-4-yl)propyl]-1-[3-(pyridazin-4-yl)-prop-2-ynyl]-pipéridine-3-carboxylique
Acide (3R,4R)-4-[3-(6-méthoxyquinolin-4-yl)propyl]-1-[3-phényl-prop-2-ynyl]-pipéridine-3-acétique
Acide (3R,4R)-4-[3-(6-méthoxyquinolin-4-yl)propyl]-1-[3-(2-fluoro-phényl)-prop-2-ynyl]-pipéridine-3-acétique
Acide (3R,4R)-4-[3-(6-méthoxyquinolin-4-yl)propyl]-1-[3-(3-fluoro-phényl)-prop-2-ynyl]-pipéridine-3-acétique
Acide (3R,4R)-4-[3-(6-méthoxyquinolin-4-yl)propyl]-1-[3-(4-fluoro-phényl)-prop-2-ynyl]-pipéridine-3-acétique
Acide (3R,4R)-4-[3-(6-méthoxyquinolin-4-yl)propyl]-1-[3-(2-chloro-phényl)-prop-2-ynyl]-pipéridine-3-acétique
Acide (3R,4R)-4-[3-(6-méthoxyquinolin-4-yl)propyl]-1-[3-(3-chloro-phényl)-prop-2-ynyl]-pipéridine-3-acétique
Acide (3R,4R)-4-[3-(6-méthoxyquinolin-4-yl)propyl]-1-[3-(4-chloro-phényl)-prop-2-ynyl]-pipéridine-3-acétique
Acide (3R,4R)-4-[3-(6-méthoxyquinolin-4-yl)propyl]-1-[3-(2-méthyl-phényl)-prop-2-ynyl]-pipéridine-3-acétique
Acide (3R,4R)-4-[3-(6-méthoxyquinolin-4-yl)propyl]-1-[3-(3-méthyl-phényl)-prop-2-ynyl]-pipéridine-3-acétique
Acide (3R,4R)-4-[3-(6-méthoxyquinolin-4-yl)propyl]-1-[3-(4-méthyl-phényl)-prop-2-ynyl]-pipéridine-3-acétique
Acide (3R,4R)-4-[3-(6-méthoxyquinolin-4-yl)propyl]-1-[3-(2-trifluorométhyl-phényl)-prop-2-ynyl]-pipéndine-3-acétique
Acide (3R,4R)-4-[3-(6-méthoxyquinolin-4-yl)propyl]-1-[3-(3-trifluorométhyl-phényl)-prop-2-ynyl]-pipéridine-3-acétique
Acide (3R,4R)-4-[3-(6-méthoxyquinolin-4-yl)propyl]-1-[3-(4-trifluorométhyl-phényl)-prop-2-ynyl]-pipéridine-3-acétique
Acide (3R,4R)-4-[3-(6-méthoxyquinolin-4-yl)propyl]-1-[3-(2-méthoxy-phényl)-prop-2-ynyl]-pipéridine-3-acétique
Acide (3R,4R)-4-[3-(6-méthoxyquinolin-4-yl)propyl]-1-[3-(3-méthoxy-phényl)-prop-2-ynyl]-pipéridine-3-acétique
Acide (3R,4R)-4-[3-(6-méthoxyquinolin-4-yl)propyl]-1-[3-(4-méthoxy-phényl)-prop-2-ynyl]-pipéridine-3-acétique
Acide (3R,4R)-4-[3-(6-méthoxyquinolin-4-yl)propyl]-1-[3-(3,4-difluoro-phényl)-prop-2-ynyl]-pipéridine-3-acétique
Acide (3R,4R)-4-[3-(6-méthoxyquinolin-4-yl)propyl]-1-[3-(2,4-difluoro-phényl)-prop-2-ynyl]-pipéridine-3-acétique
Acide (3R,4R)-4-[3-(6-méthoxyquinolin-4-yl)propyl]-1-[3-(3,4-dichloro-phényl)-prop-2-ynyl]-pipéridine-3-acétique
Acide (3R,4R)-4-[3-(6-méthoxyquinolin-4-yl)propyl]-1-[3-(2,3-dichloro-phényl)-prop-2-ynyl]-pipéridine-3-acétique
Acide (3R,4R)-4-[3-(6-méthoxyquinolin-4-yl)propyl]-1-[3-(2,4-dichloro-phényl)-prop-2-ynyl]-pipéridine-3-acétique
Acide (3R,4R)-4-[3-(6-méthoxyquinolin-4-yl)propyl]-1-[3-(2,4,6-trichloro-phényl)-prop-2-ynyl]-pipéridine-3-acétique
Acide (3R,4R)-4-[3-(6-méthoxyquinolin-4-yl)propyl]-1-[3-(3,5-dichloro-phényl)-prop-2-ynyl]-pipéridine-3-acétique
Acide (3R,4R)-4-[3-(6-méthoxyquinolin-4-yl)propyl]-1-[3-(4-chloro-3-fluoro-phényl)-prop-2-ynyl]-pipéridine-3-acétique
Acide (3R,4R)-4-[3-(6-méthoxyquinolin-4-yl)propyl]-1-[3-(3-chloro-4-fluoro-phényl)-prop-2-ynyl]-pipéridine-3-acétique
Acide (3R,4R)-4-[3-(6-méthoxyquinolin-4-yl)propyl]-1-[3-(2-chloro-4-fluoro-phényl)-prop-2-ynyl]-pipéridine-3-acétique
Acide (3R,4R)-4-[3-(6-méthoxyquinolin-4-yl)propyl]-1-[3-(3-chloro-5-fluoro-phényl)-prop-2-ynyl]-pipéridine-3-acétique
Acide (3R,4R)-4-[3-(6-méthoxyquinolin-4-yl)propyl]-1-[3-(4-chloro-2-fluoro-phényl)-prop-2-ynyl]-pipéridine-3-acétique
Acide (3R,4R)-4-[3-(6-méthoxyquinolin-4-yl)propyl]-1-[3-{3-fluoro-4-méthyl-phényl)-prop-2-ynyl]-pipéridine-3-acétique
Acide (3R,4R)-4-[3-(6-méthoxyquinolin-4-yl)propyl]-1-[3-(4-chloro-3-trifluorométhyl-phényl)-prop-2-ynyl]-pipéridine-3-acétique
Acide (3R,4R)-4-[3-(6-méthoxyquinolin-4-yl)propyl]-1-[3-(2-chloro-4-trifluorométhyl-phényl)-prop-2-ynyl]-pipéridine-3-acétique
Acide (3R,4R)-4-[3-(6-méthoxyquinolin-4-yl)propyl]-1-[3-(2-chloro-5-trifluorométhyl-phényl)-prop-2-ynyl]-pipéridine-3-acétique
Acide (3R,4R)-4-[3-(6-méthoxyquinolin-4-yl)propyl]-1-[3-(5-chloro-2-méthoxy-phényl)-prop-2-ynyl]-pipéridine-3-acétique
Acide (3R,4R)-4-[3-(6-méthoxyquinolin-4-yl)propyl]-1-[3-(3,5-bis-trifluorométhyl-phényl)-prop-2-ynyl]-pipéridine-3-acétique
Acide (3R,4R)-4-[3-(6-méthoxyquinolin-4-yl)propyl]-1-[3-(3,5-diméthyl-phényl)-prop-2-ynyl]-pipéridine-3-acétique
Acide (3R,4R)-4-[3-(6-méthoxyquinolin-4-yl)propyl]-1-[3-(2,4-dichloro-6-méthyl-phényl)-prop-2-ynyl]-pipéridine-3-acétique
Acide (3R,4R)-4-[3-(6-méthoxyquinolin-4-yl)propyl]-1-[3-(thien-2-yl)-prop-2-ynyl]-pipéridine-3-acétique
Acide (3R,4R)-4-[3-(6-méthoxyquinolin-4-yl)propyl]-1-[3-(5-chloro-thien-2-yl)-prop-2-ynyl]-pipéridine-3-acétique
Acide (3R,4R)-4-[3-(6-méthoxyquinolin-4-yl)propyl]-1-[3-(3-chloro-thien-2-yl)-prop-2-ynyl]-pipéridine-3-acétique
Acide (3R,4R)-4-[3-(6-méthoxyquinolin-4-yl)propyl]-1-[3-(5-méthyl-thien-2-yl)-prop-2-ynyl]-pipéridine-3-acétique
Acide (3R,4R)-4-[3-(6-méthoxyquinolin-4-yl)propyl]-1-[3-(3-méthyl-thien-2-yl)-prop-2-ynyl]-pipéridine-3-acétique
Acide (3R,4R)-4-[3-(6-méthoxyquinolin-4-yl)propyl]-1-[3-(thien-3-yl)-prop-2-ynyl]-pipéridine-3-acétique
Acide (3R,4R)-4-[3-(6-méthoxyquinolin-4-yl)propyl]-1-[3-(1-méthyl-pyrrol-2-yl)-prop-2-ynyl]-pipéridine-3-acétique
Acide (3R,4R)-4-[3-(6-méthoxyquinolin-4-yl)propyl]-1-[3-(1-méthyl-pyrrol-3-yl)-prop-2-ynyl]-pipéridine-3-acétique
Acide (3R,4R)-4-[3-(6-méthoxyquinolin-4-yl)propyl]-1-[3-(thiazol-2-yl)-prop-2-ynyl]-pipéridine-3-acétique
Acide (3R,4R)-4-[3-(6-méthoxyquinolin-4-yl)propyl]-1-[3-(thiazol-4-yl)-prop-2-ynyl]-pipéridine-3-acétique
Acide (3R,4R)-4-[3-(6-méthoxyquinolin-4-yl)propyl]-1-[3-(thiazol-5-yl)-prop-2-ynyl]-pipéridine-3-acétique
Acide (3R,4R)-4-[3-(6-méthoxyquinolin-4-yl)propyl]-1-[3-(1-méthyl-imidazol-2-yl)-prop-2-ynyl]-pipéridine-3-acétique
Acide (3R,4R)-4-[3-(6-méthoxyquinolin-4-yl)propyl]-1-[3-(3-méthyl-imidazol-4-yl)-prop-2-ynyl]-pipéridine-3-acétique
Acide (3R,4R)-4-[3-(6-méthoxyquinolin-4-yl)propyl]-1-[3-(3-méthyl-pyrazol-4-yl)-prop-2-ynyl]-pipéridine-3-acétique
Acide (3R,4R)-4-[3-(6-méthoxyquinolin-4-yl)propyl]-1-[3-(oxazol-2-yl)-prop-2-ynyl]-pipéridine-3-acétique
Acide (3R,4R)-4-[3-(6-méthoxyquinolin-4-yl)propyl]-1-[3-(oxazol-4-yl)-prop-2-ynyl]-pipéridine-3-acétique
Acide (3R,4R)-4-[3-(6-méthoxyquinolin-4-yl)propyl]-1-[3-(oxazol-5-yl)-prop-2-ynyl]-pipéridine-3-acétique
Acide (3R,4R)-4-[3-(6-méthoxyquinolin-4-yl)propyl]-1-[3-(pyridin-2-yl)-prop-2-ynyl]-pipéridine-3-acérique
Acide (3R,4R)-4-[3-(6-méthoxyquinolin-4-yl)propyl]-1-[3-(pyridin-3-yl)-prop-2-ynyl]-pipéridine-3-acétique
Acide (3R,4R)-4-[3-(6-méthoxyquinolin-4-yl)propyl]-1-[3-(pyridin-4-yl)-prop-2-ynyl]-pipéridine-3-acétique
Acide (3R,4R)-4-[3-(6-méthoxyquinolin-4-yl)propyl]-1-[3-(pyrimidin-2-yl)-prop-2-ynyl]-pipéridine-3-acétique
Acide (3R,4R)-4-(3-(6-méthoxyquinolin-4-yl)propyl]-1-[3-(pyrimidin-4-yl)-prop-2-ynyl]-pipéridine-3-acétique
Acide (3R,4R)-4-[3-(6-méthoxyquinolin-4-yl)propyl]-1-[3-(pyrimidin-5-yl)-prop-2-ynyl]-pipéridine-3-acétique
Acide (3R,4R)-4-[3-(6-méthoxyquinolin-4-yl)propyl]-1-[3-(pyrazin-2-yl)-prop-2-ynyl]-pipéridine-3-acétique
Acide (3R,4R)-4-[3-(6-méthoxyquinolin-4-yl)propyl]-1-[3-(pyridazin-3-yl)-prop-2-ynyl]-pipéridine-3-acétique
Acide (3R,4R)-4-[3-(6-méthoxyquinolin-4-yl)propyl]-1-[3-(pyridazin-4-yl)-prop-2-ynyl]-pipéridine-3-acétique
Acide (3R,4R)-4-[3-(R,S)-hydroxy-3-(6-méthoxyquinolin-4-yl)propyl]-1-[3-phényl-prop-2-ynyl]-pipéridine-3-acétique
Acide (3R,4R)-4-[3-(R,S)-hydroxy-3-(6-méthoxyquinolin-4-yl)propyl]-1-[3-(2-fluoro-phényl)-prop-2-ynyl]-pipéridine-3-acétique
Acide (3R,4R)-4-[3-(R,S)-hydroxy-3-(6-méthoxyquinolin-4-yl)propyl]-1-[3-(3-fluoro-phényl)-prop-2-ynyl]-pipéridine-3-acétique
Acide (3R,4R)-4-[3-(RS)-hydroxy-3-(6-méthoxyquinolin-4-yl)propyl]-1-[3-(4-fluorophényl)-prop-2-ynyl]-pipéridine-3-acétique
Acide (3R,4R)-4-[3-(R,S)-hydroxy-3-(6-méthoxyquinolin-4-yl)propyl]-1-[3-(2-chlorophényl)-prop-2-ynyl]-pipéridine-3-acétique
Acide (3R,4R)-4-[3-(R,S)-hydroxy-3-(6-méthoxyquinolin-4-yl)propyl]-1-[3-(3-chlorophényl)-prop-2-ynyl]-pipéridine-3-acétique
Acide (3R,4R)-4-[3-(R,S)-hydroxy-3-(6-méthoxyquinolin-4-yl)propyl]-1-[3-(4-chlorophényl)-prop-2-ynyl]-pipéridine-3-acétique
Acide (3R,4R)-4-[3-(R,S)-hydroxy-3-(6-méthoxyquinolin-4-yl)propyl]-1-[3-(2-méthylphényl)-prop-2-ynyl]-pipéridine-3-acétique
Acide (3R,4R)-4-[3-(R,S)-hydroxy-3-(6-méthoxyquinolin-4-yl)propyl]-1-[3-(3-méthylphényl)-prop-2-ynyl]-pipéridine-3-acétique
Acide (3R,4R)-4-[3-(R,S)-hydroxy-3-(6-méthoxyquinolin-4-yl)propyl]-1-[3-(4-méthylphényl)-prop-2-ynyl]-pipéridine-3-acétique
Acide (3R,4R)-4-[3-(R,S)-hydroxy-3-(6-méthoxyquinolin-4-yl)propyl]-1-[3-(2-trifluorométhyl-phényl)-prop-2-ynyl]-pipéridine-3-acétique
Acide (3R,4R)-4-[3-(R,S)-hydroxy-3-(6-méthoxyquinolin-4-yl)propyl]-1-[3-(3-trifluorométhyl-phényl)-prop-2-ynyl]-pipéridine-3-acétique
Acide (3R,4R)-4-[3-(R,S)-hydroxy-3-(6-méthoxyquinolin-4-yl)propyl]-1-[3-(4-trifluorométhyl-phényl)-prop-2-ynyl]-pipéridine-3-acétique
Acide (3R,4R)-4-[3-(R,S)-hydroxy-3-(6-méthoxyquinolin-4-yl)propyl]-1-[3-(2-méthoxyphényl)-prop-2-ynyl]-pipéridine-3-acétique
Acide (3R,4R)-4-[3-(R,S)-hydroxy-3-(6-méthoxyquinolin-4-yl)propyl]-1-[3-(3-méthoxyphényl)-prop-2-ynyl]-pipéridine-3-acétique
Acide (3R,4R)-4-[3-(R,S)-hydroxy-3-(6-méthoxyquinolin-4-yl)propyl]-1-[3-(4-méthoxyphényl)-prop-2-ynyl]-pipéridine-3-acétique
Acide (3R,4R)-4-[3-(R,S)-hydroxy-3-(6-méthoxyquinolin-4-yl)propyl]-1-[3-(3,4-difluorophényl)-prop-2-ynyl]-pipéridine-3-acétique
Acide (3R,4R)-4-[3-(R,S)-hydroxy-3-(6-méthoxyquinolin-4-yl)propyl]-1-[3-(2,4-difluorophényl)-prop-2-ynyl]-pipéridine-3-acétique
Acide (3R,4R)-4-[3-(R,S)-hydroxy-3-(6-méthoxyquinolin-4-yl)propyl]-1-[3-(3,4-dichlorophényl)-prop-2-ynyl]-pipéridine-3-acétique
Acide (3R,4R)-4-[3-(R,S)-hydroxy-3-(6-méthoxyquinolin-4-yl)propyl]-1-[3-(2,3-dichlorophényl)-prop-2-ynyl]-pipéridine-3-acétique
Acide (3R,4R)-4-[3-(R,S)-hydroxy-3-(6-méthoxyqumolin-4-yl)propyl]-1-[3-(2,4-dichlorophényl)-prop-2-ynyl]-pipéridine-3-acétique
Acide (3R,4R)-4-[3-(R,S)-hydroxy-3-(6-méthoxyquinolin-4-yl)propyl]-1-[3-(2,4,6-trichlorophényl)-prop-2-ynyl]-pipéridine-3-acétique
Acide (3R,4R)-4-[3-(R,S)-hydroxy-3-(6-méthoxyquinolin-4-yl)propyl]-1-[3-(3,5-dichlorophényl)-prop-2-ynyl]-pipéridine-3-acétique
Acide (3R,4R)-4-[3-(R,S)-hydroxy-3-(6-méthoxyquinolin-4-yl)propyl]-1-[3-(4-chloro-3-fluoro-phényl)-prop-2-ynyl]-pipéridine-3-acétique
Acide (3R,4R)-4-[3-(R,S)-hydroxy-3-(6-méthoxyquinolin-4-yl)propyl]-1-[3-(3-chloro-4-fluoro-phényl)-prop-2-ynyl]-pipéridine-3-acétique
Acide (3R,4R)-4-[3-(R,S)-hydroxy-3-(6-méthoxyquinolin-4-yl)propyl]-1-[3-(2-chloro-4-fluoro-phényl)-prop-2-ynyl]-pipéridine-3-acétique
Acide (3R,4R)-4-[3-(R,S)-hydroxy-3-(6-méthoxyquinolin-4-yl)propyl]-1-[3-(3-chloro-5-fluoro-phényl)-prop-2-ynyl]-pipéridine-3-acétique
Acide (3R,4R)-4-[3-(R,S)-hydroxy-3-(6-méthoxyquinolin-4-yl)propyl]-1-[3-(4-chloro-2-fluoro-phényl)-prop-2-ynyl]-pipéridine-3-acétique
Acide (3R,4R)-4-[3-(R,S)-hydroxy-3-(6-méthoxyqumolin-4-yl)propyl]-1-[3-(3-fluoro-4-méthyl-phényl)-prop-2-ynyl]-pipéridine-3-acétique
Acide (3R,4R)-4-[3-(R,S)-hydroxy-3-(6-méthoxyquinolin-4-yl)propyl]-1-[3-(4-chloro-3-trifluorométhyl-phényl)-prop-2-ynyl]-pipéridine-3-acétique
Acide (3R,4R)-4-[3-(R,S)-hydroxy-3-(6-méthoxyquinolin-4-yl)propyl]-1-[3-(2-chloro-4-trifluorométhyl-phényl)-prop-2-ynyl]-pipéridine-3-acérique
Acide (3R,4R)-4-[3-(R,S)-hydroxy 3-(6-méthoxyquinolin-4-yl)propyl]-1-[3-(2-chloro-5-trifluorométhyl-phényl)-prop-2-ynyl]-pipéridine-3-acétique
Acide (3R,4R)-4-[3-(R,S)-hydroxy-3-(6-méthoxyquinolin-4-yl)propyl]-1-[3-(5-chloro-2-méthoxy-phényl)-prop-2-ynyl]-pipéridine-3-acétique
Acide (3R,4R)-4-[3-(R,S)-hydroxy-3-(6-méthoxyquinolin-4-yl)propyl]-1-[3-(3,5-bis-trifluoro-méthyl-phényl)-prop-2-ynyl]-pipéridine-3-acétique
Acide (3R,4R)-4-[3-(R,S)-hydroxy-3-(6-méthoxyquinolin-4-yl)propyl]-1-[3-(3,5-diméthylphényl)-prop-2-ynyl]-pipéridine-3-acétique
Acide (3R,4R)-4-[3-(R,S)-hydroxy-3-(6-méthoxyquinolin-4-yl)propyl]-1-[3-(2,4-dichloro-6-méthyl-phényl)-prop-2-ynyl]-pipéridine-3-acétique
Acide (3R,4R)-4-[3-(R,S)-hydroxy-3-(6-méthoxyquinolin-4-yl)propyl]-1-[3-(thien-2-yl)-prop-2-ynyl]-pipéridine-3-acétique
Acide (3R,4R)-4-[3-(R,S)-hydroxy-3-(6-méthoxyquinolin-4-yl)propyl]-1-[3-(5-chloro-thien-2-yl)-prop-2-ynyl]-pipéridine-3-acétique
Acide (3R,4R)-4-[3-(R,S)-hydroxy-3-(6-méthoxyquinolin-4-yl)propyl]-1-[3-(3-chloro-thien-2-yl)-prop-2-ynyl]-pipéridine-3-acétique
Acide (3R,4R)-4-[3-(R,S)-hydroxy-3-(6-méthoxyquinolin-4-yl)propyl]-1-[3-(5-méthyl-thien-2-yl)-prop-2-ynyl]-pipéridine-3-acétique
Acide (3R,4R)-4-[3-(R,S)-hydroxy-3-(6-méthoxyquinolin-4-yl)propyl]-1-[3-(3-méthyl-thien-2-yl)-prop-2-ynyl]-pipéridine-3-acétique
Acide (3R,4R)-4-[3-(R,S)-hydroxy-3-(6-méthoxyquinolin-4-yl)propyl]-1-[3-(thien-3-yl)-prop-2-ynyl]-pipéridine-3-acétique
Acide (3R,4R)-4-[3-(R,S)-hydroxy-3-(6-méthoxyquinolin-4-yl)propyl]-1-[3-(1-méthylpyrrol-2-yl)-prop-2-ynyl]-pipéridine-3-acétique
Acide (3R,4R)-4-[3-(R,S)-hydroxy-3-(6-méthoxyquinolin-4-yl)propyl]-1-[3-(1-méthylpyrrol-3-yl)-prop-2-ynyl]-pipéridine-3-acétique
Acide (3R,4R)-4-[3-(R,S)-hydroxy-3-(6-méthoxyquinolin-4-yl)propyl]-1-[3-(thiazol-2-yl)-prop-2-ynyl]-pipéridine-3-acérique
Acide (3R,4R)-4-[3-(R,S)-hydroxy-3-(6-méthoxyquinolin-4-yl)propyl]-1-[3-(thiazol-4-yl)-prop-2-ynyl]-pipéridine-3-acétique
Acide (3R,4R)-4-[3-(R,S)-hydroxy-3-(6-méthoxyquinolin-4-yl)propyl]-1-[3-(thiazol-5-yl)-prop-2-ynyl]-pipéridine-3-acétique
Acide (3R,4R)-4-[3-(R,S)-hydroxy-3-(6-méthoxyquinolin-4-yl)propyl]-1-[3-(1-méthylimidazol-2-yl)-prop-2-ynyl]-pipéridine-3-acétique
Acide (3R,4R)-4-[3-(R,S)-hydroxy-3-(6-méthoxyquinolin-4-yl)propyl]-1-[3-(3-méthylimidazol-4-yl)-prop-2-ynyl]-pipéridine-3-acétique
Acide (3R,4R)-4-[3-(R,S)-hydroxy-3-(6-méthoxyquinolin-4-yl)propyl]-1-[3-(3-méthylpyrazol-4-yl)-prop-2-ynyl]-pipéridine-3-acétique
Acide (3R,4R)-4-[3-(R,S)-hydroxy-3-(6-méthoxyquinolin-4-yl)propyl]-1-[3-(oxazol-2-yl)-prop-2-ynyl]-pipéridine-3-acétique
Acide (3R,4R)-4-[3-(R,S)-hydroxy-3-(6-méthoxyquinolin-4-yl)propyl]-1-[3-(oxazol-4-yl)-prop-2-ynyl]-pipéridine-3-acétique
Acide (3R,4R)-4-[3-(RS)-hydroxy-3-(6-méthoxyquinolin-4-yl)propyl]-1-[3-(oxazol-5-yl)-prop-2-ynyl]-pipéndine-3-acétique
Acide (3R,4R)-4-[3-(R,S)-hydroxy-3-(6-méthoxyquinolin-4-yl)propyl]-1-[3-(pyridin-2-yl)-prop-2-ynyl]-pipéridine-3-acétique
Acide (3R,4R)-4-[3-(R,S)-hydroxy-3-(6-méthoxyquinolin-4-yl)propyl]-1-[3-(pyridin-3-yl)-prop-2-ypyl]-pipéridine-3-acétique
Acide (3R,4R)-4-[3-(R,S)-hydroxy-3-(6-méthoxyquinolin-4-yl)propyl]-1-[3-(pyridin-4-yl)-prop-2-ynyl]-pipéridine-3-acétique
Acide (3R,4R)-4-[3-(R,S)-hydroxy-3-(6-méthoxyquinolin-4-yl)propyl]-1-[3-(pyrimidin-2-yl)-prop-2-ynyl]-pipéndine-3-acétique
Acide (3R,4R)-4-[3-(R,S)-hydroxy-3-(6-méthoxyquinolin-4-yl)propyl]-1-[3-(pyrimidm-4-yl)-prop-2-ynyl]-pipéridine-3-acétique
Acide (3R,4R)-4-[3-(R,S)-hydroxy-3-(6-méthoxyquinolin-4-yl)propyl]-1-[3-(pyrimidin-5-yl)-prop-2-ynyl]-pipéridine-3-acétique
Acide (3R,4R)-4-[3-(R,S)-hydroxy-3-(6-méthoxyquinolin-4-yl)propyl]-1-[3-(pyrazin-2-yl)-prop-2-ynyl]-pipéridine-3-acétique
Acide (3R,4R)-4-[3-(R,S)-hydroxy-3-(6-méthoxyquinolin-4-yl)propyl]-1-[3-(pyridazin-3-yl)-prop-2-ynyl]-pipéridine-3-acétique
Acide (3R,4R)-4-[3-(R,S)-hydroxy-3-(6-méthoxyquinolin-4-yl)propyl]-1-[3-(pyridazin-4-yl)-prop-2-ynyl]-pipéridine-3-acétique
Acide (3R,4R)-4-[3-(R,S)-fluoro-3-(6-méthoxyquinolin-4-yl)propyl]-1-[3-phényl-prop-2-ynyl]-pipéridine-3-acétique
Acide (3R,4R)-4-[3-(R,S)-fluoro-3-(6-méthoxyquinolin-4-yl)propyl]-1-[3-(2-fluoro-phényl)-prop-2-ynyl]-pipéridine-3-acétique
Acide (3R,4R)-4-[3-(R,S)-fluoro-3-(6-méthoxyquinolin-4-yl)propyl]-1-[3-(3-fluoro-phényl)-prop-2-ynyl]-pipéridine-3-acétique
Acide (3R,4R)-4-[3-(R,S)-fluoro-3-(6-méthoxyquinolin-4-yl)propyl]-1-[3-(4-fluoro-phényl)-prop-2-ynyl]-pipéridine-3-acétique
Acide (3R,4R)-4-[3-(R,S)-fluoro-3-(6-méthoxyquinolin-4-yl)propyl]-1-[3-(2-chloro-phényl)-prop-2-ynyl]-pipéridine-3-acétique
Acide (3R,4R)-4-[3-(R,S)-fluoro-3-(6-méthoxyquinolin-4-yl)propyl]-1-[3-(3-chloro-phényl)-prop-2-ynyl]-pipéridine-3-acétique
Acide (3R,4R)-4-[3-(R,S)-fluoro-3-(6-méthoxyquinolin-4-yl)propyl]-1-[3-(4-chloro-phényl)-prop-2-ynyl]-pipéridine-3-acétique
Acide (3R,4R)-4-[3-(R,S)-fluoro-3-(6-méthoxyquinolin-4-yl)propyl]-1-[3-(2-méthylphényl)-prop-2-ynyl]-pipéridine-3-acétique
Acide (3R,4R)-4-[3-(R,S)-fluoro-3-(6-méthoxyquinolin-4-yl)propyl]-1-[3-(3-méthylphényl)-prop-2-ynyl]-pipéridine-3-acétique
Acide (3R,4R)-4-[3-(R,S)-fluoro-3-(6-méthoxyquinolin-4-yl)propyl]-1-[3-(4-méthylphényl)-prop-2-ynyl]-pipéridine-3-acétique
Acide (3R,4R)-4-[3-(R,S)-fluoro-3-(6-méthoxyquinolin-4-yl)propyl]-1-[3-(2-trifluorométhyl-phényl)-prop-2-ynyl]-pipéridine-3-acétique
Acide (3R,4R)-4-[3-(R,S)-fluoro-3-(6-méthoxyquinolin-4-yl)propyl]-1-[3-(3-trifluorométhyl-phényl)-prop-2-ynyl]-pipéridine-3-acétique
Acide (3R,4R)-4-[3-(R,S)-fluoro-3-(6-méthoxyquinolin-4-yl)propyl]-1-[3-(4-trifluorométhyl-phényl)-prop-2-ynyl]-pipéridine-3-acétique
Acide (3R,4R)-4-[3-(R,S)-fluoro-3-(6-méthoxyquinolin-4-yl)propyl]-1-[3-(2-méthoxyphényl)-prop-2-ynyl]-pipéridine-3-acétique
Acide (3R,4R)-4-[3-(R,S)-fluoro-3-(6-méthoxyquinolin-4-yl)propyl]-1-[3-(3-méthoxyphényl)-prop-2-ynyl]-pipéridine-3-acétique
Acide (3R,4R)-4-[3-(R,S)-fluoro-3-(6-méthoxyquinolin-4-yl)propyl]-1-[3-(4-méthoxyphényl)-prop-2-ynyl]-pipéridine-3-acétique
Acide (3R,4R)-4-[3-(R,S)-fluoro-3-(6-méthoxyquinolin-4-yl)propyl]-1-[3-(3,4-difluorophényl)-prop-2-ynyl]-pipéridine-3-acétique
Acide (3R,4R)-4-[3-(R,S)-fluoro-3-(6-méthoxyquinolin-4-yl)propyl]-1-[3-(2,4-difluorophényl)-prop-2-ynyl]-pipéridine-3-acétique
Acide (3R,4R)-4-[3-(R,S)-fluoro-3-(6-méthoxyquinolin-4-yl)propyl]-1-[3-(3,4-dichlorophényl)-prop-2-ynyl]-pipéridine-3-acétique
Acide (3R,4R)-4-[3-(R,S)-fluoro-3-(6-méthoxyquinolin-4-yl)propyl]-1-[3-(2,3-dichlorophényl)-prop-2-ynyl]-pipéridine-3-acétique
Acide (3R,4R)-4-[3-(R,S)-fluoro-3-(6-méthoxyquinolin-4-yl)propyl]-1-[3-(2,4-dichlorophényl)-prop-2-ynyl]-pipéridine-3-acérique
Acide (3R,4R)-4-[3-(R,S)-fluoro-3-(6-méthoxyquinolin-4-yl)propyl]-1-[3-(2,4,6-trichlorophényl)-prop-2-ynyl]-pipéridine-3-acétique
Acide (3R,4R)-4-[3-(R,S)-fluoro-3-(6-méthoxyquinolin-4-yl)propyl]-1-[3-(3,5-dichlorophényl)-prop-2-ynyl]-pipéridine-3-acétique
Acide (3R,4R)-4-[3-(R,S)-fluoro-3-(6-méthoxyquinolin-4-yl)propyl]-1-[3-(4-chloro-3-fluoro-phényl)-prop-2-ynyl]-pipéridine-3-acétique
Acide (3R,4R)-4-[3-(R,S)-fluoro-3-(6-méthoxyquinolin-4-yl)propyl]-1-[3-(3-chloro-4-fluoro-phényl)-prop-2-ynyl]-pipéridine-3-acétique
Acide (3R,4R)-4-[3-(R,S)-fluoro-3-(6-méthoxyquinolin-4-yl)propyl]-1-[3-(2-chloro-4-fluoro-phényl)-prop-2-ynyl]-pipéridine-3-acétique
Acide (3R,4R)-4-[3-(R,S)-fluoro-3-(6-méthoxyquinolin-4-yl)propyl]-1-[3-(3-chloro-5-fluoro-phényl)-prop-2-ynyl]-pipéridine-3-acétique
Acide (3R,4R)-4-[3-(R,S)-fluoro-3-(6-méthoxyquinolin-4-yl)propyl]-1-[3-(4-chloro-2-fluoro-phényl)-prop-2-ynyl]-pipéridine-3-acétique
Acide (3R,4R)-4-[3-(R,S)-fluoro-3-(6-méthoxyquinolin-4-yl)propyl]-1-[3-(3-fluoro-4-méthyl-phényl)-prop-2-ynyl]-pipéridine-3-acétique
Acide (3R,4R)-4-[3-(R,S)-fluoro-3-(6-méthoxyquinolin-4-yl)propyl]-1-[3-(4-chloro-3-trifluorométhyl-phényl)-prop-2-ynyl]-pipéridine-3-acétique
Acide (3R,4R)-4-[3-(R,S)-fluoro-3-(6-méthoxyquinolin-4-yl)propyl]-1-[3-(2-chloro-4-trifluorométhyl-phényl)-prop-2-ynyl]-pipéridine-3-acétique
Acide (3R,4R)-4-[3-(R,S)-fluoro-3-(6-méthoxyquinolin-4-yl)propyl]-1-[3-(2-chloro-5-trifluorométhyl-phényl)-prop-2-ynyl]-pipéridine-3-acétique
Acide (3R,4R)-4-[3-(R,S)-fluoro-3-(6-méthoxyquinolin-4-yl)propyl]-1-[3-(5-chloro-2-méthoxy-phényl)-prop-2-ynyl]-pipéridine-3-acétique
Acide (3R,4R)-4-[3-(R,S)-fluoro-3-(6-méthoxyquinolin-4-yl)propyl]-1-[3-(3,5-bis-trifluorométhyl-phényl)-prop-2-ynyl]-pipéridine-3-acétique
Acide (3R,4R)-4-[3-(R,S)-fluoro-3-(6-méthoxyquinolin-4-yl)propyl]-1-[3-(3,5-diméthylphényl)-prop-2-ynyl]-pipéridine-3-acétique
Acide (3R,4R)-4-[3-(R,S)-fluoro-3-(6-méthoxyqumolin-4-yl)propyl]-1-[3-(2,4-dichloro-6-méthyl-phényl)-prop-2-ynyl]-pipéridine-3-acétique
Acide (3R,4R)-4-[3-(R,S)-fluoro-3-(6-méthoxyquinolin-4-yl)propyl]-1-[3-(thien-2-yl)-prop-2-ynyl]-pipéridine-3-acétique
Acide (3R,4R)-4-[3-(R,S)- fluoro-3-( 6-méthoxyquinolin-4-yl)propyl]-1-f3-(5-chloro-thien-2-yl)-prop-2-ynyl]-pipéridine-3-acétique
Acide (3R,4R)-4-[3-(R,S)-fluoro-3-(6-méthoxyquinolin-4-yl)propyl]-1-[3-(3-chloro-thien-2-yl)-prop-2-ynyl]-pipéridine-3-acétique
Acide (3R,4R)-4-[3-(R,S)-fluoro-3-(6-méthoxyquinolin-4-yl)propyl]-1-[3-(5-méthyl-thien-2-yl)-prop-2-ynyl]-pipéridine-3-acétique
Acide (3R,4R)-4-[3-(R,S)-fluoro-3-(6-méthoxyquinolin-4-yl)propyl]-1-[3-(3-méthyl-thien-2-yl)-prop-2-ynyl]-pipéridine-3-acétique
Acide (3R,4R)-4-[3-(R,S)-fluoro-3-(6-méthoxyquinolin-4-yl)propyl]-1-[3-(thien-3-yl)-prop-2-ynyl]-pipéridine-3-acétique
Acide (3R,4R)-4-[3-(R,S)-fluoro-3-(6-méthoxyquinolin-4-yl)propyl]-1-[3-(1-méthyl-pyrrol-2-yl)-prop-2-ynyl]-pipéridine-3-acétique
Acide (3R,4R)-4-[3-(R,S)-fluoro-3-(6-méthoxyquinolin-4-yl]propyl]-1-[3-(1-méthyl-pyrrol-3-yl)-prop-2-ynyl]-pipéridine-3-acérique
Acide (3R,4R)-4-[3-(R,S)-fluoro-3-(6-méthoxyquinolin-4-yl)propyl]-1-[3-(thiazol-2-yl)-prop-2-ynyl]-pipéridine-3-acétique
Acide (3R,4R)-4-[3-(R,S)-fluoro-3-(6-méthoxyquinolin-4-yl)propyl]-1-[3-(thiazol-4-yl)-prop-2-ynyl]-pipéridine-3-acétique
Acide (3R,4R)-4-[3-(R,S)-fluoro-3-(6-méthoxyquinolin-4-yl)propyl]-1-[3-(thiazol-5-yl)-prop-2-ynyl]-pipéridine-3-acétique
Acide (3R,4R)-4-[3-(R,S)-fluoro-3-(6-méthoxyquinolin-4-yl)propyl]-1-[3-(1-méthylimidazol-2-yl)-prop-2-ynyl]-pipéridine-3-acétique
Acide (3R,4R)-4-[3-(R,S)-fluoro-3-(6-méthoxyquinolin-4-yl)propyl]-1-[3-(3-méthylimidazol-4-yl)-prop-2-ynyl]-pipéridine-3-acétique
Acide (3R,4R)-4-[3-(R,S)-fluoro-3-(6-méthoxyquinolin-4-yl)propyl]-1-[3-(3-méthylpyrazol-4-yl)-prop-2-ynyl]-pipéridine-3-acétique
Acide (3R,4R)-4-[3-(R,S)-fluoro-3-(6-méthoxyquinolin-4-yl)propyl]-1-[3-(oxazol-2-yl)-prop-2-ynyl]-pipéridine-3-acétique
Acide (3R,4R)-4-[3-(R,S)-fluoro-3-(6-méthoxyquinolin-4-yl)propyl]-1-[3-(oxazol-4-yl)-prop-2-ynyl]-pipéridine-3-acétique
Acide (3R,4R)-4-[3-(R,S)-fluoro-3-(6-méthoxyquinolin-4-yl)propyl]-1-[3-(oxazol-5-yl)-prop-2-ynyl]-pipéridine-3-acétique
Acide (3R,4R)-4-[3-(R,S)-fluoro-3-(6-méthoxyquinolin-4-yl)propyl]-1-[3-(pyridin-2-yl)-prop-2-ynyl]-pipéridine-3-acétique
Acide (3R,4R)-4-[3-(R,S)-fluoro-3-(6-méthoxyquinolin-4-yl)propyl]-1-[3-pyridin-3-yl)-prop-2-ynyl]-pipéridine-3-acérique
Acide (3R,4R)-4-[3-(R,S)-fluoro-3-(6-méthoxyquinolin-4-yl)propyl]-1-[3-(pyridin-4-yl)-prop-2-ynyl]-pipéridine-3-acétique
Acide (3R,4R)-4-[3-(R,S)-fluoro-3-(6-méthoxyquinolin-4-yl)propyl]-1-[3-(pyrimidin-2-yl)-prop-2-ynyl]-pipéridine-3-acétique
Acide (3R,4R)-4-[3-(R,S)-fluoro-3-(6-méthoxyquinolin-4-yl)propyl]-1-[3-(pyrimidin-4-yl)-prop-2-ynyl]-pipéridine-3-acétique
Acide (3R,4R)-4-[3-(R,S)-fluoro-3-(6-méthoxyquinolin-4-yl)propyl]-1-[3-(pyrimidin-5-yl)-prop-2-ynyl]-pipéridine-3-acétique
Acide (3R,4R)-4-[3-(R,S)-fluoro-3-(6-méthoxyquinolin-4-yl)propyl]-1-[3-(pyrazin-2-yl)-prop-2-ynyl]-pipéridine-3-acétique
Acide (3R,4R)-4-[3-(R,S)-fluoro-3-(6-méthoxyquinolin-4-yl)propyl]-1-[3-(pyridazin-3-yl)-prop-2-ynyl]-pipéridine-3-acétique
Acide (3R,4R)-4-[3-(R,S)-fluoro-3-(6-méthoxyquinolin-4-yl)propyl]-1-[3-(pyridazin-4-yl)-prop-2-ynyl]-pipéridine-3-acétique
Acide (3R,4R)-3-{4-[3-(6-méthoxyquinolin-4-yl)propyl]-1-[3-phényl-prop-2-ynyl]-piperidin-3-yl}propan-1-oique
Acide (3R,4R)-3-{4-[3-(6-méthoxyquinolin-4-yl)propyl]-1-[3-(2-fluoro-phényl)-prop-2-ynyl]-piperidin-3-yl}propan-1-oique
Acide (3R,4R)-3-{4-[3-(6-méthoxyquinolin-4-yl)propyl]-1-[3-(3-fluoro-phényl)-prop-2-ynyl]-piperidin-3-yl}propan-1-oique
Acide (3R,4R)-3- {4-[3-(6-méthoxyquinolin-4-yl)propyl]-1-[3-(4-fluoro-phényl)-prop-2-ynyl]-piperidin-3-yl}propan-1-oique
Acide (3R,4R)-3-{4-[3-(6-méthoxyquinolin-4-yl)propyl]-1-[3-(2-chloro-phényl)-prop-2-ynyl]-piperidin-3-yl}propan-1-oique
Acide (3R,4R)-3-{4-[3-(6-méthoxyquinolin-4-yl)propyl]-1-[3-(3-chloro-phényl)-prop-2-ynyl]-piperidin-3-yl}propan-1-oique
Acide (3R,4R)-3- {4-[3-(6-méthoxyquinolin-4-yl)propyl]-1-[3-(4-chloro-phényl)-prop-2-ynyl]-piperidin-3-yl}propan-1-oique
Acide (3R,4R)-3-{4-[3-(6-méthoxyquinolin-4-yl)propyl]-1-[3-(2-méthyl-phényl)-prop-2-ynyl]-piperidin-3-yl}propan-1-oique
Acide (3R,4R)-3-{4-[3-(6-méthoxyquinolin-4-yl)propyl]-1-[3-(3-méthyl-phényl)-prop-2-ynyl]-piperidin-3-yl}propan-1-oique
Acide (3R,4R)-3-{4-[3-(6-méthoxyquinolin-4-yl)propyl]-1-[3-(4-méthyl-phényl)-prop-2-ynyl]-piperidin-3-yl}propan-1-oique
Acide (3R,4R)-3-{4-[3-(6-méthoxyquinolin-4-yl)propyl]-1-[3-(2-trifluorométhyl-phényl)-prop-2-ynyl]-piperidin-3-yl}propan-1-oique
Acide (3R,4R)-3-{4-[3-(6-méthoxyquinolin-4-yl)propyl]-1-[3-(3-trifluorométhyl-phényl)-prop-2-ynyl]-piperidin-3-yl}propan-1-oique
Acide (3R,4R)-3-{4-[3-(6-méthoxyquinolin-4-yl)propyl]-1-[3-(4-trifluorométhyl-phényl)-prop-2-ynyl]-piperidin-3-yl}propan-1-oique
Acide (3R,4R)-3-{4-[3-(6-méthoxyquinolin-4-yl)propyl]-1-[3-(2-méthoxy-phényl)-prop-2-ynyl]-piperidin-3-yl}propan-1-oique
Acide (3R,4R)-3-{4-[3-(6-méthoxyquinolin-4-yl)propyl]-1-[3-(3-méthoxy-phényl)-prop-2-ynyl]-piperidin-3-yl}propan-1-oique
Acide (3R,4R)-3-{4-[3-(6-méthoxyquinolin-4-yl)propyl]-1-[3-(4-méthoxy-phényl)-prop-2-ynyl]-piperidin-3-yl}propan-1-oique
Acide (3R,4R)-3-{4-[3-(6-méthoxyquinolin-4-yl)propyl]-1-[3-(3,4-difluoro-phényl)-prop-2-ynyl]-piperidin-3-yl}propan-1-oique
Acide (3R,4R)-3-{4-[3-(6-méthoxyquinolin-4-yl)propyl]-1-[3-(2,4-difluoro-phényl)-prop-2-ynyl]-piperidin-3-yl}propan-1-oique
Acide (3R,4R)-3-{4-[3-(6-méthoxyquinolin-4-yl)propyl]-1-[3-(3,4-dichloro-phényl)-prop-2-ynyl]-piperidin-3-yl}propan-1-oique
Acide (3R,4R)-3-{4-[3-(6-méthoxyquinolin-4-yl)propyl]-1-[3-(2,3-dichloro-phényl)-prop-2-ynyl]-piperidin-3-yl}propan-1-oique
Acide (3R,4R)-3-{4-[3-(6-méthoxyquinolin-4-yl)propyl]-1-[3-(2,4-dichloro-phényl)-prop-2-ynyl]-piperidin-3-yl}propan-1-oique
Acide (3R,4R)-3-{4-[3-(6-méthoxyquinolin-4-yl)propyl]-1-[3-(2,4,6-trichloro-phényl)-prop-2-ynyl]-piperidin-3-yl}propan-1-oique
Acide (3R,4R)-3-{4-[3-(6-méthoxyquinolin-4-yl)propyl]-1-[3-(3,5-dichloro-phényl)-prop-2-ynyl]-piperidin-3-yl}propan-1-oique
Acide (3R,4R)-3-{4-[3-(6-méthoxyquinolin-4-yl)propyl]-1-[3-(4-chloro-3-fluoro-phényl)-prop-2-ynyl]-piperidin-3-yl}propan-1-oique
Acide (3R,4R)-3-{4-[3-(6-méthoxyquinolin-4-yl)propyl]-1-[3-(3-chloro-4-fluoro-phényl)-prop-2-ynyl]-piperidin-3-yl}propan-1-oique
Acide (3R,4R)-3-{4-[3-(6-méthoxyquinolin-4-yl)propyl]-1-[3-(2-chloro-4-fluoro-phényl)-prop-2-ynyl]-piperidin-3-yl}propan-1-oique
Acide (3R,4R)-3-{4-[3-(6-méthoxyquinolin-4-yl)propyl]-1-[3-(3-chloro-5-fluoro-phényl)-prop-2-ynyl]-piperidin-3-yl}propan-1-oique
Acide (3R,4R)-3-{4-[3-(6-méthoxyquinolin-4-yl)propyl]-1-[3-(4-chloro-2-fluoro-phényl)-prop-2-ynyl]-piperidin-3-yl}propan-1-oique
Acide (3R,4R)-3-{4-[3-(6-méthoxyquinolin-4-yl)propyl]-1-[3-(3-fluoro4-méthyl-phényl)-prop-2-ynyl]-piperidin-3-yl}propan-1-oique
Acide (3R,4R)-3-{4-[3-(6-méthoxyquinolin-4-yl)propyl]-1-[3-(4-chloro-3-trifluorométhylphényl)-prop-2-ynyl]-piperidin-3-yl}propan-1-oique
Acide (3R,4R)-3-{4-[3-(6-méthoxyquinolin-4-yl)propyl]-1-[3-(2-chloro-4-trifluorométhylphényl)-prop-2-ynyl]-piperidin-3-yl}propan-1-oique
Acide (3R,4R)-3-{4-[3-(6-méthoxyquinolin-4-yl)propyl]-1-[3-(2-chloro-5-trifluorométhylphényl)-prop-2-ynyl]-piperidin-3-yl}propan-1-oique
Acide (3R,4R)-3-{4-[3-(6-méthoxyquinolin-4-yl)propyl]-1-[3-(5-chloro-2-méthoxy-phényl)-prop-2-ynyl]-piperidin-3-yl}propan-1-oique
Acide (3R,4R)-3-{4-[3-(6-méthoxyquinolin-4-yl)propyl]-1-[3-(3,5-bis-trifluorométhylphényl)-prop-2-ynyl]-piperidin-3-yl}propan-1-oique
Acide (3R,4R)-3-{4-[3-(6-méthoxyquinolin-4-yl)propyl]-1-[3-(3,5-diméthyl-phényl)-prop-2-ynyl]-piperidin-3-yl}propan-1-oique
Acide (3R,4R)-3-{4-[3-(6-méthoxyquinolin-4-yl)propyl]-1-[3-(2,4-dichloro-6-méthylphényl)-prop-2-ynyl]-piperidin-3-yl}propan-1-oique
Acide (3R,4R)-3-{4-[3-(6-méthoxyquinolin-4-yl)propyl]-1-[3-(thien-2-yl)-prop-2-ynyl]-piperioEn-3-yl}propan-1-oique
Acide (3R,4R)-3-{4-[3-(6-méthoxyquinolin-4-yl)propyl]-1-[3-(5-chloro-thien-2-yl)-prop-2-ynyl]-piperidin-3-yl}propan-1-oique
Acide (3R,4R)-3-{4-[3-(6-méthoxyquinolin-4-yl)propyl]-1-[3-(3-chloro-thien-2-yl)-prop-2-ynyl]-piperidin-3-yl}propan-1-oique
Acide (3R,4R)-3- {4-[3-(6-méthoxyquinolin-4-yl)propyl]-1-[3-(5-méthyl-thien-2-yl)-prop-2-ynyl]-piperidin-3-yl}propan-1-oique
Acide (3R,4R)-3-{4-[3-(6-méthoxyquinolin-4-yl)propyl]-1-[3-(3-méthyl-thien-2-yl)-prop-2-ynyl]-piperidin-3-yl}propan-1-oique
Acide (3R,4R)-3-{4-[3-(6-méthoxyquinolin-4-yl)propyl]-1-[3-(thien-3-yl)-prop-2-ynyl]-piperidin-3-yl}propan-1-oique
Acide (3R,4R)-3-{4-[3-(6-méthoxyquinolin-4-yl)propyl]-1-[3-(1-méthyl-pyrrol-2-yl)-prop-2-ynyl]-piperidin-3-yl}propan-1-oique
Acide (3R,4R)-3-{4-[3-(6-méthoxyquinolin-4-yl)propyl]-1-[3-(1-méthyl-pyrrol-3-yl)-prop-2-ynyl]-piperidin-3-yl}propan-1-oique
Acide (3R,4R)-3-{4-[3-(6-méthoxyquinolin-4-yl)propyl]-1-[3-(thiazol-2-yl)-prop-2-ynyl]-piperidin-3-yl}propan-1-oique
Acide (3R,4R)-3-{4-[3-(6-méthoxyquinolin-4-yl)propyl]-1-[3-(thiazol-4-yl)-prop-2-ynyl]-piperidin-3-yl}propan-1-oique
Acide (3P,4R)-3-{4-[3-(6-méthoxyquinolin-4-yl)propyl]-1-[3-(thiazol-5-yl)-prop-2-ynyl]-piperidin-3-yl}propan-1-oique
Acide (3R,4R)-3-{4-[3-(6-méthoxyquinolin-4-yl)propyl]-1-[3-(1-méthyl-imidazol-2-yl)-prop-2-ynyl]-piperidin-3-yl}propan-1-oique
Acide (3R,4R)-3-{4-[3-(6-méthoxyquinolin-4-yl)propyl]-1-[3-(3-méthyl-imidazol-4-yl)-prop-2-ynyl]-piperidin-3-yl}propan-1-oique
Acide (3R,4R)-3-{4-[3-(6-méthoxyquinolin-4-yl)propyl]-1-[3-(3-méthyl-pyrazol-4-yl)-prop-2-ynyl]-piperidin-3-yl}propan-1-oique
Acide (3R,4R)-3-{4-[3-(6-méthoxyquinolin-4-yl)propyl]-1-[3-(oxazol-2-yl)-prop-2-ynyl]-piperidin-3-yl}propan-1-oique
Acide (3R,4R)-3-{4-[3-(6-méthoxyquinolin-4-yl)propyl]-1-[3-(oxazol-4-yl)-prop-2-ynyl]-piperidin-3-yl}propan-1-oique
Acide (3R,4R)-3-{4-[3-(6-méthoxyquinolin-4-yl)propyl]-1-[3-(oxazol-5-yl)-prop-2-ynyl]-piperidin-3-yl}propan-1-oique
Acide (3R,4R)-3-{4-[3-(6-méthoxyquinolin-4-yl)propyl]-1-[3-(pyridin-2-yl)-prop-2-ynyl]-piperidin-3-yl}propan-1-oique
Acide (3R,4R)-3-{4-[3-(6-méthoxyquinolin-4-yl)propyl]-1-[3-(pyridin-3-yl)-prop-2-ynyl]-piperidin-3-yl}propan-1-oique
Acide (3R,4R)-3-{4-[3-(6-méthoxyquinolin-4-yl)propyl]-1-[3-(pyridin-4-yl)-prop-2-ynyl]-piperidin-3-yl}propan-1-oique
Acide (3R,4R)-3-{4-[3-(6-méthoxyquinolin-4-yl)propyl]-1-[3-(pyrimidin-2-yl)-prop-2-ynyl]-piperidin-3-yl}propan-1-oique
Acide (3R,4R)-3-{4-[3-(6-méthoxyquinolin-4-yl)propyl]-1-[3-(pyrimidin-4-yl)-prop-2-ynyl]-piperidin-3-yl}propan-1-oique
Acide (3R,4R)-3-{4-[3-(6-méthoxyquinolin-4-yl)propyl]-1-[3-(pyrimidin-5-yl)-prop-2-ynyl]-piperidin-3-yl}propan-1-oique
Acide (3R,4R)-3-{4-[3-(6-méthoxyquinolin-4-yl)propyl]-1-[3-(pyrazin-2-yl)-prop-2-ynyl]-piperidin-3-yl}propan-1-oique
Acide (3R,4R)-3-{4-[3-(6-méthoxyquinolin-4-yl)propyl]-1-[3-(pyridazin-3-yl)-prop-2-ynyl]-piperidin-3-yl}propan-1-oique
Acide (3R,4R)-3-{4-[3-(6-méthoxyquinolin-4-yl)propyl]-1-[3-(pyridazin-4-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-phényl-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2-fluorophényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-fluorophényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(4-fluorophényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2-chlorophényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-chlorophényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(4-chlorophényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2-méthylphényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-méthylphényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(4-méthylphényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2-trifluorométhyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-trifluorométhyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(4-trifluorométhyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2-méthoxyphényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-méthoxyphényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(4-méthoxyphényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3,4-difluoro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2,4-difluoro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3,4-dichloro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2,3-dichloro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2,4-dichloro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2,4,6-trichloro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3,5-dichloro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(4-chloro-3-fluoro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-chloro-4-fluoro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2-chloro-4-fluoro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-chloro-5-fluoro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(4-chloro-2-fluoro-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-fluoro-4-méthyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(4-chloro-3-trifluorométhyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2-chloro-4-trifluoromémyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2-chloro-5-trifluorométhyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(5-chloro-2-méthoxy-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3,5-bis-trifluorométhyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3,5-diméthyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(2,4-dichloro-6-méthyl-phényl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(thien-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(5-chlorothien-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-chlorothien-2-yl)-prop-2-ynyl]-piperidin-3-yl}propan-1-oique
Acide (3R,4R)-3-{4-[3-(R,S)-hydroxy-3-(6-méthoxyqumolin-4-yl)propyl]-1-[3-(5-méthylthien-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-méthylthien-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(thien-3-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(1-méthylpyrrol-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(1-méthylpyrrol-3-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(thiazol-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(thiazol-4-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(thiazol-5-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(1-méthylimidazol-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-méthylimidazol-4-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(3-méthylpyrazol-4-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(oxazol-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(oxazol-4-yl)-prop-2-ynyl]-pipendin-3-yl}propan-1-oique
Acide (3R,4R)-3-{4-[3-(R,S)-hydroxy-3-(6-méthoxyquinolin-4-yl)propyl]-1-[3-(oxazol-5-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyridin-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyridin-3-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyridin-4-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyrimidin-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyrimidin-4-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyrimidin-5-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyrazin-2-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyridazin-3-yl)-prop-2-ynyl]-piperidin-3-yl}propan-1-oique
Acide (3R,4R)-3-{4-[3-(R,S)-hydroxy-3-(6-méthoxyquinolin-4-yl)propyl]-1-[3-(pyridazin-4-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-phényl-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2-fluorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-fluorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(4-fluorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2-chlorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-chlorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(4-chlorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2-méthylphényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-méthylphényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(4-méthylphényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2-trifluorométhyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-trifluorométhyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(4-trifluorométhyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2-méthoxyphényl)-prop-2-ynyl]-piperidin-3-yl}propan-1-oique
Acide (3R,4R)-3-{4-[3-(R,S)-fluoro-3-(6-méthoxyquinolin-4-yl)ropyl]-1-[3-(3-méthoxyphényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(4-méthoxyphényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3,4-difluorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2,4-difluorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3,4-dichlorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2,3-dichlorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2,4-dichlorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2,4,6-trichloro-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3,5-dichlorophényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(4-chloro-3-fluoro-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-chloro-4-fluoro-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2-chloro-4-fluoro-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-chloro-5-fluoro-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(4-chloro-2-fluoro-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-fluoro-4-méthyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(4-chloro-3-trifluorométhyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2-chloro-4-trifluorométhyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2-chloro-5-trifluorométhyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(5-chloro-2-méthoxy-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3,5-bistrifluorométhyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3,5-diméthylphényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(2,4-dichloro-6-méthyl-phényl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(thien-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(5-chlorothien-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-chlorothien-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(5-méthylthien-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-méthylthien-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(thien-3-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(1-méthylpyrrol-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(1-méthylpyrrol-3-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(thiazol-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(thiazol-4-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(thiazol-5-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-{3-(1-méthylimidazol-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-méthylimidazol-4-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(3-méthylpyrazol-4-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(oxazol-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(oxazol-4-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(oxazol-5-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyridin-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyridin-3-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyridin-4-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyrimidin-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyrimidin-4-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyrimidin-5-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyrazin-2-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyridazin-3-yl)-prop-2-ynyl]-piperidin-3-yl}propan-1-oique
Acide (3R,4R)-3-{4-[3-(R,S)-fluoro-3-(6-méthoxyquinolin-4-yl)propyl]-1-[3-(pyridazin-4-yl)-prop-2-ynyl]-piperidin-3-yl}propan-1-oique
(3R,4R)-3-hydroxyméthyl-4-[3-(6-méthoxyquinolin-4-yl)propyl]-1-[3-phényl-prop-2-ynyl]-pipéridine
(3R,4R)-3-hydroxyméthyl-4-[3-(6-méthoxyquinolin-4-yl)propyl]-1-[3-(2-fluoro-phényl)-prop-2-ynyl]-pipéridine
(3R,4R)-3-hydroxyméthyl-4-[3-(6-méthoxyquinolin-4-yl)propyl]-1-[3-(3-fluoro-phényl)-prop-2-ynyl]-pipéridine
(3R,4R)-3-hydroxyméthyl-4-[3-(6-méthoxyquinolin-4-yl)propyl]-1-[3-(4-fluoro-phényl)-prop-2-ynyl]-pipéridine
(3R,4R)-3-hydroxyméthyl-4-[3-(6-méthoxyquinolin-4-yl)propyl]-1-[3-(2-chloro-phényl)-prop-2-ynyl]-pipéridine
(3R,4R)-3-hydroxyméthyl-4-[3-(6-méthoxyquinolin-4-yl)propyl]-1-[3-(3-chloro-phényl)-prop-2-ynyl]-pipéridine
(3R,4R)-3-hydroxyméthyl-4-[3-(6-méthoxyquinolin-4-yl)propyl]-1-[3-(4-chloro-phényl)-prop-2-ynyl]-pipéridine
(3R,4R)-3-hydroxyméthyl-4-[3-(6-méthoxyquinolin-4-yl)propyl]-1-[3-(2-méthyl-phényl)-prop-2-ynyl]-pipéridine
(3R,4R)-3-hydroxyméthyl-4-[3-(6-méthoxyquinolin-4-yl)propyl]-1-[3-(3-méthyl-phényl)-prop-2-ynyl]-pipéridine
(3R,4R)-3-hydroxyméthyl-4-[3-(6-méthoxyquinolin-4-yl)propyl]-1-[3-(4-méthyl-phényl)-prop-2-ynyl]-pipéridine
(3R,4R)-3-hydroxyméthyl-4-[3-(6-méthoxyquinolin-4-yl)propyl]-1-[3-(2-trifluorométhylphényl)-prop-2-ynyl]-pipéridine
(3R,4R)-3-hydroxyméthyl-4-[3-(6-méthoxyquinolin-4-yl)propyl]-1-[3-(3-trifluorométhylphényl)-prop-2-ynyl]-pipéridine
(3R,4R)-3-hydroxyméthyl-4-[3-(6-méthoxyquinolin-4-yl)propyl]-1-[3-(4-trifluorométhylphényl)-prop-2-ynyl]-pipéridine
(3R,4R)-3-hydroxyméthyl-4-[3-(6-méthoxyquinolin-4-yl)propyl]-1-[3-(2-méthoxy-phényl)-prop-2-ynyl]-pipéridine
(3R,4R)-3-hydroxyméthyl-4-[3-(6-méthoxyquinolin-4-yl)propyl]-1-[3-(3-méthoxy-phényl)-prop-2-ynyl]-pipéridine
(3R,4R)-3-hydroxyméthyl-4-[3-(6-méthoxyquinolin-4-yl)propyl]-1-[3-(4-méthoxy-phényl)-prop-2-ynyl]-pipéridine
(3R,4R)-3-hydroxyméthyl-4-[3-(6-méthoxyquinolin-4-yl)propyl]-1-[3-(thien-2-yl)-prop-2-ynyl]-pipéridine
(3R,4R)-3-hydroxyméthyl-4-[3-(6-méthoxyquinolin-4-yl)propyl]-1-[3-(thien-3-yl)-prop-2-ynyl]-pipéridine
(3R,4R)-3-hydroxyméthyl-4-[3-(6-méthoxyquinolin-4-yl)propyl]-1-[3-(thiazol-2-yl)-prop-2-ynyl]-pipéridine
(3R,4R)-3-hydroxyméthyl-4-[3-(6-méthoxyquinolin-4-yl)propyl]-1-[3-(thiazol-4-yl)-prop-2-ynyl]-pipéridine
(3R,4R)-3-hydroxyméthyl-4-[3-(6-méthoxyquinolin-4-yl)propyl]-1-[3-(thiazol-5-yl)-prop-2-ynyl]-pipéridine
(3R,4R)-3-hydroxyméthyl-4-[3-(6-méthoxyquinolin-4-yl)propyl]-1-[3-(oxazol-2-yl)-prop-2-ynyl]-pipéridine
(3R,4R)-3-hydroxyméthyl-4-[3-(6-méthoxyquinolin-4-yl)propyl]-1-[3-(oxazol-4-yl)-prop-2-ynyl]-pipéridine
(3R,4R)-3-hydroxyméthyl-4-[3-(6-méthoxyquinolin-4-yl)propyl]-1-[3-(oxazol-5-yl)-prop-2-ynyl]-pipéridine
(3R,4R)-3-hydroxyméthyl-4-[3-(6-méthoxyquinolin-4-yl)propyl]-1-[3-(pyridin-2-yl)-prop-2-ynyl]-pipéridine
(3R,4R)-3-hydroxyméthyl-4-[3-(6-méthoxyquinolin-4-yl)propyl]-1-[3-(pyridin-3-yl)-prop-2-ynyl]-pipéridine
(3R,4R)-3-hydroxyméthyl-4-[3-(6-méthoxyquinolin-4-yl)propyl]-1-[3-(pyridin-4-yl)-prop-2-ynyl]-pipéridine
(3R,4R)-3-hydroxyméthyl-4-[3-(6-méthoxyquinolin-4-yl)propyl]-1-[3-(pyrimidin-2-yl)-prop-2-ynyl]-pipéridine
(3R,4R)-3-hydroxyméthyl-4-[3-(6-méthoxyquinolin-4-yl)propyl]-1-[3-(pyrazin-2-yl)-prop-2-ynyl]-pipéridine
(3R,4R)-3-hydroxyméthyl-4-[3-(6-méthoxyquinolin-4-yl)propyl]-1-[3-(pyridazin-3-yl)-prop-2-ynyl]-pipéridine
(3R,4R)-3-hydroxyméthyl-4-[3-(6-méthoxyquinolin-4-yl)propyl]-1-[3-(pyridazin-4-yl)-prop-2-ynyl]-pipéridine
(3R,4R)-3-hydroxyméthyl-4-[3-(R,S)-hydroxy-3-(6-méthoxyquinolin-4-yl)propyl]-1-[3-phényl-prop-2-ynyl]-pipéridine
(3R,4R)-3-hydroxyméthyl-4-[3-(R,S)-hydroxy-3-(6-méthoxyquinolin-4-yl)propyl]-1-[3-(2-fluoro-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(3-fluoro-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(4-fluoro-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(2-chloro-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(3-chloro-phényl)-pmp-2-ynyl]-pipéridine
(3R,4R)-3-hydroxyméthyl-4-[3-(R,S)-hydroxy-3-(6-méthoxyquinolin-4-yl)propyl]-1-[3-(4-chloro-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(2-méthyl-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(3-méthyl-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(4-méthyl-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(2-trifluorométhyl-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(3-trifluorométhyl-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(4-trifluorométhyl-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(2-méthoxy-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(3-méthoxy-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(4-méthoxy-phényl)-prop-2-ynyl]-pipéridine
(3R,4R)-3-hydroxyméthyl-4-[3-(R,S)-hydroxy-3-(6-méthoxyquinolin-4-yl)propyl]-1-[3-(thien-2-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(thien-3-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(thiazol-2-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(thiazol-4-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(thiazol-5-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(oxazol-2-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(oxazol-4-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(oxazol-5-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(pyridin-2-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(pyridin-3-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(pyridin-4-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(pyrimidin-2-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(pyrazin-2-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(pyridazin-3-yl)-prop-2-ynyl]-pipéridine
(3R,4R)-3-hydroxyméthyl-4-[3-(R,S)-hydroxy-3-(6-méthoxyquinolin-4-yl)propyl]-1-[3-(pyridazin-4-yl)-prop-2-ynyl]-pipéridine
(3R,4R)-3-hydroxyméthyl-4-[3-(R,S)-fluoro-3-(6-méthoxyquinolin-4-yl)propyl]-1-[3-phényl-prop-2-ynyl]-pipéridine
(3R,4R)-3-hydroxyméthyl-4-[3-(R,S)-fluoro-3-(6-méthoxyquinolin-4-yl)propyl]-1-[3-(2-fluoro-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(3-fluoro-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(4-fluoro-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(2-chloro-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(3-chloro-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(4-chloro-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(2-méthyl-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(3-méthyl-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(4-méthyl-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(2-trifluorométhyl-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(3-trifluorométhyl-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(4-trifluorométhyl-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(2-méthoxy-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(3-méthoxy-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(4-méthoxy-phényl)-prop-2-ynyl]-pipéridine
(3R,4R)-3-hydroxyméthyl-4-[3-(R,S)-fluoro-3-(6-méthoxyquinolin-4-yl)propyl]-1-[3-(thien-2-yl)-prop-2-ynyl]-pipéndme
(3R,4R)-3-hydroxyméthyl-4-[3-(R,S)-fluoro-3-(6-méthoxyquinolin-4-yl)propyl]-1-[3-(thien-3-yl)-prop-2-ynyl]-pipéridine
(3R,4R)-3-hydroxyméthyl-4-[3-(R,S)-fluoro-3-(6-méthoxyquinolin-4-yl)pmpyl]-1-[3-(thiazol-2-yl)-prop-2-ynyl]-pipéridine
(3R,4R)-3-hydroxyméthyl-4-[3-(R,S)-fluoro-3-(6-méthoxyquinolin-4-yl)propyl]-1-[3-(thiazol-4-yl)-prop-2-ynyl]-pipéridine
(3R,4R)-3-hydroxyméthyl-4-[3-(R,S)-fluoro-3-(6-méthoxyquinolin-4-yl)propyl]-1-[3-(thiazol-5-yl)-prop-2-ynyl]-pipéridine
(3R,4R)-3-hydroxyméthyl-4-[3-(R,S)-fluoro-3-(6-méthoxyquinolin-4-yl)propyl]-1-[3-(oxazol-2-yl)-prop-2-yhyl]-pipéridine
(3R,4R)-3-hydroxyméthyl-4-[3-(R,S)-fluoro-3-(6-méthoxyquinolin-4-yl)propyl]-1-[3-(oxazol-4-yl)-prop-2-ynyl]-pipéridine
(3R,4R)-3-hydroxyméthyl-4-[3-(R,S)-fluoro-3-(6-méthoxyquinolin-4-yl)propyl]-1-[3-(oxazol-5-yl)-prop-2-ynyl]-pipéridine
(3R,4R)-3-hydroxyméthyl-4-[3-(R,S)-fluoro-3-(6-méthoxyquinolin-4-yl)propyl]-1-[3-(pyridin-2-yl)-prop-2-ynyl]-pipéridine
(3R,4R)-3-hydroxyméthyl-4-[3-(R,S)-fluoro-3-(6-méthoxyquinolin-4-yl)propyl]-1-[3-(pyridin-3-yl)-prop-2-ynyl]-pipéridine
(3R,4R)-3-hydroxyméthyl-4-[3-(R,S)-fluoro-3-(6-méthoxyquinolin-4-yl)propyl]-1-[3-(pyridin-4-yl)-prop-2-ynyl]-pipéridine
(3R,4R)-3-hydroxyméthyl-4-[3-(R,S)-fluoro-3-(6-méthoxyquinolin-4-yl)propyl]-1-[3-(pyrimidin-2-yl)-prop-2-ynyl]-pipéridine
(3R,4R)-3-hydroxyméthyl-4-[3-(R,S)-fluoro-3-(6-méthoxyquinolin-4-yl)propyl]-1-[3-(pyrazin-2-yl)-prop-2-ynyl]-pipéridine
(3R,4R)-3-hydroxyméthyl-4-[3-(R,S)-fluoro-3-(6-méthoxyquinolin-4-yl)propyl]-1-[3-(pyridazin-3-yl)-prop-2-ynyl]-pipéridine
(3R,4R)-3-hydroxyméthyl-4-[3-(R,S)-fluoro-3-(6-méthoxyquinolin-4-yl)propyl]-1-[3-(pyridazin-4-yl)-prop-2-ynyl]-pipéridine
Acide (3R,4R)-4-[3-(6-méthoxyquinolin-4-yl)propyl]-1-[3-(2,3-difluoro-phényl)-prop-2-ynyl]pipéridine-3-carboxylique
Acide (3R,4R)-4-[3-(6-méthoxyquinolin-4-yl)propyl]-1-[3-(2,5-difluoro-phényl)-prop-2-ynyl]pipéridine-3-carboxylique
Acide (3R,4R)-4-[3-(6-méthoxyquinolin-4-yl)propyl]-1-[3-(3,5-difluoro-phényl)-prop-2-ynyl]pipéridine-3-carboxylique
Acide (3R,4R)-4-[3-(6-méthoxyquinolin-4-yl)propyl]-1-[3-(2,6-difluoro-phényl)-prop-2-ynyl]pipéridine-3-carboxylique
Acide (3R,4R)-4-[3-(6-méthoxyquinolin-4-yl)propyl]-1-[3-(2,3,5-trifluoro-phényl)-prop-2-ynyl]pipéridine-3-carboxylique
Acide (3R,4R)-4-[3-(6-méthoxyquinolin-4-yl)propyl]-1-[3-(2,3,6-trifluorophényl)-prop-2-ynyl]pipéridine-3-carboxylique
Acide (3R,4R)-4-[3-(6-méthoxyquinolin-4-yl)propyl]-1-[3-(2-cyano-3-fluorophényl)-prop-2-ynyl]pipéridine-3-carboxylique
Acide (3R,4R)-4-[3-(6-méthoxyquinolin-4-yl)propyl]-1-[3-cyano-6-fluorophényl)-prop-2-ynyl]pipéridine-3-carboxylique
Acide (3R,4R)-4-[3-(6-méthoxyquinolin-4-yl)propyl]-1-[2-acétamido-5-fluorophényl)-prop-2-ynyl]pipéridine-3-carboxylique
Acide (3R,4R)-4-[3-(6-méthoxyquinolin-4-yl)propyl]-1-[3-trifluorométhoxyphényl)-prop-2-ynyl]pipéridine-3-carboxylique
Acide (3R,4R)-4-[3-(R,S)-hydroxy-3-(6-méthoxyquinolin-4-yl)propyl]-1-[3-(2,3,5-trifluoro-phényl)-prop-2-ynyl]pipéridine-3-carboxylique
Acide (3R,4R)-4-[3-(R,S)-hydroxy-3-(6-méthoxyquinolin-4-yl)propyl]-1-[3-(2,3,6-trifluoro-phényl)-prop-2-ynyl]pipéridine-3-carboxylique
Acide (3R,4R)-4-[3-(R,S)-hydroxy-3-(6-méthoxyquinolin-4-yl)propyl]-1-[3-(2-cyano-3-fluoro-phényl)-prop-2-ynyl]pipéridine-3-carboxylique
Acide (3R,4R)-4-[3-(R,S)-hydroxy-3-(6-méthoxyquinolin-4-yl)propyl]-1-[3-cyano-6-fluoro-phényl)-prop-2-ynyl]pipéridine-3-carboxylique
Acide (3R,4R)-4-[3-(R,S)-hydroxy-3-(6-méthoxyquinolin-4-yl)propyl]-1-[2-acétamido-5-fluoro-phényl)-prop-2-ynyl]pipéridine-3-carboxylique
Acide (3R,4R)-4-[3-(R,S)-hydroxy-3-(6-méthoxyquinolin-4-yl)propyl]-1-[3-trifluorométhoxy-phényl)-prop-2-ynyl]pipéridine-3-carboxylique
Acide (3R,4R)-4-[3-(R,S)-fluoro-3-(6-méthoxyquinolin-4-yl)propyl]-1-[3-(2,3-difluoro-phényl)-prop-2-ynyl]pipéridine-3-carboxylique
Acide (3R,4R)-4-[3-(R,S)-fluoro-3-(6-méthoxyquinolin-4-yl)propyl]-1-[3-(2,5-difluoro-phényl)-prop-2-ynyl]pipéridine-3-carboxylique
Acide (3R,4R)-4-[3-(R,S)-fluoro-3-(6-méthoxyquinolin-4-yl)propyl]-1-[3-(3,5-difluoro-phényl)-prop-2-ynyl]pipéridine-3-carboxylique
Acide (3R,4R)-4-[3-(R,S)-fluoro-3-(6-méthoxyquinolin-4-yl)propyl]-1-[3-(2,6-difluoro-phényl)-prop-2-ynyl]pipéridine-3-carboxylique
Acide (3R,4R)-4-[3-(R,S)-fluoro-3-(6-méthoxyquinolin-4-yl)propyl]-1-[3-(2,3,5-trifluoro-phényl)-prop-2-ynyl]pipéridine-3-carboxylique
Acide (3R,4R)-4-[3-(R,S)-fluoro-3-(6-méthoxyquinolin-4-yl)propyl]-1-[3-(2,3,6-trifluoro-phényl)-prop-2-ynyl]pipéridine-3-carboxylique
Acide (3R,4R)-4-[3-(R,S)-fluoro-3-(6-méthoxyquinolin-4-yl)propyl]-1-[3-(2-cyano-3-fluoro-phényl)-prop-2-ynyl]pipéridine-3-carboxylique
Acide (3R,4R)-4-[3-(R,S)-fluoro-3-(6-méthoxyquinolin-4-yl)propyl]-1-[3-cyano-6-fluoro-phényl)-prop-2-ynyl]pipéridine-3-carboxylique
Acide (3R,4R)-4-[3-(R,S)-fluoro-3-(6-méthoxyquinolin-4-yl)propyl]-1-[3-trifluorométhoxy-phényl)-prop-2-ynyl]pipéridine-3-carboxylique
Acide (3R,4R)-4-[3-(6-méthoxyquinolin-4-yl)propyl]-1-[3-(2,3-difluoro-phényl)-prop-2-ynyl]pipéridine-3-acétique
Acide (3R,4R)-4-[3-(6-méthoxyquinolin-4-yl)propyl]-1-[3-(2,5-difluoro-phényl)-prop-2-ynyl]pipéridine-3-acétique
Acide (3R,4R)-4-[3-(6-méthoxyquinolin-4-yl)propyl]-1-[3-(2,6-difluoro-phényl)-prop-2-ynyl]pipéridine-3-acétique
Acide (3R,4R)-4-[3-(6-méthoxyquinolin-4-yl)propyl]-1-[3-(2,3,5-trifluoro-phényl)-prop-2-ynyl]pipéridine-3-acétique
Acide (3R,4R)-4-[3-(6-méthoxyquinolin-4-yl)propyl]-1-[3-(2,3,6-trifluoro-phényl)-prop-2-ynyl]pipéridine-3-acétique
Acide (3R,4R)-4-[3-(6-méthoxyquinolin-4-yl)propyl]-1-[3-(2-cyano-3-fluorophényl)-prop-2-ynyl]pipéridine-3-acétique
Acide (3R,4R)-4-[3-(6-méthoxyquinolin-4-yl)propyl]-1-[3-cyano-6-fluoro-phényl)-prop-2-ynyl]pipéridine-3-acétique
Acide (3R,4R)-4-[3-(6-méthoxyquinolin-4-yl)propyl]-1-[3-trifluorométhoxy-phényl)-prop-2-ynyl]pipéridine-3-acétique
Acide (3R,4R)-4-[3-(R,S)-hydroxy-3-(6-méthoxyquinolin-4-yl)propyl]-1-[3-(2,3-difluoro-phényl)-prop-2-ynyl]pipéridine-3-acétique
Acide (3R,4R)-4-[3-(R,S)-hydroxy-3-(6-méthoxyquinolin-4-yl)propyl]-1-[3-(2,5-difluoro-phényl)-prop-2-ynyl]pipéridine-3-acétique
Acide (3R,4R)-4-[3-(R,S)-hydroxy-3-(6-méthoxyquinolin-4-yl)propyl]-1-[3-(3,5-difluoro-phényl)-prop-2-ynyl]pipéridine-3-acétique
Acide (3R,4R)-4-[3-(R,S)-hydroxy-3-(6-méthoxyquinolin-4-yl)propyl]-1-[3-(2,6-difluoro-phényl)-prop-2-ynyl]pipéridine-3-acétique
Acide (3R,4R)-4-[3-(R,S)-hydroxy-3-(6-méthoxyquinolin-4-yl)propyl]-1-[3-(2,3,5-trifluoro-phényl)-prop-2-ynyl]pipéridine-3-acétique
Acide (3R,4R)-4-[3-(R,S)-hydroxy-3-(6-méthoxyquinolin-4-yl)propyl]-1-[3-(2,3,6-trifluoro-phényl)-prop-2-ynyl]pipéridine-3-acétique
Acide (3R,4R)-4-[3-(R,S)-hydroxy-3-(6-méthoxyquinolin-4-yl)propyl]-1-[3-(2-cyano-3-fluoro-phényl)-prop-2-ynyl]pipéridine-3-acétique
Acide (3R,4R)-4-[3-(R,S)-hydroxy-3-(6-méthoxyquinolin-4-yl)propyl]-1-[3-cyano-6-fluoro-phényl)-prop-2-ynyl]pipéridine-3-acétique
Acide (3R,4R)-4-[3-(R,S)-hydroxy-3-(6-méthoxyquinolin-4-yl)propyl]-1-[3-trifluorométhoxy-phényl)-prop-2-ynyl]pipéridine-3-acétique
Acide (3R,4R)-4-[3-(R,S)-fluoro-3-(6-méthoxyquinolin-4-yl)propyl]-1-[3-(2,3-difluoro-phényl)-prop-2-ynyl]pipéridine-3-acétique
Acide (3R,4R)-4-[3-(R,S)-fluoro-3-(6-méthoxyquinolin-4-yl)propyl]-1-[3-(2,5-difluoro-phényl)-prop-2-ynyl]pipéridine-3-acétique
Acide (3R,4R)-4-[3-(R,S)-fluoro-3-(6-méthoxyquinolin-4-yl)propyl]-1-[3-(3,5-difluoro-phényl)-prop-2-ynyl]pipéridine-3-acétique
Acide (3R,4R)-4-[3-(R,S)-fluoro-3-(6-méthoxyquinolin-4-yl)propyl]-1-[3-(2,6-difluoro-phényl)-prop-2-ynyl]pipéridine-3-acétique
Acide (3R,4R)-4-[3-(R,S)-fluoro-3-(6-méthoxyquinolin-4-yl)propyl]-1-[3-(2,3,5-trifluoro-phényl)-prop-2-ynyl]pipéridine-3-acétique
Acide (3R,4R)-4-[3-(R,S)-fluoro-3-(6-méthoxyquinolin-4-yl)propyl]-1-[3-(2,3,6-trifluoro-phényl)-prop-2-ynyl]pipéridine-3-acétique
Acide (3R,4R)-4-[3-(R,S)-fluoro-3-(6-méthoxyquinolin-4-yl)propyl]-1-[3-(2-cyano-3-fluoro-phényl)-prop-2-ynyl]pipéridine-3-acétique
Acide (3R,4R)-4-[3-(R,S)-fluoro-3-(6-méthoxyquinolin-4-yl)propyl]-1-[3-cyano-6-fluoro-phényl)-prop-2-ynyl]pipéridine-3-acétique
Acide (3R,4R)-4-[3-(R,S)-fluoro-3-(6-méthoxyquinolin-4-yl)propyl]-1-[3-trifluorométhoxy-phényl)-prop-2-ynyl]pipéridine-3-acétique
(3R,4R)-3-hydroxyméthyl-4-[3-(6-méthoxyquinolin-4-yl)propyl]-1-[3-(2,3-difluorophényl)-prop-2-ynyl]pipéridine
(3R,4R)-3-hydroxyméthyl-4-[3-(6-méthoxyquinolin-4-yl)propyl]-1-[3-(2,5-difluorophényl)-prop-2-ynyl]pipéridine
(3R,4R)-3-hydroxyméthyl-4-[3-(6-méthoxyquinolin-4-yl)propyl]-1-[3-(3,5-difluorophényl)-prop-2-ynyl]pipéridine
(3R,4R)-3-hydroxyméthyl-4-[3-(6-méthoxyquinolin-4-yl)propyl]-1-[3-(2,6-difluorophényl)-prop-2-ynyl]pipéridine
(3R,4R)-3-hydroxyméthyl-4-[3-(6-méthoxyquinolin-4-yl)propyl]-1-[3-(2,3,5-trifluoro-phényl)-prop-2-ynyl]pipéridine
(3R,4R)-3-hydroxyméthyl-4-[3-(6-méthoxyquinolin-4-yl)propyl]-1-[3-(2,3,6-trifluoro-phényl)-prop-2-ynyl]pipéridine
(3R,4R)-3-hydroxyméthyl-4-[3-(6-méthoxyquinolin-4-yl)propyl]-1-[3-(2-cyano-3-fluoro-phényl)-prop-2-ynyl]pipéridine
(3R,4R)-3-hydroxyméthyl-4-[3-(6-méthoxyquinolin-4-yl)propyl]-1-[3-cyano-6-fluorophényl)-prop-2-ynyl]pipéridine
(3R,4R)-3-hydroxyméthyl-4-[3-(6-méthoxyquinolin-4-yl)propyl]-1-[3-trifluorométhoxy-phényl)-prop-2-ynyl]pipéridine
(3R,4R)-3-hydroxyméthyl-4-[3-(R,S)-hydroxy-3-(6-méthoxyquinolin-4-yl)propyl]-1-[3-(2,3-difluoro-phényl)-prop-2-ynyl]pipéridine
(3R,4R)-3-hydroxyméthyl-4-[3-(R,S)-hydroxy-3-(6-méthoxyquinolin-4-yl)propyl]-1-[3-(2,5-difluoro-phényl)-prop-2-ynyl]pipéridine
(3R,4R)-3-hydroxyméthyl-4-[3-(R,S)-hydroxy-3-(6-méthoxyquinolin-4-yl)propyl]-1-[3-(3,5-difluoro-phényl)-prop-2-ynyl]pipéridine
(3R,4R)-3-hydroxyméthyl-4-[3-(R,S)-hydroxy-3-(6-méthoxyquinolin-4-yl)propyl]-1-[3-(2,6-difluoro-phényl)-prop-2-ynyl]pipéridine
(3R,4R)-3-hydroxyméthyl-4-[3-(R,S)-hydroxy-3-(6-méthoxyquinolin-4-yl)propyl]-1-[3-(2,3,5-trifluoro-phényl)-prop-2-ynyl]pipéridine
(3R,4R)-3-hydroxyméthyl-4-[3-(R,S)-hydroxy-3-(6-méthoxyquinolin-4-yl)propyl]-1-[3-(2,3,6-trifluoro-phényl)-prop-2-ynyl]pipéridine
(3R,4R)-3-hydroxyméthyl-4-[3-(R,S)-hydroxy-3-(6-méthoxyquinolin-4-yl)propyl]-1-[3-(2-cyano-3-fluoro-phényl)-prop-2-ynyl]pipéridine
(3R,4R)-3-hydroxyméthyl-4-[3-(R,S)-hydroxy-3-(6-méthoxyquinolin-4-yl)propyl]-1-[3-cyano-6-fluoro-phényl)-prop-2-ynyl]pipéridine
(3R,4R)-3-hydroxyméthyl-4-[3-(R,S)-hydroxy-3-(6-méthoxyquinolin-4-yl)propyl]-1-[3-trifluorométhoxy-phényl)-prop-2-ynyl]pipéridine
(3R,4R)-3-hydroxyméthyl-4-[3-(R,S)-fluoro-3-(6-méthoxyquinolin-4-yl)propyl]-1-[3-(2,3-difluoro-phényl)-prop-2-ynyl]pipéridine
(3R,4R)-3-hydroxyméthyl-4-[3-(R,S)-fluoro-3-(6-méthoxyquinolin-4-yl)propyl]-1-[3-(2,5-difluoro-phényl)-prop-2-ynyl]pipéridine
(3R,4R)-3-hydroxyméthyl-4-[3-(R,S)-fluoro-3-(6-méthoxyquinolin-4-yl)propyl]-1-[3-(3,5-difluoro-phényl)-prop-2-ynyl]pipéridine
(3R,4R)-3-hydroxyméthyl-4-[3-(R,S)-fluoro-3-(6-méthoxyquinolin-4-yl)propyl]-1-[3-(2,6-difluoro-phényl)-prop-2-ynyl]pipéridine
(3R,4R)-3-hydroxyméthyl-4-[3-(R,S)-fluoro-3-(6-méthoxyquinolin-4-yl)propyl]-1-[3-(2,3,5-trifluoro-phényl)-prop-2-ynyl]pipéridine
(3R,4R)-3-hydroxyméthyl-4-[3-(R,S)-fluoro-3-(6-méthoxyquinolin-4-yl)propyl]-1-[3-(2,3,6-trifluoro-phényl)-prop-2-ynyl]pipéridine
(3R,4R)-3-hydroxyméthyl-4-[3-(R,S)-fluoro-3-(6-méthoxyquinolin-4-yl)propyl]-1-[3-(2-cyano-3-fluoro-phényl)-prop-2-ynyl]pipéridine
(3R,4R)-3-hydroxyméthyl-4-[3-(R,S)-fluoro-3-(6-méthoxyquinolin-4-yl)propyl]-1-[3-cyano-6-fluoro-phényl)-prop-2-ynyl]pipéridine
(3R,4R)-3-hydroxyméthyl-4-[3-(R,S)-fluoro-3-(6-méthoxyquinolin-4-yl)propyl]-1-[3-trifluorométhoxy-phényl)-prop-2-ynyl]pipéridine

Les exemples suivants donnés à titre non limitatif illustrent la présente invention.

### Exemple 2

### Dichlorhydrate de l'acide (3R,4R)-4-[3-(6-méthoxyquinolin-4-yl)propyl]-1-[3-(thién-2-yl)prop-2-ynyl)]pipéridine-3-carboxylique

A une solution de 0,185 g d'acide (3R,4R)-4-[3-(6-méthoxyquinolin-4-yl)propyl]-1-(prop-2-ynyl)pipéridine-3-carboxylique dans 4 cm³ de diméthylformamide anhydre, on ajoute sous agitation 0,057 cm³ de 2-iodothiophène et 1,42 cm³ de triéthylamine, puis 0,038 g de tétrakis (triphénylphosphine) palladium et 0,019 g d'iodure cuivreux. La solution est agitée pendant 20 heures à une température voisine de 20°C. 75 cm³ d'acétate d'éthyle et 75 cm³ d'eau sont ajoutés au mélange réactionnel. Après agitation du mélange, la phase aqueuse est décantée, puis neutralisée à pH 6 par addition d'une solution aqueuse d'acide chlorhydrique 0,1N. La phase aqueuse est extraite par 50 cm³ d'acétate d'éthyle ; l'extrait est lavé par 2 fois 75 cm³ d'une solution aqueuse saturée de chlorure de sodium. La solution organique est séchée sur sulfate de magnésium, filtrée, puis mélangée sous pression réduite (5 kPa), à une température voisine de 40°C. On obtient 0,090 g d'une huile de couleur jaune que l'on purifie par chromatographie sur une colonne de gel de silice (granulométrie 20-45 µ ; diamètre 1 cm ; hauteur 30 cm), en éluant, sous une pression due 50 kPa d'azote, par un mélange dichlorométhane-méthanol (92/8 en volumes), et en recueillant des fractions de 50 cm³. Les fractions 12 à 15 sont réunies, concentrées sous pression réduite (5 kPa), à une température voisine de 40°C. L'huile obtenue est reprise par 1 cm³ de dioxanne chlorhydrique 4N. Après concentration dans les mêmes conditions que précédemment, et reprise du résidu dans 10 cm³ d'éther diéthylique, on recueille après filtration, 0,030 g de dichlorhydrate d'acide (3R,4R)-4-[3-(6-méthoxyquinolin-4-yl)propyl]-1-[3-(thién-2-yl)prop-2-ynyl)]pipéridine-3-carboxylique, sous forme d'un solide blanc. Spectre de R.M.N. ¹H (400 MHz, (CD₃)₂SO d6, δ en ppm) : de 1,15 à 2,10 et de 3,00 à 3,65 (mts : 14H) ; 3,98 (s : 3H) ; de 4,25 à 4,55 (mf : 2H) ; 7,16 (dd, J = 5 et 3 Hz : 1H) ; de 7,40 à 7,60 (mt : 4H) ; 7,75 (d large, J = 5 Hz : 1H) ; 7,96 (mt : 1H) ; 8,79 (mt : 1H) ; de 10,50 à 10,70 (mf étalé : 1H) ; de 12,85 à 13,15 (mf étalé : 1H).

L'acide (3R,4R)-4-[3-(6-méthoxyquinolin-4-yl)propyl]-1-(prop-2-ynyl)pipéridine-3-carboxylique peut être préparé de la manière suivante :
0,3 g de (3R,4R)-4-[3-(6-méthoxyquinolin-4-yl)propyl]-1-(prop-2-ynyl)pipéridine-3-carboxylate de (prop-2-ynyle) dans 3 cm³ de dioxanne et 1,48 cm³ de soude N sont chauffés, sous agitation, à une température voisine de 70°C pendant 17 heures. Le mélange réactionnel est concentré sous pression réduite (5 kPa), à une température voisine de 50°C. Au résidu solide obtenu, on ajoute 1,48 cm³ d'acide chlorhydrique aqueux normal, puis 10 cm³ d'eau. La solution obtenue est extraite par 5 fois 20 cm³ de dichlorométhane. Les extraits organiques sont réunis, puis concentrés sous pression réduite (5 kPa). On obtient 0,189 g d'acide (3R,4R)-4-[3-(6-méthoxyquinolin-4-yl)propyl]-1-(prop-2-ynyl)pipéridine-3-carboxylique, sous forme d'une meringue de couleur blanche.

Le (3R,4R)-4-[3-(6-methoxyquinolin-4-yl)propyl]-1-(prop-2-ynyl)pipéridine-3-carboxylate de -prop-2-ynyle peut être préparé de la manière suivante :

A une solution de 0,835 g de chlorhydrate d'acide (3R,4R)-4-[3-(6-méthoxyquinolin-4-yl)propyl]pipéridine-3-carboxylique, d ans 15 cm³ de diméthylformamide anhydre, on ajoute 0,95 g de carbonate de potassium, puis 0,36 cm³ de bromure de propargyle. Le mélange est agité sous atmosphère d'azote, à une température voisine de 70°C pendant 18 heures. On ajoute au mélange réactionnel 100 cm³ d'acétate d'éthyle et 100 cm³ d'eau distillée. La phase organique est décantée, puis lavée par 5 fois 40 cm³ d'eau, et 2 fois 50 cm³ d'une solution saturée de chlorure de sodium. La solution organique est séchée sur sulfate de magnésium, filtrée, puis mélangée sous pression réduite (5 kPa), à une température voisine de 40°C, jusqu'à concentration maximum. L'huile obtenue est purifiée par chromatographie sur une colonne de gel de silice (granulométrie 20-45 µ ; diamètre 2 cm ; hauteur 40 cm), en éluant, sous une pression d'azote de 50 kPa, par de l'acétate d'éthyle, et en recueillant des fractions de 50 cm³. Les fractions 17 à 21 sont réunies, concentrées sous pression réduite (5 kPa), à une température de 40°C. On obtient 0,300 g de (3R,4R)-4-[3-(6-methoxyquinolin-4-yl)propyl]-1-(prop-2-ynyl)-3-pipéridinecarboxylate de prop-2-ynyle, sous forme d'une huile de couleur jaune.

Le chlorhydrate de l'acide (3R,4R)-4-[3-(6-méthoxyquinolin-4-yl)propyl]pipéridine-3-carboxylique peut être préparé de la manière suivante :

8,8 g de d'acide (3R,4R)-1-benzoyl-4-[3-(6-méthoxyquinolin-4-yl)propyl]pipéridine-3-carboxylique sont chauffés, sous agitation, dans 200 cm³ d'acide chlorhydrique aqueux 5N, à une température voisine de 100°C pendant 48 heures. Le mélange réactionnel est concentré sous pression réduite (5 kPa) à une température voisine de 50°C. Le résidu est repris par 100 cm³ d'acétone. Le mélange est concentré sous pression réduite (5 kPa), à une température voisine de 60°C. Cette opération est répétée deux fois supplémentaires. Le résidu est enfin trituré dans 100 cm³ d'acétone, jusqu'à cristallisation. Après filtration des cristaux, et séchage au dessicateur sous pression réduite (10 kPa), on obtient 7,2 g de chlorhydrate d'acide (3R,4R)-4-[3-(6-méthoxyquinolin-4-yl)propyl]pipéridine-3-carboxylique, sous forme d'un solide beige fondant aux environs de 270°C (fusion pâteuse).

L'acide (3R,4R)-1-benzoyl-4-[3-(6-méthoxyquinolin-4-yl)propyl]pipéridine-3-carboxylique peut être préparé de la manière suivante :

25 g de (3R,4R)-1-benzoyl-4-[3-(6-méthoxyquinolin-4-yl)propyl]-3-vinyl pipéridine sont dissous dans un mélange de 250 cm³ de tétrachlorure de carbone et 250 cm³ d'acétonitrile. 51,3 g de métapériodate de sodium en solution dans 325 cm³ d'eau sont ajoutés à une température voisine de 20°C, sous bonne agitation, puis 0,27 g de trichlorure de ruthénium hydrate. La réaction, légèrement exothermique, est maintenue au voisinage de 30°C pendant 15 minutes après l'addition des réactifs. Le mélange est agité 2 heures à température ambiante. La suspension obtenue est filtrée, l'insoluble lavé par 5 fois 80 cm³ de dichlorométhane. Après agitation du filtrat, la phase organique est décantée, la phase aqueuse saturée par du chlorure de sodium, puis extraite par deux portions supplémentaires de 300 cm³ de dichlorométhane. Les extraits organiques réunis sont lavés à l'eau (3 fois 200 cm³), séchés sur sulfate de magnésium, filtrés sur papier, puis concentrés sous pression réduite ( 5 kPa), à une température voisine de 40°C. On obtient 23,2 g d'une huile que l'on purifie par chromatographie, à pression atmosphérique, sur gel de silice (granulométrie 20-45 µ; diamètre 6,5 cm ; hauteur 30 cm), en éluant par un mélange de dichlorométhane-méthanol (97/3 en volumes), et en recueillant des fractions de 400 cm³. Les fractions 4 à 8 sont réunies, puis concentrées sous pression réduite (5 kPa). On obtient 11,8 g d'une huile brune. Celle-ci est dissoute dans 60 cm³ d'acétonitrile portés au reflux pendant quelques minutes en présence de 0,5 g de charbon animal. Après filtration, la solution obtenue est refroidie. Le produit qui a cristallisé est essoré, lavé par 2 fois 10 cm³ d'acétonitrile. Le solide est séché au dessiccateur sous vide potassique (10 kPa). On obtient 8,8 g d'acide (3R,4R)-1-benzoyl-4-[3-(6-méthoxyquinolin-4-yl)propyl]pipéridine-3-carboxylique sous forme d'un solide beige fondant à 160°C.

La (3R,4R)-1-benzoyl-4-[3-(6-méthoxyquinolin-4-yl)propyl]-3-vinylpipéridine peut être préparée de la manière suivante :
A une solution agitée de 20,8 g de (3R,4R)-4-[3-(6-méthoxyquinolin-4-yl)propyl]-3-vinylpipéridine dans 270 cm³ de chloroforme, on ajoute 18,4 cm³ de triéthylamine, puis, en 1 heure, une solution de 7,2 cm³ de chlorure de benzoyle dans 50 cm³ de chloroforme. Après 1 heure 30 minutes d'agitation du mélange à une température voisine de 20°C, 100 cm³ d'eau distillée sont ajoutés au mélange réactionnel. La phase chloroformique est décantée, lavée par 2 fois 100 cm³ d'eau, puis séchée sur sulfate de magnésium. Après filtration sur papier, la solution chloroformique est concentrée sous pression réduite (5 kPa), à une température voisine de 40°C. On obtient 25 g de (3R,4R)-1-benzoyl-4-[3-(6-méthoxyquinolin-4-yl)propyl]-3-vinylpipéridine, sous forme d'huile brune.
La (3R,4R)-4-[3-(6-méthoxyquinolin-4-yl)propyl]-3-vinylpipéridine peut être obtenue par application de la méthode décrite dans la demande de brevet FR 2354771.

### Exemple 3

### Acide (3R,4R)-4-[3-(6-méthoxyquinolin-4-yl)propyl]-1-(3-phénylprop-2-ynyl) pipéridine-3-carboxylique

Une solution de 0,25 g de (3R,4R)-4-[3-(6-méthoxyquinolin-4-yl)propyl]-1-(3-phénylprop-2-ynyl)pipéridine-3-carboxylate d e m éthyle d ans 6 cm³ de méthanol est additionnée de 0,41 cm³ de soude aqueuse 5N, puis chauffée à une température voisine de 57°C pendant 18 heures 30 minutes en atmosphère inerte. Le mélange est refroidi puis concentré sous pression réduite (2 kPa), à une température voisine de 40°C. Le résidu est repris par 10 cm³ d'eau, acidifié par 2 cm³ d'acide chlorhydrique aqueux N, concentré sous pression réduite (2 kPa), à une température voisine de 45°C. Le résidu obtenu est trituré dans 10 cm³ d'un mélange de dichlorométhane-méthanol (90/10 en volumes), puis filtré. L'insoluble est lavé par 2 fois 10 cm³ de dichlorométhane. Les filtrats organiques réunis sont concentrés sous pression réduite (2 kPa), à une température voisine de 30°C. On obtient un résidu de 0,22 g que l'on agite dans un mélange de 20 cm³ d'eau et 15 cm³ de dichlorométhane. La phase aqueuse est décantée, puis extraite par 3 fois 10 cm³ de dichlorométhane. Cette phase aqueuse est concentrée à sec, sous pression réduite (5 kPa), à une température voisine de 40°C. Le résidu est trituré dans 10 cm³ d'un mélange dichlorométhane-méthanol (90/10 en volumes). L'insoluble est filtré, puis le gâteau est lavé par 5 cm³ du même mélange. Le filtrat est séché sur sulfate de magnésium, puis concentré sous pression réduite, dans les mêmes conditions que précédemment, et enfin séché sous pression partielle (13 Pa), à une température voisine de 40°C pendant 2 heures. On obtient 0,11 g d'acide (3R,4R)-4-[3-(6-méthoxyquinolin-4-yl)propyl]-1-(3-phénylprop-2-ynyl) pipéridine-3-carboxylique sous forme d'un solide meringué de couleur jaune-pâle, fondant aux environs de 166°C en devenant pâteux.

Spectre infra rouge (KBr): 2931; 2859 cm⁻¹ (ν CH₂); 3000 et 2750 cm⁻¹ (ν OH acide) ; 2800 et 1900 cm⁻¹ (ν N⁺H ( sel d'amine tertiaire + sel de quinoline)) ; 1719 cm⁻¹ (ν C=O acide) ; 1618 ;1601 ;1542 et 1492 cm⁻¹ (ν C=C noyaux aromatiques) ; 1275 cm⁻¹ (ν C-O acide) ; 1225 cm⁻¹ (νₐₛ C-O éther) ; 1022 cm⁻¹ (νₛ C-O éther) ; 846 cm⁻¹ (γ CH quinoline 4-6 disubstituée) ; 761 et 693 cm⁻¹ (γ CH phényl monosubstitué) .
Spectre de masse (IE- m/z) 442 (M⁺); 398 (M-CO₂)⁺; 327 (M-C₉H₇)⁺; 283 ; 327 (M-CO₂)⁺; 186 (C₁₂H₁₂ON⁺); 115 (C₉H₇⁺); 44 (CO₂⁺); 36 (HCl⁺ pic de base)

### (3R,4R)-4-[3-(6-méthoxyquinolin-4-yl)propyl]-1-(3-phénylprop-2-ynyl) pipéridine-3-carboxylate de méthyle

A une solution agitée de 0,7 g de (3R,4R)-4-[3-(6-méthoxyquinolin-4-yl)propyl]-1-(prop-2-ynyl)pipéridine-3-carboxylate de méthyle dans 12 cm³ d'acétonitrile, on ajoute, sous atmosphère inerte, à une température voisine de 20°C, 0,138 g de tétrakis(triphénylphosphine)palladium, 0,041 g de triphénylphosphine et 0,070 g d'iodure cuivreux. On ajoute ensuite 0,56 g d'iodobenzène, puis 0,51 cm³ de triéthylamine. Le mélange est agité pendant 22 heures à une température voisine de 20°C, puis filtré. Le gâteau est lavé par 3 fois 10 cm³ d'acétonitrile. Les filtrats réunis sont additionnés de 100 cm³ de dichlorométhane et 100 cm³ d'eau, puis agités. La phase organique est décantée, lavée par 3 fois 50 cm³ d'une solution saturée de chlorure de sodium. Après séchage sur sulfate de magnésium, puis filtration, la solution organique est mélangée sous pression réduite (2 kPa), à une température voisine de 40°C. On obtient 1,1 g d'une huile que l'on purifie par chromatographie sous pression d'argon (50 kPa), sur une colonne de gel de silice (granulométrie 40-63 µ ; diamètre 3 cm ; 65 g), en é luant par l'acétate d'éthyle, et en recueillant des fractions de 2,5 cm³. Les fractions 52 à 210 sont réunies, concentrées sous pression réduite (2 kPa), à une température voisine de 35°C. On obtient 0,64 g de (3R,4R)-4-[3-(6-méthoxyquinolin-4-yl)propyl]-1-(3-phénylprop-2-ynyl)pipéridine-3-carboxylate de méthyle sous forme d'une huile de couleur brun-clair.

Le (3R,4R)-4-[3-(6-méthoxyquinolin-4-yl)propyl]-1-(prop-2-ynyl)pipéridine-3-carboxylate de méthyle peut être préparé de la manière suivante :
A une suspension agitée de 10 g de chlorhydrate de (3R,4R)-4-[3-(6-méthoxyquinolin-4-yl)propyl]pipéridine-3-carboxylate de méthyle dans 100 cm³ de diméthylformamide anhydre, et sous atmosphère inerte, on ajoute, à une température voisine de 20°C, 14,7 cm³ de triéthylamine, puis au bout de 45 minutes, 3 cm³ de bromure de propargyle dilués dans 10 cm³ de diméthylformamide anhydre. Après 15 minutes d'agitation à une température voisine de 20°C, le mélange est chauffé pendant 4 heures à une température voisine de 45°C. Après refroidissement, le mélange réactionnel est versé dans un mélange de 250 cm³ d'acétate d'éthyle et 250 cm³ d'eau distillée. Le mélange est agité quelques minutes, puis la phase organique est décantée. La phase aqueuse est extraite par 2 fois 250 cm³d'acétate d'éthyle. Les phases organiques sont réunies, lavées par 3 fois 200 cm³ d'eau distillée, séchées sur sulfate de magnésium. Après filtration, puis mélange du solvant sous pression réduite (2 kPa), à une température voisine de 40°C, on obtient 7,8 g d'une huile que l'on purifie par chromatographie sous pression d'argon (50 kPa), sur une colonne de gel de silice (granulométrie 40-63 µ ; diamètre 7 cm ; 475 g), en éluant par l'acétate d'éthyle et en recueillant des fractions de 8 cm³. Les fractions 468 à 612 sont réunies, puis concentrées sous pression réduite (2 kPa), à une température voisine de 45°C. On obtient 4,7 g de (3R,4R)-4-[3-(6-méthoxyquinolin-4-yl)propyl]-1-(prop-2-ynyl)pipéridine-3-carboxylate de méthyle, sous forme d'huile de couleur orangée.

Le chlorhydrate de (3R,4R)-4-[3-(6-méthoxyquinolin-4-yl)propyl]pipéridine-3-carboxylate de méthyle peut être préparé de la manière suivante :
A une suspension agitée de 4,29 g d'acide (3R,4R)-4-[3-(6-méthoxyquinolin-4-yl)propyl]-1-(tert-butyloxycarbonyl)pipéridine-3-carboxylique dans 50 cm³ de méthanol et refroidie à une température voisine de -30°C par un bain réfrigérant d'acétone et de carboglace, on ajoute goutte à goutte 2 cm³ de chlorure de thionyle. La solution obtenue est ramenée à une température voisine de 20°C, et le mélange réactionnel est agité pendant 16 heures à cette température. Après mélange de la solution sous pression réduite (5 kPa), à une température voisine de 40°C, le résidu obtenu est trituré dans 30 cm³ environ d'éther diisopropylique. Les cristaux obtenus sont essorés, lavés par 2 fois 10 cm³ d'éther diisopropylique, puis séchés à l'air. On obtient 4,20 g de chlorhydrate de (3R,4R)-4-[3-(6-méthoxyquinolin-4-yl)propyl] pipéridine-3-carboxylate de méthyle, sous forme d'un solide de couleur jaune clair, fondant en se ramollissant à une température voisine de 140°C.

L'acide (3R,4R)-4-[3-(6-méthoxyquinolin-4-yl)propyl]-1-(tert-butyloxycarbonyl) pipéridine-3-carboxylique peut être préparé de la manière suivante :
3 g de (3R,4R)-4-[3-(6-méthoxyquinolin-4-yl)propyl]-3-vinylpipéridine sont dissous dans 3 cm³ d'acétone. A cette solution, refroidie à une température voisine de 5°C par un bain de glace et d'acétone, on ajoute sous agitation 14,5 cm³ d'acide sulfurique 3M préalablement refroidis à cette même température. A la solution obtenue, on ajoute en 30 minutes une solution de 4,64 g de permanganate de sodium monohydraté dans 25 cm³ d'eau, en maintenant la température entre 0 et 7°C. Le mélange réactionnel est agité pendant 4 heures à une température comprise entre 10 et 17°C. La masse réactionnelle est filtrée ; l'insoluble est lavé par 2 fois 10 cm³ d'eau. On obtient d'une part une solution de couleur orangée, et d'autre part une masse minérale de couleur noire. La solution orangée est amenée à pH 10 par addition de 4,6 g de carbonate de sodium. Le mélange est filtré : on obtient une solution (1) et un insoluble minéral (2). La masse minérale de couleur noire est agitée pendant 30 minutes dans 20 cm³ d'eau après que le pH ait été amené à 12 par addition de 2 cm³ de lessive de potasse. Après filtration du mélange, on obtient une solution (3) et un insoluble minéral (4). Les insolubles (2) et (4) sont agités pendant 15 minutes dans 15 cm³ d'eau additionnés de 3 cm³ de lessive de potasse. La suspension est filtrée. On obtient une solution (5). Les solutions aqueuses (1), (3) et (5) sont réunies, additionnées de 2,31 g de di-tertiobutyl dicarbonate, et agitées pendant 15 heures à une température voisine de 20°C. Le mélange est extrait par 6 fois 10 cm³ d'acétate d'éthyle. Les phases organiques sont réunies, lavées par 20 cm³ d'eau, puis par 20 cm³ d'une solution aqueuse saturée de chlorure de sodium. Après séchage sur sulfate de sodium, filtration, puis concentration sous pression réduite (5 kPa), à une température voisine de 35°C, on obtient 2,86 g d'acide (3R,4R)-4-[3-(6-méthoxyquinolin-4-yl)propyl]-1-(tert-butyloxycarbonyl) pipéridine-3-carboxylique, sous forme d'un solide de couleur beige, devenant pâteux à 154°C.

La (3R,4R)-4-[3-(6-méthoxyquinolin-4-yl)propyl]-3-vinylpipéridine peut être obtenue par application de la méthode décrite dans la demande de brevet FR 2354771.

### Exemple 4

### Acide (3R,4R)-4-[3-(6-méthoxyquinolin-4-yl)propyl]-1-[3-(2-fluorophényl)prop-2-ynyl]pipéridine-3-carboxylique

Une solution de 0,28 g de(3R,4R)-4-[3-(6-méthoxyquinolin-4-yl)propyl]-1-[3-(2-fluorophényl)prop-2-ynyl]pipéridine-3-carboxylate de méthyle dans 10 cm³ de méthanol est additionnée de 0,44 cm³ de soude aqueuse 5N, puis chauffée à une température voisine de 57°C pendant 20 heures. Après refroidissement, la solution est mélangée sous pression réduite (2 kPa), à une température voisine de 40°C. On obtient un résidu de 0,41 g qui est repris par 20 cm³ d'eau additionnés de 3,5 cm³ d'acide chlorhydrique 1N. Après extraction de la phase aqueuse par 5 fois 15 cm³ de dichlorométhane, la phase aqueuse est concentrée à sec sous pression réduite (5 kPa), à une température voisine de 40°C. Le résidu obtenu est trituré dans 10 cm³ d'un mélange de dichlorométhane-méthanol (90/10 en volumes). L'insoluble est filtré, puis le gâteau est lavé par 10 cm³ du même mélange. Le filtrat est séché sur sulfate de magnésium, puis concentré sous pression réduite (5 kPa), à une température voisine de 40°C, et enfin séché sous vide (13 Pa), à une température voisine de 40°C pendant 2 heures. On obtient 0,15 g d'acide (3R,4R)-4-[3-(6-méthoxyquinolin-4-yl)propyl]-1 [3-(2-fluorophényl)prop-2-ynyl]pipéridine-3-carboxylique sous forme d'un solide meringué de couleur jaune-pâle, fondant aux environs de 154°C, en devenant pâteux.

Spectre infra rouge (KBr) : 3057 cm⁻¹ (ν CH aromatiques) ; 2933 ; 2864 cm⁻¹ (ν CH₂); 3000 ; 2750 cm⁻¹ (ν. OH acide) ; 2800 et 1900 cm⁻¹ (ν N⁺H ( sel d'amine tertiaire + sel de quinoline)) ; 1722 cm⁻¹ (ν C=O acide) ; 1618 ;1601 ;1542 et 1493 cm⁻¹ (ν C=C noyaux aromatiques) ; 1275 cm⁻¹ (ν C-O acide) ; 1217 cm⁻¹ (νₐₛ C-O éther) ; 1022 cm⁻¹ (νₛ C-O éther) ; 847 cm⁻¹ (γ CH quinoline 4-6 disubstituée) ; 765 cm⁻¹ (γ CH phényl orthodisubstitué).

Spectre de masse : (DCI) m/z=461 MH⁺

### (3R,4R)-4-[3-(6-méthoxyquinolin-4-yl)propyl]-1-[3-(2-fluorophényl)prop-2-ynyl]pipéridine-3-carboxylate de méthyle

A une solution agitée de 0,7 g de (3R,4R)-4-[3-(6-méthoxyquinolin-4-yl)propyl]-1-(prop-2-ynyl)pipéridine-3-carboxylate de méthyle dans 14 cm³ d'acétonitrile, on ajoute, à une température voisine de 20°C et sous atmosphère inerte, 0,041 g de tétrakis(triphénylphosphine)palladium et 0,070 g d'iodure cuivreux. On ajoute ensuite 0,32 cm³ de fluoro-1 iodo-2 benzène et 0,51 cm³ de triéthylamine. Le mélange est agité à une température voisine de 20°C pendant 20 heures. Le mélange réactionnel est filtré, le gâteau lavé par 3 fois 10 cm³ d'acétonitrile. Les filtrats réunis sont repris sous agitation par un mélange de 100 cm³ de dichlorométhane et 100 cm³ d'eau. La phase organique est décantée, lavée par 3 fois 50 cm³ d'une solution saturée de chlorure de sodium, séchée sur sulfate de magnésium, puis, après filtration, concentration sous pression réduite (2 kPa), à une température voisine de 35°C. Le résidu obtenu est purifié par chromatographie sous pression d'argon (50 kPa), sur une colonne de gel de silice (granulométrie 40-63 µ; diamètre 4 cm ; hauteur 14 cm), en éluant par de l'acétate d'éthyle, et en recueillant des fractions de 2 cm³. Les fractions 33 à 160 sont réunies, puis mélangées sous pression réduite (2 kPa), à une température voisine de 40°C. On obtient 0,56 g de (3R,4R)-4-[3-(6-méthoxyquinolin-4-yl)propyl]-1-[3-(2-fluorophényl)prop-2-ynyl]pipéridine-3-carboxylate de méthyle, sous forme d'une huile de couleur brun-clair.

Le (3R,4R)-4-[3-(6-méthoxyquinolin-4-yl)propyl]-1-(prop-2-ynyl)pipéridine-3-carboxylate de méthyle a été préparé dans les conditions de l'exemple 3.

### Exemple 5

### Dichlorhydrate de l'acide (3R,4R)-4-[3-(6-méthoxyquinolin-4-yl)propyl]-1-[3-(3-fluorophényl)prop-2-ynyl]pipéridine-3-carboxylique

Une solution de 0,66 g de (3R,4R)-4-[3-(6-méthoxyquinolin-4-yl)propyl]-1-[3-(3-fluorophényl)prop-2-ynyl]pipéridine-3-carboxylate de méthyle est additionnée de 10,5 cm³ d'une solution aqueuse d'acide chlorhydrique 6N, puis chauffée à une température voisine de 100°C pendant 3 heures à l'issue desquelles sont additionnés 3,5 cm³ supplémentaires de solution aqueuse d'acide chlorhydrique 6N. Après 4 heures, le mélange réactionnel est refroidi à une température voisine de 40°C puis concentré sous pression réduite (2 kPa), à une température voisine de 60°C. Le résidu obtenu est repris dans 10 cm³ d'eau et 8 cm³ de dichlorométhane, puis décanté. La phase aqueuse est extraite 2 fois par 6 cm³ de dichlorométhane. La solution aqueuse est concentrée à sec sous pression réduite (1 kPa), à une température voisine de 50°C. Le résidu obtenu est dissout dans 5 cm³ d'un mélange dichlorométhane-méthanol (90/10 en volumes), séché sur sulfate de sodium, puis, après filtration, le solvant est mélangeé sous pression réduite (1 kPa), à une température voisine de 40°C. Le résidu est repris dans un mélange de propanol-2 et d'éther d'isopropyle, dissous à chaud, puis filtré sur papier. Le filtrat est refroidi à une température voisine de 25°C puis mélangé sous pression réduite (1 kPa), à une température voisine de 45°C. Le résidu est repris dans 10 cm³ d'un mélange dichlorométhane-méthanol (90/10 en volumes) et reconcentré dans les mêmes conditions. On obtient 0,44 g de dichlorhydrate de l'acide (3R,4R)-4-[3-(6-méthoxyquinolin-4-yl)propyl]-1-[3-(3-fluorophényl)prop-2-ynyl] pipéridine-3-carboxylique, sous forme d'une meringue de couleur jaune, fondant aux environs de 222°C, en devenant pâteux.

Spectre de R.M.N. ¹H (400 MHz, (CD₃)₂SO d6, δ en ppm) : de 1,20 à 2,15 (mf : 6H) ; 2,35 (mf : 1H) ; de 3,00 à 3,90 (mt : 7H) ; 4.02 (s : 3H) ; 4,40 (s large : 2H) ; de 7,25 à 7,55 (mt : 4H) ; 7,60 (s large : 1H) ; 7,75 (dd, J = 9 et 2 Hz : 1H) ; 7,82 (d, J = 5 Hz : 1H) ; 8,27 (d, J = 9 Hz : 1H) ; 8,97 (d, J = 5 Hz :1) ; de 11,15 à 11,45 (mf étalé : 1H).

### (3R,4R)-4-[3-(6-méthoxyquinolin-4-yl)propyl]-1-[3-(3-fluorophényl)prop-2-ynyl]pipéridine-3-carboxylate de méthyle

A une solution agitée de 1,2 g d e (3R,4R)-4-[3-(6-méthoxyquinolin-4-yl)propyl]-1-[prop-2-ynyl]pipéridine-3-carboxylate de méthyle dans 25 cm³ d'acétonitrile, on ajoute, sous atmosphère inerte et à une température voisine de 20°C, 0,07 g de triphénylphosphine, 0,237 g de tétrakis(triphénylphosphine)palladium et 0,12 g d'iodure cuivreux. On ajoute ensuite 0,56 cm³ de 1-fluoro-3-iodo-benzène et 0,88 cm³ de triéthylamine. Après agitation pendant 20 heures à une température voisine de 20°C, le mélange réactionnel est filtré sur Célite et le gâteau est lavé par de l'acétonitrile. Le filtrat est mélangeé sous pression réduite (2 kPa), à une température voisine de 35°C. Le résidu obtenu est repris par un mélange de 80 cm³ de dichlorométhane et 80 cm³ d'eau. Après décantation, la phase organique est lavée par 3 fois 50 cm³ d'une solution saturée de chlorure de sodium. La solution organique est séchée sur sulfate de magnésium, filtrée, puis concentrée sous pression réduite (2 kPa), à une température voisine de 40°C. On obtient 1,91 g d'une huile que l'on purifie par chromatographie sous pression d'argon (50 kPa), sur une colonne de gel de silice (granulométrie 40-63 µ ; diamètre 3 cm ; 77 g), en éluant par l'acétate d'éthyle, et en recueillant des fractions de 5 cm³. Les fractions contenant le produit attendu sont réunies, puis mélangées sous pression réduite (2 kPa), à une température voisine de 40°C. On obtient 1,08 g de (3R,4R)-4-[3-(6-méthoxyquinolin-4-yl)propyl]-1-[3-(3-fluorophényl)prop-2-ynyl]pipéridine-3-carboxylate de méthyle, sous forme d'une huile de couleur jaune.

Le (3R,4R)-4-[3-(6-méthoxyquinolin-4-yl)propyl]-1-[prop-2-ynyl]pipéridine-3-carboxylate de méthyle a été préparé dans les conditions de l'exemple 3.

### Exemple 6

### Acide (3R,4R)-4-[3-(6-méthoxyquinolin-4-yl)propyl]-1-[3-(thièn-3-yl)prop-2-ynyl]pipéridine-3-carboxylique

Une solution de 0,6 g de (3R,4R)-4-[3-(6-méthoxyquinolin-4-yl)propyl]-1-[3-(thièn-3-yl)prop-2-ynyl] pipéridine-3-carboxylate de méthyle dans 10 cm³ de dioxanne est additionnée de 3,9 cm³ de soude aqueuse 1N, puis chauffée à une température voisine de 65°C pendant 16 heures Le mélange est refroidi puis la phase organique est extraite par 3 fois 50 cm³ d'acétate d'éthyle. La phase aqueuse est acidifiée par 3,9 cm³ d'acide chlorhydrique 1N. La solution est reprise par 10 cm³ d'une solution aqueuse saturée de bicarbonate de sodium puis la phase organique est extraite par 2 fois 20 cm³ d'acétate d'éthyle et 2 fois 20 cm³ de dichlorométhane, séchée sur du sulfate de sodium, filtrée et concentrée à sec sous pression réduite (2 kPa), à une température voisine de 45°C. On obtient 0,32 g d'acide (3R,4R)-4-[3-(6-méthoxyquinolin-4-yl)propyl]-1-[3-(thièn-3-yl)prop-2-ynyl] pipéridine-3-carboxylique sous forme d'une meringue de couleur blanche.

Spectre de R.M.N. ¹H (300 MHz, (CD₃)₂SO d6, δ en ppm) : de 1,20 à 1,90 (mt : 7H) ; 2,43 (mt : 1H) ; de 2,50 à 3,00 (mt : 4H) ; 3,04 (t large, J = 7,5 Hz : 2H) ; 3,55 (s : 2H) ; 3,93 (s : 3H) ; 7,15 (dd, J = 5 et 1,5 Hz : 1H) ; 7,32 (d, J = 5 Hz : 1H) ; de 7,35 à 7,50 (mt : 2H) ; 7,62 (dd, J = 5 et 3 Hz : 1H) ; 7,75 (dd, J = 3 et 1,5 Hz : 1H) ; 7,92 (d, J = 9 Hz : 1H) ; 8,62 (d, J = 5 Hz : 1H) ; de 12,00 à 13,00 (mf très étalé : 1H).

### (3R,4R)-4-[3-(6-méthoxyquinolin-4-yl)propyl]-1-[3-(thièn-3-yl)prop-2-ynyl] pipéridine-3-carboxylate de méthyle

On opère comme à l'exemple 2 à partir de 1 g de (3R,4R)-4-[3-(6-méthoxyquinolin-4-yl)propyl]-1-[3-yl-prop-2-ynyl] pipéridine-3-carboxylate de méthyle, de 0,9 g de 3-iodothiophène, de 0,2 g de tétrakis(triphénylphosphine)palladium, 0,1 g d'iodure cuivreux, 0,060 g de triphénylphosphine et 0,75 cm³ de triéthylamine. Le résidu obtenu est purifié par chromatographie à pression atmosphérique sur une colonne de gel de silice (granulométrie 40-63 µ; diamètre 3,5 cm ; hauteur de la colonne 35 cm), en éluant par de l'acétate d'éthyle et en recueillant des fractions de 40 cm³. Les fractions 18 à 49 sont réunies, puis mélangées sous pression réduite (5 kPa), à une température voisine de 40°C. On obtient 0,6 g de (3R,4R)-4-[3-(6-méthoxyquinolin-4-yl)propyl]-1-[3-(thièn-3-yl)prop-2-ynyl] pipéridine-3-carboxylate de méthyle sous forme d'une huile brune.

Le 3-iodothiophène peut-être préparé selon N.A. PETASIS et coll., SYNLETT. 1999, 141.

### Exemple 7 :

### Le (3R,4R)-4-[3-oxo-3-(6-méthoxyquinolin-4-yl)propyl]pipéridine-3-carboxylate de méthyle peut être préparé de la manière suivante :

Une solution de 19,4 g d'acide (3R,4R)-4-[3-oxo-3-(6-méthoxyquinolin-4-yl)propyl]-1-(t-butyloxocarbonyl)pipéridine-3-carboxylique (teneur à 80 %) dans 355 cm³ de méthanol est refroidie à une température voisine de -30°C. On ajoute sous agitation 7,7 cm³ de chlorure de thionyle en maintenant la température entre -25 et -30°C. Après l'addition, on maintient le mélange aux environs de -30°C pendant 30 minutes, puis on laisse revenir la température aux environs de 20°C. Après agitation à température ambiante pendant 19 heures, le mélange réactionnel est concentré sous pression réduite (5 kPa), à une température voisine de 30°C. Le résidu obtenu est repris par 300 cm³ d'eau additionnés de 200 cm³ de dichlorométhane, puis agité. La phase organique est décantée ; la phase aqueuse est à nouveau extraite par 200 cm³ de dichlorométhane. La solution aqueuse est amenée à pH 8 par addition progressive d'hydrogénocarbonate de sodium solide. Après extraction de la solution alcaline obtenue par 3 fois 200 cm³ de dichlorométhane, les extraits organiques réunis sont lavés par 2 fois 200 cm³ d'eau, puis séchés sur sulfate de magnésium. Après filtration sur papier, la solution organique est concentrée sous pression réduite (5 kPa), à une température voisine de 40°C. On obtient 4,51 g de (3R,4R)-4-[3-oxo-3-(6-méthoxyquinolin-4-yl)propyl]pipéridine-3-carboxylate de méthyle sous forme d'une laque de couleur brune.

L'acide (3R,4R)-4-[3-oxo-3-(6-méthoxyquinolin-4-yl)propyl]-1-(tert-butoxy-carbonyl)pipéridine-3-carboxylique peut être préparé de la manière suivante :

Une solution de 36 g de (3R,4R)-4-[3-oxo-3-(6-méthoxyquinolin-4-yl)propyl]-3-vinylpipéridine dans 54 cm³ d'acétone est refroidie à une température voisine de 0°C.

On ajoute en 15 minutes, sous agitation, 150 cm³ d'acide sulfurique 3 M, en maintenant la température entre 0 et 5°C. On abaisse la température au voisinage de 0°C et l'on ajoute goutte à goutte au mélange une solution de 32 g de permanganate de sodium dans 200 cm³ d'eau distillée. Le mélange réactionnel est agité 45 minutes supplémentaires à une température comprise entre 10 et 15°C, puis on laisse remonter la température au voisinage de 20°C. Après agitation 3 heures à cette température, la masse réactionnelle est refroidie à une température voisine de 0°C, puis on ajoute lentement 160 cm³ de lessive de potasse à 38 % à une température inférieure à 10°C. Après 30 minutes d'agitation à une température voisine de 10°C, le mélange est filtré. Le gâteau est repris dans 300 cm³ d'eau additionnés de 15 cm³ de lessive de potasse à 38 %, et agité pendant 20 minutes. Après filtration, puis lavage du gâteau par 2 fois 200 cm³ d'eau distillée, les filtrats sont réunis puis additionnés de 24 g de di-tertiobutyldicarbonate. La solution est agitée à une température voisine de 2 0°C pendant 15 heures. Après addition d'un litre d'acétate d'éthyle, et agitation, le mélange est décanté, la phase aqueuse séparée puis amenée à pH 5 par addition de 38 cm³ d'acide chlorhydrique aqueux concentré à 37 %. Le mélange est extrait à nouveau par 5 fois 1 litre d'acétate d'éthyle. Les extraits sont réunis puis lavés par 2 fois 1 litre d'eau saturée en chlorure de sodium. La solution organique est séchée sur sulfate de magnésium, filtrée sur papier, concentrée sous pression réduite (5 kPa), à une température voisine de 40°C. On obtient 21,2 g d'acide (3R,4R)-4-[3-oxo-3-(6-méthoxyquinolin-4-yl)propyl]-1-(t-butyloxycarbonyl)pipéridine-3-carboxylique, sous forme d'un solide brun fondant à 114°C en devenant pâteux.

La (3R,4R)-4-[3-oxo-3-(6-méthoxyquinolin-4-yl)propyl]-3-vinylpipéridine peut être obtenue par application de la méthode décrite dans la demande de brevet FR 2354771.

### Exemple 8

### Dichlorhydrate de l'acide (3R,4R)-4-[3-(R,S)-hydroxy-(6-méthoxyquinolin-4-yl)propyl]-1-[3-(3-fluorophényl)prop-2-ynyl]pipéridine-3-carboxylique

Une solution de 1,3 g de (3R,4R)-4-[3-(R,S)-hydroxy-(6-méthoxyquinolin-4-yl)propyl]-1-[3-(3-fluorophényl)prop-2-ynyl]pipéridine-3-carboxylate de méthyle dans 15 cm³ de dioxanne est additionnée de 2,1 cm³ de soude aqueuse 5N, puis chauffée à une température voisine de 60°C pendant 16 heures. Après refroidissement, solution obtenue est concentrée sous pression réduite (2 kPa), à une température voisine de 45°C. Le résidu obtenu est purifié par chromatographie sous pression d'argon (50 kPa) sur une colonne de gel de silice Amicon (granulométrie 20-45 µ ; diamètre 4 cm ; hauteur 24 cm), en éluant par un mélange de chloroforme, méthanol et ammoniac (24/12/1 en volumes) et en recueillant des fractions de 30 cm³. Les fractions 41 à 58 sont réunies, puis mélangées sous pression réduite (2 kPa), à une température voisine de 40°C. On obtient 1,0 g d'une meringue qui est mise en solution dans 8 cm³ d'acétone. Cette solution est ajoutée à 5 cm3 d'une solution éther chlorhydrique 1N. Après 5 minutes d'agitation, les solide obtenu est filtré puis séché à poids constant sous pression réduite (2 kPa), à une température voisine de 40°C. On obtient 820 mg de dichlorhydrate de l'acide (3R,4R)-4-[3-(R,S)-hydroxy-(6-méthoxyquinolin-4-yl)propyl]-1-[3-(3-fluorophényl)prop-2-ynyl] pipéridine-3-carboxylique sous forme d'un solide blanc hygroscopique.

Spectre de R.M.N. ¹H (300 MHz, (CD₃)₂SO d6 avec ajout de quelques gouttes de CD₃COOD d4, δ en ppm) : de 1,35 à 2,30 et de 2,90 à 3,65 (mts : 12H) ; 3,99 (s : 3H) ; de 4,20 à 4,50 (mt : 2H) ; de 5,40 à 5,60 (mt : 1H); de 7,25 à 7,70 (mt : 5H); de 7,70 à 7,80 (mt : 1H); 7,99 (mt : 1H); 8,20 (d, J = 9 Hz : 1H); 9,01 (d largue, J = 5 Hz : 1H).

### (3R,4R)-4-[3-(R,S)-hydroxy-(6-méthoxyquinolin-4-yl)propyl]-1-[3-(3-fluorophényl)prop-2-ynyl]pipéridine-3-carboxylate de méthyle

A une solution agitée de 2 g de (3R,4R)-4-[3-oxo-(6-méthoxyquinolin-4-yl)propyl]-1-[3-(3-fluorophényl)prop-2-ynyl]pipéridine-3-carboxylate de méthyle dans 30 cm³ de méthanol, on ajoute en deux portions, à une température voisine de 20°C et sous atmosphère inerte, 0,193 g de borohydrure de sodium. Le mélange est ensuite agité pendant 3 heures à une température voisine de 20°C. Puis, on ajoute 10 cm³ d'eau distillée en maintenant la même température. Le mélange, est concentré sous pression réduite (5 kPa), à une température voisine de 40°C. Le résidu obtenu est repris dans 25 cm³ d'eau distillée. Le mélange est extrait par 150 cm³ au total de dichlorométhane. Les phases organiques sont réunies, puis lavées à trois reprises par 30 cm³ d'eau puis séchées sur sulfate de magnésium. Après filtration sur papier, puis mélange du solvant sous pression réduite (5 kPa), à une température voisine de 40°C, on obtient 1,8 g d'une meringue qui est purifiée par chromatographie sous pression atmosphérique sur une colonne de gel de silice Amicon (granulométrie 20-45 µ; diamètre 3 cm ; hauteur 30 cm), en éluant par de l'acétate d'éthyle et en recueillant des fractions de 50 cm³. Les fractions 17 à 28 sont réunies, puis mélangées sous pression réduite (2 kPa), à une température voisine de 40°C. On obtient ainsi 1,4 g de (3R,4R)-4-[3-(R,S)-hydroxy-(6-méthoxyquinolin-4-yl)propyl]-1-[3-(3-fluoro phényl)prop-2-ynyl]pipéridine-3-carboxylate de méthyle sous la forme d'une meringue jaune clair.

Le (3R,4R)-4-[3-oxo-(6-méthoxyquinolin-4-yl)propyl]-1-[3-(3-fluorophényl)prop-2-ynyl] pipéridine-3-carboxylate de méthyle peut être obtenu de la manière suivante :
A une solution agitée de 1,97 g de (3R,4R)-4-[3-oxo-(6-méthoxyquinolin-4-yl) propyl]-1-(prop-2-ynyl)pipéridine-3-carboxylate de méthyle dans 40 cm³ d'acétonitrile, on ajoute, sous atmosphère inerte, à une température voisine de 20°C, 0,404 g de tétrakis(triphényl-phosphine) palladium, 0,118 g de triphénylphosphine et 0,191 g d'iodure cuivreux. On ajoute ensuite 0,90 cm³ de 3-fluoroiodobenzène, puis 1,40 cm³ de triéthylamine. Le mélange est agité pendant 15 heures à une température voisine de 20°C, puis filtré sur celite. Le gâteau est lavé par 3 fois 10 cm³ d'acétonitrile. Les filtrats réunis sont concentrés sous pression réduite (2 kPa), à une température voisine de 40°C. On obtient 4,3 g d'une huile que l'on purifie par chromatographie sous pression atmosphérique, sur une colonne de gel de silice (granulométrie 20-45 µ; diamètre 3 cm ; hauteur 60 cm), en éluant par l'acétate d'éthyle, et en recueillant des fractions de 50 cm³. Les fractions 21 à 42 sont réunies, mélangées sous pression réduite (2 kPa), à une température voisine de 35°C. On obtient 2 g de (3R,4R)-4-[3-oxo-(6-méthoxyquinolin-4-yl)propyl]-1-[3-(3-fluoro phényl)prop-2-ynyl]pipéridine-3-carboxylate de méthyle sous forme d'une huile de couleur jaune.

Le (3R,4R)-4-[3-oxo-(6-méthoxyquinolin-4-yl)propyl]-1-(prop-2-ynyl)pipéridine-3-carboxylate de méthyle peut être obtenu de la manière suivante :
A une suspension agitée de 15 g de dichlorhydrate de (3R,4R)-4-(3-oxo-(6-methoxy-quinolin-4-yl)-propyl]-pipéridine-3-carboxylate de méthyle, dans 150 cm³ de diméthylformamide anhydre, et sous atmosphère inerte, on ajoute, à une température voisine de 20°C, 19,6 cm³ de triéthylamine, puis au bout de 45 minutes, 3,95 cm³ de bromure de propargyle dilués dans 5 cm³ de diméthylformamide anhydre. Après 15 minutes d'agitation à une température voisine de 20°C, le mélange est chauffé pendant 4 heures à une température voisine de 45°C. Après refroidissement, le mélange réactionnel est versé dans un mélange de 150 cm³ d'acétate d'éthyle et 150 cm³ d'eau distillée. Le mélange est agité quelques minutes, puis la phase organique est décantée. La couche aqueuse est extraite par 2 fois 150 cm³ d'acétate d'éthyle. Les phases organiques sont réunies, lavées par 3 fois 200 cm³ d'eau distillée, séchées sur sulfate de sodium. Après filtration, puis mélange du solvant sous pression réduite (2 kPa), à une température voisine de 40°C, on obtient 13,8 g d'une huile que l'on purifie par chromatographie sous pression d'argon (50 kPa), sur une colonne de gel de silice (granulométrie 40-63 µ ; diamètre 5 cm ; hauteur 34 cm), en éluant par un mélange d'acétate d'éthyle et de cyclohexane (9/1 en volumes) jusqu'à la fraction 40 puis par de l'acétate d'éthyle pour les suivantes et en recueillant des fractions de 50 cm³. Les fractions 23 à 70 sont réunies, puis mélangées sous pression réduite (2 kPa), à une température voisine de 45°C. On obtient 8,2 g (3R,4R)-4-[3-oxo-(6-méthoxyquinolin-4-yl)propyl]-1-(prop-2-ynyl)pipéridine-3-carboxylate de méthyle, sous forme d'huile de couleur orangée.
Le dichlorhydrate de (3R,4R)-4-[3-oxo-3-(6-methoxy-quinolin-4-yl)-propyl]-pipériding-3-carboxylate de méthyle, peut être obtenu comme décrit à l'exemple 7

### Exemple 9

### Dichlorhydrate de l'acide (3R,4R)-4-[3-(R,S)-hydroxy-(6-méthoxyquinolin-4-yl)propyl]-1-[3-(thién-2-yl)prop-2-ynyl]pipéridine-3-carboxylique

Une solution de 1,3 g de (3R,4R)-4-[3-(R,S)-hydroxy-(6-méthoxyquinolin-4-yl)propyl]-1-[3-(thién-2-yl)prop-2-ynyl]pipéridine-3-carboxylate de méthyle dans 13 cm³ de dioxanne est additionnée de 2 cm³ de soude aqueuse 5N, puis chauffée à une température voisine de 60°C pendant 3 heures. Après refroidissement, la solution obtenue est concentrée sous pression réduite (2 kPa), à une température voisine de 45°C. Le résidu obtenu est repris dans 75 cm³ d'eau distillée. La phase aqueuse est lavée par 75 cm³ au total de dichlorométhane. La phase aqueuse est concentrée jusqu'à un volume voisin de 10 cm³, refroidie à une température voisine de 5°C puis acidifiée à un pH voisin de 1 par addition d'acide chlorhydrique 5N. Après 12 heures d'agitation à une température voisine de 20°C, la phase aqueuse est concentrée sous pression réduite (2 kPa), à une température voisine de 45°C. Le résidu obtenu est repris dans 50 cm³ d'acétone. La solution obtenue est concentrée sous pression réduite (2 kPa), à une température voisine de 45°C. Le résidu obtenu est purifié par chromatographie sous pression d'argon (50 kPa) sur une colonne de gel de silice (granulométrie 20-45 µ ; diamètre 3 cm ; hauteur 30 cm), en éluant par un mélange de chloroforme, méthanol et ammoniac (24/12/1 en volumes) et en recueillant des fractions de 15 cm³. Les fractions 19 à 53 sont réunies, puis mélangées sous pression réduite (2 kPa), à une température voisine de 40°C. On obtient 0,8 g d'une meringue qui est mise en solution dans 7 cm³ de dichlorométhane. Cette solution est ajoutée à 9 cm³ d'une solution d'éther chlorhydrique 1N. Après 5 minutes d'agitation à une température voisine de 20°C, le solide obtenu est filtré, lavé par 50 cm³ au total d'oxyde de diéthyle puis séché à poids constant sous pression réduite (2 kPa), à une température voisine de 40°C. On obtient 0,92 g de dichlorhydrate de l'acide (3R,4R)-4-[3-(R,S)-hydroxy-(6-méthoxyquinolin-4-yl)propyl]-1-[3-(thién-2-yl)prop-2-ynyl] pipéridine-3-carboxylique sous la forme de cristaux de couleur blanc cassé.

Spectre de R.M.N. ¹H (400 MHz, CD₃OD d4, δ en ppm) : de 1,50 à 2,30 (mt : 7H) ; 3,06 et 3,11 (2 mts : 1H) ; de 3,15 à 3,95 (mt : 4H) ; 4,03 et 4,04 (2s : 3H) ; de 4,15 à 4,45 (mt : 2H) ; 5,55 et 5,66 (2 mts : 1H) ; 7,04 (mt : 1H) ; 7,37 (mt : 1H) ; 7,51 (d, J = 5 Hz : 1H) ; 7,62 et 7,69 (2s larges : 1H) ; 7,77 (dd, J = 9 et 2 Hz : 1H) ; de 8,10 à 8,20 (mt : 2H) ; 8,92 et 8,94 (mt : 1H).

### (3R,4R)-4-[3-(R,S)-hydroxy-(6-méthoxyquinolin-4-yl)propyl]-1-[3-(thién-2-yl)prop-2-ynyl]pipéridine-3-carboxylate de méthyle

A une solution agitée de 1,6 g (3R,4R)-4-[3-oxo-(6-méthoxyquinolin-4-yl)propyl]-1-[3-(thién-2-yl)prop-2-ynyl]pipéridine-3-carboxylate de méthyle dans 15 cm³ de méthanol, on ajoute en une portion, à une température voisine de 20°C et sous atmosphère inerte, 0,15 g de borohydrure de sodium. Le mélange est ensuite agité pendant 2,5 heures à une température voisine de 25°C. Puis, on ajoute goutte à goutte en approximativement 10 minutes, 15 cm³ d'eau distillée en maintenant à une température voisine de 15°C. Le mélange est concentré sous pression réduite (5 kPa), à une température voisine de 40°C. Le résidu obtenu est repris dans 20 cm³ d'eau distillée. Le mélange est extrait par 100 cm³ au total de dichlorométhane. Les phases organiques sont réunies, séchées sur sulfate de sodium puis mélangées sous pression réduite (5 kPa), à une température voisine de 40°C. Le solide obtenu est purifié par chromatographie sous pression d'argon (50 kPa) sur une colonne de gel de silice (granulométrie 20-45 µ; diamètre 3 cm ; hauteur 35 cm), en éluant par un mélange d'acétate d'éthyle et de cyclohexane (8/2 en volumes) et en recueillant des fractions de 15 cm³. Les fractions 38 à 59 sont réunies, puis mélangées sous pression réduite (2 kPa), à une température voisine de 40°C. On obtient ainsi 1,3 g (3R,4R)-4-[3-(R,S)-hydroxy-(6-méthoxyquinolin-4-yl)propyl]-1-[3-(thién-2-yl)prop-2-ynyl] pipéridine-3-carboxylate de sous la forme d'une meringue.

Le (3R,4R)-4-[3-oxo-(6-méthoxyquinolin-4-yl)propyl]-1-[3-(thién-2-yl)prop-2-ynyl] pipéridine-3-carboxylate de méthyle peut être obtenu de la manière suivante :
A une solution agitée de 1,97 g de (3R,4R)-4-[3-oxo-(6-méthoxyquinolin-4-yl) propyl]-1-(prop-2-ynyl)pipéridine-3-carboxylate de méthyle dans 40 cm³ d'acétonitrile, on ajoute, sous atmosphère inerte, à une température voisine de 20°C, 0,404 g de tétrakis (triphénylphosphine)palladium, 0,118 g de triphénylphosphine et 0,191 g d'iodure cuivreux. On ajoute ensuite 0,84 cm³ de 2-iodothiophène, puis 1,40 cm³ de triéthylamine. Le mélange est agité pendant 48 heures à une température voisine de 20°C, puis filtré sur celite. Le gâteau est lavé par de l'acétonitrile. Les filtrats réunis sont concentrés sous pression réduite (2 kPa), à une température voisine de 40°C. On obtient 4,2 g d'une huile que l'on purifie par chromatographie sous pression d'argon (50 kPa), sur une colonne de gel de silice (granulométrie 20-45 µ; diamètre 3 c m ; hauteur 30 cm), en éluant par un mélange d'acétate d'éthyle et de cyclohexane (8/2 en volumes), et en recueillant des fractions de 15 cm³. Les fractions 16 à 32 sont réunies, mélangées sous pression réduite (2 kPa), à une température voisine de 35°C. On obtient 1,6 g de (3R,4R)-4-[3-oxo-(6-méthoxyquinolin-4-yl) propyl]-1-[3-(thién-2-yl)prop-2-ynyl]pipéridine-3-carboxylate de méthyle sous forme d'une huile de couleur orange.
(3R,4R)-4-[3-oxo-(6-méthoxyquinolin-4-yl)propyl]-1-(prop-2-ynyl)pipéridine-3-carboxylate de méthyle peut être obtenu comme indiqué à l'exemple 8.

### Exemple 10

### Monochlorhydrate de l'acide (3R,4R)-4-[3-(R,S)-fluoro-(6-méthoxyquinolin-4-yl) propyl]-1-[3-(thién-2-yl)prop-2-ynyl]pipéridine-3-carboxylique

Une solution de 1,6 g de (3R,4R)-4-[3-(R,S)-fluoro-(6-méthoxyquinolin-4-yl)propyl]-1-[3-(thién-2-yl)prop-2-ynyl]pipéridine-3-carboxylate de méthyle dans 16 cm³ de dioxanne est additionnée de 2,5 cm³ de soude aqueuse 5N, puis chauffée à une température voisine de 60°C pendant 50 heures. A près refroidissement, la solution obtenue est concentrée sous pression réduite (2 kPa), à une température voisine de 45°C. Le résidu obtenu est repris dans 50 cm³ d'acétone puis concentré sous pression réduite (2 kPa), à une température voisine de 45°C. Le résidu obtenu est purifié par chromatographie sous pression d'argon (50 kPa), sur une colonne de gel de silice (granulométrie 20-45 µ; diamètre 3,5 cm ; hauteur 34 cm), en éluant par un mélange de chloroforme, méthanol et ammoniac (24/12/1 en volumes) et en recueillant des fractions de 15 cm³. Les fractions 16 à 28 sont réunies, puis mélangées sous pression réduite (2 kPa), à une température voisine de 40°C. Le solide est repris dans de l'acétone puis concentré sous pression réduite (2 kPa), à une température voisine de 45°C. On obtient 0,72 g d'une meringue qui est mise en solution dans 8 cm³ de dichlorométhane. Cette solution est ajoutée à 8 cm³ d'une solution d'éther chlorhydrique 1N. Après 15 minutes d'agitation à une température voisine de 20°C, le solide obtenu est filtré, puis séché à poids constant sous pression réduite (2 kPa), à une température voisine de 40°C. On obtient 0,74 g de monochlorhydrate de l'acide (3R,4R)-4-[3-(R,S)-fluoro-(6-méthoxyquinolin-4-yl)propyl]-1-[3-(thién-2-yl)prop-2-ynyl]pipéridine-3-carboxylique sous la forme de cristaux de couleur blanc cassé fondant à 166°C.

Spectre de R.M.N. ¹H (400 MHz, (CD₃)₂SO d6, à une température de 383K, δ en ppm) : 1,68 (mt : 2H); 1,87 (mt : 1H); 1,89 (mt : 1H); de 2,05 à 2,25 (mt : 3H); de 3,05 à 3,45 (mt : 5H); 3,98 (s : 3H); 4,25 (AB limite : 2H); 6,31 (mt, J_{HF} = 16 Hz : 1H); 7,12 (dd, J = 5 et 3,5 Hz : 1H); de 7,35 à 7,45 (mt : 2H) ; de 7,45 à 7,55 (mt : 2H) ; 7,64 (d, J = 5 Hz : 1H); 8,05 (d, J = 9 Hz : 1H); 8,80 (d, J = 5 Hz : 1H).

### (3R,4R)-4-[3-(R,S)-fluoro-(6-méthoxyquinolin-4-yl)propyl]-1-[3-(thién-2-yl)prop-2-ynyl]pipéridine-3-carboxylate de méthyle

A une solution de 3,4 g de (3R,4R)-4-[3-(R,S)-hydroxy-(6-méthoxyquinolin-4-yl)propyl]-1-[3-(thién-2-yl)prop-2-ynyl]pipéridine-3-carboxylate de méthyle sous atmosphère inerte dans 50 cm³ de dichlorométhane on additionne goutte à goutte en approximativement 15 minutes une solution de 1,14 cm³ de diéthylamino trisulfure de soufre dans 10 cm³ de dichlorométhane. Après 9 heures d'agitation à une température voisine de 20°C, le mélange réactionnel est refroidi à une température voisine de 10°C et 60 cm³ d'une solution saturée en hydrogénocarbonate de sodium est ajoutée en approximativement 15 minutes. La phase organique est décantée puis lavée avec 300 cm³ au total d'eau distillée. La phase organique est séchée sur sulfate de sodium, filtrée puis concentrée sous pression réduite (2 kPa) à une température voisine de 40°C. Le résidu obtenu est purifié par chromatographie sous pression d'argon (50 kPa) sur une colonne de gel de silice (granulométrie 20-45 µ ; diamètre 4 cm ; hauteur 31 cm), en éluant par un mélange d'acétate d'éthyle et de cyclohexane (1/1 en volumes) et en recueillant des fractions de 15 cm³. Les fractions 34 à 65 sont réunies, puis mélangées sous pression réduite (2 kPa), à une température voisine de 40°C. Le solide est repris dans de l'acétone puis concentré sous pression réduite (2 kPa), à une température voisine de 45°C. On obtient 1,8 g de 0.74 g de (3R,4R)-4-[3-(R,S)-fluoro-(6-méthoxyquinolin-4-yl)propyl]-1-[3-(thién-2-yl)prop-2-ynyl]pipéridine-3-carboxylase de méthyle sous la forme d'une huile jaune.

Le (3R,4R)-4-[3-(R,S)-hydroxy-(6-méthoxyquinolin-4-yl)propyl]-1-[3-(thién-2-yl) prop-2-ynyl]pipéridine-3-carboxylate de méthyle peut être préparé comme décrit à l'exemple 9.

### Exemple 11

### Dichlorhydrate de l'acide (3R,4R)-4-[3-(R,S)-fluoro-(6-méthoxyquinolin-4-yl) propyl]-1-[3-(3-fluorophényl)prop-2-ynyl]pipéridine-3-carboxylique

Une solution de 1,48 g de (3R,4R)-4-[3-(R,S)-fluoro-(6-méthoxyquinolin-4-yl)propyl]-1-[3-(3-fluorophényl)prop-2-ynyl]pipéridine-3-carboxylate de méthyle dans 15 cm³ de dioxanne et de 2,4 cm³ de soude aqueuse 5N, est chauffée à une température voisine de 60°C pendant 17 heures. Après refroidissement, la solution obtenue est concentrée sous pression réduite (2 kPa), à une température voisine de 45°C. Le résidu obtenu est repris dans 50 cm³ d'acétone puis concentré sous pression réduite (2 kPa), à une température voisine de 45°C. Le résidu obtenu est purifié par chromatographie sous pression atmosphérique sur une colonne de gel de silice (granulométrie 20-45 µ ; diamètre 2,8 cm; volume: 150 cm³), en éluant par un mélange de dichlorométhane, méthanol et ammoniac (120/20/3 en volumes) et en recueillant d es fractions de 20 cm³. Les fractions contenant le produit attendu sont réunies, puis mélangées sous pression réduite (2 kPa), à une température voisine de 40°C. Le solide est repris dans 25 cm³ d'acétone puis on ajoute 5 cm³ d'une solution d'éther chlorhydrique 1N et 20 cm³ d'oxyde de diéthyle. Après 2 heures d'agitation à une température voisine de 20°C, le solide obtenu est filtré, puis séché à poids constant sous pression réduite (2 kPa), à une température voisine de 40°C. On obtient 0.6 g de dichlorhydrate de l'acide (3R,4R)-4-[3-(R,S)-fluoro-(6-méthoxyquinolin-4-yl)propyl]-1-[3-(3-fluorophényl)prop-2-ynyl]pipéridine-3-carboxylique sous la forme d'un solide.

Spectre de R.M.N. ¹H (400 MHz, (CD₃)₂SO d6, à une température de 373K, δ en ppm) : de 1,55 à 1,95 (mt : 3H) ; de 2,00 à 2,30 (mts : 4H) ; de 3,15 à 3,50 (mt : 5H) ; 3,99 (s : 3H) ; 4,31 (AB limite : 2H) ; 6,38 (mt, J_{HF} = 47 Hz : 1H) ; de 7,25 à 7,55 (mt : 5H) ; 7,55 (dd, J = 9 et 2,5 Hz : 1H) ; 7,60 (d, J = 5 Hz : 1H) ; 8,12 (d, J = 9 Hz : 1H); 8,84 (d, J = 5 Hz:1H).

### (3R,4R)-4-[3-(R,S)-fluoro-(6-méthoxyquinolin-4-yl)propyl]-1-[3-(3-fluorophényl) prop-2-ynyl]pipéridine-3-carboxylate de méthyle

A une solution de 4,2 g de (3R,4R)-4-[3-(R,S)-hydroxy-(6-méthoxyquinolin-4-yl) propyl]-1-[3-(3-fluorophényl)prop-2-ynyl]pipéridine-3-carboxylate de méthyle sous atmosphère inerte dans 75 cm³ de dichlorométhane refroidie à une température voisine de 15°C, on additionne goutte à goutte en approximativement 15 minutes une solution de 1,4 cm³ de diéthylamino trisulfure de soufre dans 5 cm³ de dichlorométhane. Après 9 heures d'agitation à une température voisine de 25°C, On ajoute 100 cm³ d'une solution saturée en hydrogéno-carbonate de sodium en veillant à ce que la température de dépasse pas 25°C. La phase organique est décantée puis lavée avec 100 cm³ au total d'eau distillée. La phase organique est séchée sur sulfate de magnésium, filtrée puis concentrée sous pression réduite (2 kPa), à une température voisine de 40°C. Le résidu obtenu est purifié par chromatographie sous pression atmosphérique sur une colonne de gel de silice (granulométrie 20-45 µ ; diamètre 4 cm ; hauteur 42 cm), en éluant par de l'acétate d'éthyle et en recueillant des fractions de 70 cm³. Les fractions 19 à 35 sont réunies, puis mélangées sous pression réduite (2 kPa), à une température voisine de 40°C. Le solide est repris dans de l'acétone puis concentré sous pression réduite (2 kPa), à une température voisine de 45°C. On obtient 3,1 g (3R,4R)-4-[3-(R,S)-fluoro-(6-méthoxyquinolin-4-yl)propyl]-1-[3-(3-fluorophényl)prop-2-ynyl]pipéridine-3-carboxylate de méthyle sous la forme d'une huile jaune.

Le (3R,4R)-4-[3-(R,S)-hydroxy-(6-méthoxyquinolin-4-yl)propyl]-1-[3-(3-fluoro phényl)prop-2-ynyl]pipéridine-3-carboxylate de méthyle peut être préparé comme décrit à l'exemple 8.

### Exemple 12

### (3R,4R)-3-hydroxyméthyl-4-[3-(R,S)-hydroxy-3-(6-méthoxyquinolin-4-yl) propyl]-1-[3-(thièn-2-yl)prop-2-ynyl] pipéridine

A un mélange sous agitation de 0,5 g de (3R,4R)-4-[3-(R,S)-hydroxy-3-(6-méthoxyquinolin-4-yl)propyl]-1-[3-(thièn-2-yl)prop-2-ynyl] pipéridine-3-carboxylate de méthyle dans 10 cm³ de toluène, refroidie à -20°C, on ajoute 4,2 cm³ d'une solution à 20 % d'hydrure de diisobutyl aluminium dans le toluène. L'agitation est maintenue 3 heures à cette température puis on ajoute 15 cm³ d'une solution saturée de chlorure d'ammonium, maintient l'agitation 15 minutes et laisse remonter la température à une température proche de 20°C. La phase aqueuse est décantée, séchée sur sulfate de magnésium anhydre, filtrée et concentrée à sec sous pression réduite (2 kPa). Le résidu obtenu est purifié par chromatographie sur colonne de gel de silice (granulométrie 20-45 µ ; diamètre 2 cm ; hauteur 20 cm), en éluant, sous une pression de 50 kPa d'azote par du dichlorométhane puis un mélange de dichlorométhane et de méthanol (95/5 en volumes) et en recueillant des fractions de 30 cm³. Les fractions 14 à 16 sont réunies, puis mélangées sous pression réduite (5 kPa), à une température voisine de 40°C. Le résidu obtenu est repris par 5 cm³ de dichlorométhane et filtré. Le filtrat est concentré à sec sous pression réduite (2 kPa). On obtient 0,17 g de (3R,4R)-3-hydroxyméthyl-4-[3-(R,S)-hydroxy-3-(6-méthoxyquinolin-4-yl)propyl]-1-[3-(thièn-2-yl)prop-2-ynyl] pipéridine sous forme d'une gomme.

Spectre de R.M.N. ¹H (400 MHz, (CD₃)₂SO d6, δ en ppm) : de 1,25 à 1,90 (mt : 8H); 2,22 (mt ; 2H) ; de 2,65 à 2,90 (mt : 2H); de 3,35 à 3,60 (mt : 4H) ; 3,91 et 3,93 (2s : 3H) ; 4,29 (mf: 1H); 5,28 (mt : 1H); 5,50 et 5,52 (2d, J = 4,5 Hz : 1H); 7,07 (mt : 1H); 7,28 (dd, J = 4 et 1 Hz : 1H) ; de 7,35 à 7,45 (mt : 2H) ; de 7,50 à 7,60 (mt : 2H) ; 7,95 (d, J = 9 Hz : 1H); 8,71 (d, J = 5 Hz : 1H).

Le (3R,4R)-4-[3-(R,S)-hydroxy-3-(6-méthoxyquinolin-4-yl)propyl]-1-[3-(thièn-2-yl) prop-2-ynyl] pipéridine-3-carboxylate de méthyle est obtenu comme décrit à l'exemple 9.

### Exemple 13

### Acide (3R,4R)-4-[3-(R,S)-hydroxy-3-(6-méthoxyquinolin-4-yl)propyl]-1-[3-(thién-3-yl)prop-2-ypyl]pipéridine-3-carboxylique

Une solution de 0,73 g de (3R,4R)-4-[3-(R,S)-hydroxy-3-(6-méthoxyquinolin-4-yl) propyl]-1-[3-(thién-3-yl)prop-2-ynyl]pipéridine-3-carboxylate de méthyle, 7,6 cm³ de dioxanne et 1,22 cm³ de soude aqueuse 5N est chauffée à une température voisine de 60°C, sous agitation pendant 3 heures 30 minutes. Après refroidissement à une température voisine de 20°C, le mélange réactionnel est concentré sous pression réduite (5 kPa) à une température voisine de 40°C. Le résidu obtenu est purifié par chromatographie sous pression d'argon (55 kPa) sur une colonne de gel de silice (granulométrie 40-63µ ; diamètre 5 cm ; volume silice 120 cm³) en éluant d'abord par 1120 cm³ d'un mélange de dichlorométhane-méthanol-acétonitrile (92/8/7 en volumes). On élue ensuite par 224 cm³ du même mélange (mais de composition 92/12/7 en volumes), puis 2 24 cm³ du même mélangé ( de composition 92/16/7 en volumes), puis 400 cm³ de mélange dichlorométhane-méthanol (50/50 en volumes) et enfin 400 cm³ de méthanol pur. On recueille une fraction de 112 cm³, puis des fractions de 7 cm³. Les fractions 173 à 468 sont réunies puis évaporées sous pression réduite (2 kPa) à une température voisine de 35°C. On obtient une meringue que l'on sèche sous pression réduite (30 Pa) à une température voisine de 30°C pendant 4 à 5 heures. On obtient 0,33 g d' acide (3R,4R)-4-[3-(R,S)-hydroxy-3-(6-méthoxyquinolin-4-yl)propyl]-1-[3-(thién-3-yl)prop-2-ynyl]pipéridine-3-carboxylique sous forme d'un solide d'aspect meringué, de couleur beige, fondant à 113-115°C.

Spectre de R.M.N. ¹H (300 MHz, (CD₃)₂SO d6, δ en ppm). On observe un mélange de deux diastéréoisomères :
de 1,40 à 1,95 et de 2,30 à 2,95 (mts : 12H) ; 3,53 et 3,55 (2s : 2H en totalité) ; 3,90 et 3,92 (2s : 3H en totalité) ; 5,24 (mt : 1H) ; de 5,35 à 5,65 (mf étalé : 1H) ;7,14 (d, J = 5 Hz : 1H) ; de 7,30 à 7,45 (mt : 2H) ; 7,53 et 7,55 (2d, J = 5 Hz : 1H en totalité) ; 7,60 (dd, J = 5 et 3 Hz : 1H) ; 7,74 (mt : 1H) ; 7,94 (d, J = 9,5 Hz : 1H) ; 8,70 (d, J = 5 Hz : 1H).

### (3R,4R)-4-[3-(R,S)-Hydroxy-3-(6-méthoxyquinolin-4-yl)propyl]-1-[3-(thién-3-yl)prop-2-ynyl]pipéridine-3-carboxylate de méthyle

On opère comme pour la préparation du (3R,4R)-4-[3-(6-méthoxyquinolin-4-yl) propyl]-1-[3-(thién-3-yl)prop-2-ynyl]pipéridirie-3-carboxylate de méthyle, à partir de 1,2 g de (3R,4R)-4-[3-(R,S)-hydroxy-3-(6-méthoxyquinolin-4-yl)propyl]-1-(prop-2-ynyl)pipéridine-3-carboxylate de méthyle dans 20 cm³ d'acétonitrile, 0,067 g de triphénylphosphine, 0,227 g de tétrakis(triphénylphosphine)palladium, 0,115 g d'iodure cuivreux et 10 cm³ d'acétonitrile puis 0,84 cm³ de triéthylamine, 0,95 g de 3-iodothiophène et 5 cm³ d'acétonitrile. Le mélange réactionnel est filtré puis le gâteau est lavé par 30 cm³ d'acétonitrile. Le filtrat est évaporé sous pression réduite (2 kPa) à une température voisine de 40°C. Le résidu obtenu est repris par 100 cm³ de dichlorométhane. La solution organique résultante est lavée par 3 fois 50 cm³ d'une solution saturée de chlorure de sodium, séchée sur sulfate de sodium, filtrée puis concentrée sous pression réduite dans les mêmes conditions que ci-dessus. Le résidu obtenu est purifié par chromatographie sous une pression d'argon de 55 kPa, sur une colonne de gel de silice (granulométrie 40-63µ; diamètre 4 cm ; volume silice 220 cm³) en éluant par de l'acétate d'éthyle. On recueille une fraction de 180 cm³, puis des fractions de 10 cm³. Les fractions 73 à 300 sont réunies puis concentrées sous pression réduite (2 kPa) à une température voisine de 45°C. On obtient 0,96 g d'un produit que l'on remet en réaction avec 0,76 g de 3-iodothiophène, 0,092 g d'iodure cuivreux, 0,054 g de triphénylphosphine, 0,67 cm³ de triéthylamine et 0,181 g de tétrakis(triphénylphosphine)palladium dans 27 cm³ d'acétonitrile à une température voisine de 20°C pendant 16 heures. Le mélange réactionnel est filtré ; le gâteau est lavé par 30 cm³ d'acétonitrile. Le filtrat est concentré sous pression réduite (2 kPa) à une température voisine de 40°C. Le résidu obtenu est solubilisé dans 50 cm³ de dichlorométhane ; la solution est lavée par 3 fois 25 cm³ d'une solution saturée de chlorure de sodium, décantée puis séchée sur sulfate de sodium, filtrée et concentrée sous pression réduite (2 kPa) à une température voisine de 35°C. On obtient 1,15 g d'un résidu que l'on purifie par chromatographie sous une pression d'argon de 55 kPa, sur une colonne de gel de silice (granulométrie 40-63µ; diamètre 4 cm ; volume silice 120 cm³) en éluant par l'acétate d'éthyle. On recueille d'abord une fraction de 110 cm³ puis des fractions de 8 cm³. Les fractions 68 à 260 sont réunies, concentrées sous pression réduite (2 kPa) à une température voisine de 45°C. On obtient 0,73 g de (3R,4R)-4-[3-(R,S)-hydroxy-3-(6-méthoxyquinolin-4-yl)propyl]-1-[3-(thién-3-yl)prop-2-ynyl]pipéridine-3-carboxylate de méthyle, sous forme d'une meringue de couleur jaune.

Spectre infra rouge (CCl₄) 3600-3200 cm⁻¹ νOH; 2950 cm⁻¹ νCH aliphatiques; 1739 cm⁻¹ ν C=O; 1241 cm⁻¹ ν C-O éther 626 cm⁻¹ γ CH thiophène

### (3R,4R)-4-[3-(R,S)-Hydroxy-3-(6-méthoxyquinolin-4-yl)propyl]-1-(prop-2-ynyl)pipéridine-3-carboxylate de méthyle

Une suspension de 2,45 g de (3R,4R)-4-[3-(R,S)-hydroxy-3-(6-méthoxyquinolin-4-yl) propyl]pipéridine-3-carboxylate de méthyle, 2,1 g de carbonate de potassium, 0,95 g d'iodure de potassium, 0,6 cm³ de bromure de propargyle dans 50 cm³ d'acétonitrile est agitée pendant 40 heures à une température voisine de 20°C, en atmosphère inerte. Après filtration de la masse réactionnelle, le filtrat est évaporé sous pression réduite (5 kPa) à une température voisine de 40°C. Le résidu obtenu est purifié par chromatographie sur une colonne de gel de silice (granulométrie 20-45µ ; diamètre 2,5 cm ; masse 50 g) en éluant par de l'acétate d'éthyle et en recueillant des fractions de 60 cm³. Les fractions 6 à 12 sont réunies puis concentrées sous pression réduite (5 kPa) à une température voisine de 40°C. On obtient 1,35 g de (3R,4R)-4-[3-(R,S)-hydroxy-3-(6-méthoxyquinolin-4-yl)propyl]-1-(prop-2-ynyl)pipéridine-3-carboxylate de méthyle, sous forme d'une gomme.

Spectre infra rouge (CCl₄) 3600-3200 cm⁻¹ νOH; 3311 cm⁻¹ ν CH acétylénique ; 2950 cm⁻¹ νCH aliphatiques; 1740 cm⁻¹ ν C=O; 1242 cm⁻¹ ν C-O éther

Le 3-iodothiophène peut être préparé selon N.A PETASIS et coll., SYNLETT., 141, (1988).

### Exemple 14 :

### (3R,4R)]-4-[3-(6-Méthoxyquinolin-4-yl)propyl]pipéridine-3-acétate de méthyle

Un mélange de 2,8 g d'acide (3R,4R)-4-[3-(6-méthoxyquinolin-4-yl)propyl]-1-(tert.butyloxycarbonyl)pipéridine-3-acétique dans 100 cm³ de méthanol anhydre additionnés d'1 cm³ d'acide sulfurique à 95 % est chauffé, sous agitation, à une température voisine de l'ébullition pendant 2 heures. Après refroidissement à une température voisine de 20°C la masse réactionnelle est évaporée sous pression réduite (5 kPa) à une température voisine de 40°C, puis au résidu obtenu on ajoute 20 cm³ d'une solution aqueuse saturée d'hydrogénocarbonate de sodium. Le mélange est extrait par 4 fois 20 cm³ de dichlorométhane. Les extraits réunis sont séchés sur sulfate de magnésium, filtrés puis évaporés sous pression réduite (5 kPa) à une température voisine de 40°C. On obtient 2,25 g de (3R,4R)]-4-[3-(6-méthoxyquinolin-4-yl) propyl]pipéridine-3-acétate de méthyle, sous forme d'une huile de couleur brune.

Spectre infra rouge (CH₂Cl₂) 2954 ,2865 cm⁻¹ ν CH aliphatiques ; 2788 cm⁻¹ ν CH₂ N(CH₂)₂ ;1736 cm⁻¹ νC=O ester;1242,1227 cm⁻¹ ν C-O éther; 848 cm⁻¹ γCH quinoline

### Acide (3R,4R)-4-[3-(6-méthoxyquinolin-4-yl)propyl]-1-(tert.butyloxycarbonyl) pipéridine-3-acétique

A une solution de 2 g de (3R,4R)-4-[3-(6-méthoxyquinolin-4-yl)propyl]-1-(tert.butyloxycarbonyl)pipéridine-3-acétaldéhyde dans 100 cm³ d'acétone, on ajoute sous agitation et sous atmosphère inerte à une température voisine de 20°C 1,48 g de permanganate de potassium préalablement dissous dans 45 cm³ d'eau distillée, puis 220 cm³ d'acétone. Le mélange obtenu est agité pendant 2 heures à une température voisine de 20°C, puis après refroidissement à une température comprise entre 0 et 5°C, on ajoute une solution de 5 g de sulfite de sodium dans 150 cm³ d'eau. Le précipité brun de dioxyde de manganèse est filtré sur célite, puis l'acétone est évaporée sous pression réduite (5 kPa) à une température voisine de 40°C. On ajoute au mélange réactionnel la quantité suffisante d'acide citrique pour obtenir un pH de 4-5. Le mélange est extrait par 2 fois 100 cm³ d'acétate d'éthyle. Les extraits réunis sont séchés sur sulfate de magnésium, filtrés puis évaporés sous pression réduite (5 kPa) à une température voisine de 40°C. On obtient 2,8 g d'acide (3R,4R)-4-[3-(6-méthoxyquinolin-4-yl)propyl]-1-(tert.butyloxycarbonyl)pipéridine-3-acétique, sous forme d'une meringue de couleur blanche.

Spectre infra rouge (KBr) 2977, 2932 ,2868 cm⁻¹ νCH aliphatiques ; 3000-2200 cm⁻¹ ν OH acide 1734 cm⁻¹ νC=O acide;1689 cm⁻¹ νC=O carbamate ; 1391,1365 cm⁻¹ δₐₛ CH₃ ;1246 cm⁻¹ ν C-O éther; 1170 cm⁻¹ ν C-O carbamate ; 848 cm⁻¹ γCH quinoline

### (3R,4R)-4-[3-(6-Méthoxyquinolin-4-yl)propyl]-1-(tert.butyloxycarbonyl) pipéridine-3-acétaldéhyde

A une solution de 3,5 cm³ de chlorure d'oxalyle dans 80 cm³ de dichlorométhane refroidie à une température voisine de -60°C, on ajoute successivement sous agitation et sous atmosphère d'azote une solution de 5,6 cm³ de diméthylsulfoxyde dissous dans 80 cm³ de dichlorométhane, puis 13,5 g de (3R,4R)-3-(2-hydroxyéthyl)-4-[3-(6-méthoxyquinolin-4-yl)propyl]-1-(tert.butyloxycarbonyl)pipéridine dissous dans 80 cm³ de dichlorométhane, enfin 26,5 cm³ de triéthylamine dissous dans 80 cm³ de dichlorométhane. La solution obtenue est maintenue pendant 1 heure au voisinage de -60°C, puis 3 h ½ à une température voisine de 20°C. Après dilution par 150 cm³ de dichlorométhane, le mélange réactionnel est lavé par 2 fois 300 cm³ d'eau. La solution organique décantée est séchée sur sulfate de magnésium, filtrée, puis évaporée sous pression réduite (5 kPa) à une température voisine de 40°C. On obtient 12,7 g d'une huile brune que l'on reprend par 400 cm³ d'éther diéthylique. La solution résultante est lavée par 2 fois 300 cm³ d'eau, puis 1 fois par 300 cm³ d'une solution aqueuse d'acide citrique à 5 %, enfin par 2 fois 300 cm³ d'eau. La solution organique est séchée sur sulfate de magnésium, puis concentrée sous pression réduite (5 kPa) à une température voisine de 40°C. On obtient 7,73 g de (3R,4R)-4-[3-(6-méthoxyquinolin-4-yl)propyl]-1-(tert.butyloxycarbonyl)pipéridine-3-acétaldéhyde, sous forme d'une gomme collante de couleur jaune.

Spectre infra rouge (CCl₄) 2978, 2932 ,2864 cm⁻¹ν CH aliphatiques ;2717 cm⁻¹ ν CH aldéhyde ; 1729 cm⁻¹ νC=O aldéhyde;1694 cm⁻¹ νC=O carbamate ; 1391,1366 cm⁻¹ δₐₛ CH₃ ;1242 cm⁻¹ ν C-O éther; 1158 cm⁻¹ ν C-O carbamate ; 844 cm⁻¹ γCH quinoline

### (3R,4R)-3-(2-Hydroxyéthyl)-4-[3-(6-méthoxyquinolin-4-yl)propyl]-1-(tert.butyloxycarbonyl)pipéridine

Une solution de 2 g de (3R,4R)-4-[3-(6-méthoxyquinolin-4-yl)propyl]-1-(tert.butyloxycarbonyl)-3-vinylpipéridine et de 0,72 cm³ de triéthylamine borane dans 10 cm³ de toluène est agitée sous atmosphère inerte à une température voisine de 110°C pendant 10 heures. La masse réactionnelle est concentrée à sec sous pression réduite (5 kPa) à une température voisine de 60°C. On obtient 2,1 g d'une meringue de couleur orangée que l'on solubilise dans 9 cm³ d'acétone et auxquels on ajoute 1,9 cm³ d'acide chlorhydrique aqueux à 5 %, Après agitation du mélange pendant 20 minutes à une température voisine de 20°C, la masse réactionnelle est concentrée sous pression réduite (5 kPa) à une température voisine de 40°C. Le résidu obtenu est repris par 7,5 cm³ de tétrahydrofuranne, 6,2 cm³ de soude aqueuse à 30 % et 7,5 cm³ de peroxyde d'hydrogène en solution aqueuse à 30 %. Le mélange est chauffé pendant 3 heures à une température voisine du reflux. Après refroidissement, le mélange réactionnel est agité avec 30 cm³ de chloroforme à une température voisine de 20°C.

La phase aqueuse est décantée ; la phase organique est lavée par 3 fois 30 cm³ d'eau, puis 1 fois 20 cm³ d'une solution saturée de chlorure de sodium. Après séchage sur sulfate due magnésium la solution organique est concentrée sous pression réduite (5 kPa) à une température voisine de 40°C. On obtient 1,7 g d'une huile de couleur jaune-brun que l'on purifie par chromatographie à pression atmosphérique sur une colonne de gel de silice (granulométrie 40-63 µ; diamètre 3,5 cm ; masse 60 g) en éluant par un mélange de dichlorométhane-méthanol (95/5 en volumes) et en recueillant des fractions de 20 cm³. Les fractions 4 et 5 sont réunies, évaporées sous pression réduite (5 kPa) à une température voisine de 40°C. On obtient 0,95 g de (3R,4R)-3-(2-hydroxyéthyl)-4-[3-(6-méthoxyquinolin-4-yl)propyl]-1-(tert.butyloxy-carbonyl)pipéridine, sous forme d'une meringue de couleur jaune-orangé.

Spectre infra rouge (KBr) 3550-3100 cm⁻¹ ν OH alcool ; 2972,2931,2865 cm⁻¹ ν CH aliphatiques ;1690 cm⁻¹ νC=O carbamate ;1391,1365 cm⁻¹ δₐₛ CH₃ ;1244,1228 cm⁻¹ ν C-O éther ;1158 cm⁻¹ ν C-O carbamate ;1031cm⁻¹ ν C-O alcool ; 845 cm⁻¹ γCH quinoline

### (3R,4R)-4-[3-(6-Méthoxyquinolin-4-yl)propyl]-1-(tert.butyloxycarbonyl)-3-vinylpipéridine

A une suspension de 5 g de chlorhydrate de (3R,4R)-4-[3-(6-méthoxyquinolin-4-yl)propyl]-3-vinylpipéridine dans 50 cm³ de dichlorométhane, on ajoute sous agitation à une température voisine de 20°C 4 cm³ de triéthylamine, puis 3,15 g de di-tertiobutyl dicarbonate. Après 45 minutes la solution obtenue est lavée par 2 fois 30 cm³ d'eau, séchée sur sulfate de magnésium puis concentrée sous pression réduite (5 kPa) à une température voisine de 40°C. On obtient 6 g de (3R,4R)-4-[3-(6-méthoxyquinolin-4-yl)propyl]-1-(tert.butyloxycarbonyl)-3-vinylpipéridine, sous forme d'une huile de couleur brune.

Spectre infra rouge (CH₂Cl₂) 2972, 2933, 2860 cm⁻¹ ν CH aliphatiques ;1680 cm⁻¹ νC=O carbamate ;1391,1365 cm⁻¹ δₐₛ CH₃ ;1244,1228 cm⁻¹ ν C-O éther ;1165 cm⁻¹ ν C-O carbamate ; 845 cm⁻¹ γCH quinoline

Le chlorhydrate de (3R,4R)-4-[3-(6-méthoxyquinolin-4-yl)propyl]-3-vinylpipéridine peut être obtenu par application de la méthode décrite dans la demande de brevet FR 2 354 771.

### Exemple 15

### Dichlorhydrate de l'acide (3R,4R)-4-[3-(6-méthoxyquinolin-4-yl)propyl]-1-[3-(thién-2-yl)prop-2-ynyl]pipéridine-3-acétique.

A une solution agitée de 0,43 g de (3R,4R)-4-[3-(6-méthoxyquinolin-4-yl)propyl]-1-[3-(thién-2-yl)prop-2-ynyl]pipéridine-3-acétate de méthyle dans 10 cm³ de dioxanne, sous atmosphère inerte, on ajoute 0,8 cm³ d'une solution aqueuse de soude 5N. Le mélange est chauffé pendant 20 heures à une température voisine de 60°C, puis après refroidissement à une température voisine de 20°C, le mélange réactionnel est évaporé sous pression réduite (5 kPa) à une température voisine de 45°C. On obtient 0,8 g d'un produit que l'on purifie par chromatographie à pression atmosphérique sur une colonne de gel de silice (granulométrie 20-45µ ; diamètre 3 cm ; hauteur silice 30 cm) en éluant par un mélange de chloroforme-méthanol-ammoniaque à 28% (12/3/0,5 en volumes) et en recueillant des fractions de 25 cm³. Les fractions 14 à 29 sont réunies puis évaporées sous pression réduite (5 kPa) à une température voisine de 40°C. On obtient 0,350 g d'un solide blanc dont on fait le chlorhydrate de la façon suivante : le solide obtenu est dissous dans 5 cm³ de dichlorométhane, puis la solution obtenue est ajoutée sous agitation et sous atmosphère inerte à 10 cm³ d'éther chlorhydrique 1N. Le mélange est dilué par 100 cm³ d'éther anhydre. On obtient une suspension blanche que l'on agite pendant 3 heures à une température voisine de 20°C. Les cristaux sont filtrés, lavés par 5 fois 10 cm³ d'éther diéthylique, séchés sous pression partielle (10 Pa) à une température voisine de 20°C. On obtient 0,430 g de dichlorhydrate de l'acide (3R,4R)-4-[3-(6-méthoxyquinolin-4-yl)propyl]-1-[3-(thién-2-yl)prop-2-ynyl] pipéridine-3-acétique, sous forme d'un solide blanc fondant en devenant pâteux et se décomposant au voisinage de 180°C.

Spectre de R.M.N. ¹H (400 MHz, (CD₃)₂SO d6, δ en ppm) : de 1,35 à 3,80 (mts : 16H) ; 4,00 (s : 3H) ; 4,38 et 4,43 (mfs : 2H en totalité) ; 7,16 (mt : 1H) ; 7,46 (d, J = 3,5 Hz : 1H) ; 7,53 (mt : 1H) ; 7,64 (mt : 2H) ; 7,74 (d, J = 5 Hz : 1H) ; 8,12 (d, J = 9,5 Hz : 1H) ; 8,86 (mf : 1H) ; 9,95 et 10,45 (2mfs étalés : 1H en totalité) ; de 12,20 à 12,55 (mf étalé : 1H).

### (3R,4R)-4-[3-(6-Méthoxyquinolin-4-yl)propyl]-1-[3-(thién-2-yl)prop-2-ynyl]pipéridine-3-acétate de méthyle

A une solution agitée de 0,44 g de (3R,4R)-4-[3-(6-méthoxyquinolin-4-yl)propyl]-1-(prop-2-ynyl)pipéridine-3-acétate de méthyle dans 15 cm³ d'acétonitrile anhydre, sous atmosphère inerte, on ajoute à une température voisine de 20°C 0,027 g de triphénylphosphine, 0,091 g de tétrakis(triphénylphosphine)palladium, puis 0,044 g d'iodure cuivreux. Après 15 minutes d'agitation de la solution obtenue on ajoute 0,2 cm³de 2-iodothiophène et 0,32 cm³ de triéthylamine. Après 12 heures d'agitation à une température voisine de 20°C, le mélange réactionnel est filtré sur célite, puis le gâteau est lavé par 5 fois 10 cm³ d'acétonitrile. Les extraits organiques réunis sont évaporés sous pression réduite (5 kPa) à une température voisine de 40°C. On obtient 1,1 g d'une huile que l'on purifie par chromatographie à pression atmosphérique sur une colonne de gel de silice (granulométrie 20-45µ ; diamètre 4 cm ; hauteur silice 28 cm) en éluant par l'acétate d'éthyle et en recueillant des fractions de 30 cm³. Les fractions 14 à 27 sont réunies puis évaporées sous pression réduite (5 kPa) à une température voisine de 40°C. On obtient 0,430 g de (3R,4R)-4-[3-(6-méthoxyquinolin-4-yl)propyl]-1-[3-(thién-2-yl)prop-2-ynyl]pipéridine-3-acétate de méthyle, sous forme d'une huile visqueuse de couleur jaune.

Spectre infra rouge (CH₂Cl₂) 2 936,2862 cm⁻¹ ν CH aliphatiques ;2806,2763 cm⁻¹ ν CH₂ N(CH₂)₃ ;1731 cm⁻¹ ν C=O ester ;1242,1227cm⁻¹ ν C-O éther ; 848 cm⁻¹ γCH quinoline

### (3R,4R)-4-[3-(6-Méthoxyquinolin-4-yl)propyl]-1-(prop-2-ynyl)pipéridine-3-acétate de méthyle

A une solution agitée de 0,8 g de (3R,4R)-4-[3-(6-méthoxyquinolin-4-yl) propyl]pipéridine-3-acétate de méthyle dans 15 cm³ de diméthylformamide anhydre, sous atmosphère inerte, on ajoute à une température voisine de 20°C 1,28 cm³ de triéthylamine, puis 0,26 cm³ de bromure de propargyle à 97 %. Le mélange est porté à une température voisine de 45°C pendant 6 heures, puis refroidi à environ 20°C. Après dilution par 150 cm³ d'eau le mélange est extrait par 5 fois 50 cm³ d'acétate d'éthyle. Les extraits réunis sont lavés par 3 fois 50 cm³ d'eau, séchés sur sulfate de magnésium, filtrés, concentrés sous pression réduite (5 kPa) à une température voisine de 40°C. On obtient 1 g d'un résidu huileux que l'on purifie par chromatographie sous pression atmosphérique, sur une colonne de gel de silice (granulométrie µ ; diamètre 3 cm ; hauteur silice 27 cm) en éluant par de l'acétate d'éthyle pur. On recueille des fractions de 50 cm³. Les fractions 7 à 10 sont réunies puis évaporées sous pression réduite (5 kPa) à une température voisine de 40°C. On obtient 0,45 g de (3R,4R)-4-[3-(6-méthoxyquinolin-4-yl)propyl]-1-(prop-2-ynyl)pipéridine-3-acétate de méthyle, sous forme d'une huile visqueuse incolore.

Spectre infra rouge (CH₂Cl₂) 3302 cm⁻¹ ν CH acétylénique ; 2936,2863 cm⁻¹ ν CH aliphatiques ; 2808,2764 cm⁻¹ ν CH₂ N(CH₂)₃;1731 cm⁻¹ ν C=O ester ;1242 ;1227 cm⁻¹ ν C-O éther ; 848 cm⁻¹ γCH quinoline

Le (3R,4R)-4-[3-(6-méthoxyquinolin-4-yl)propyl]pipéridine-3-acétate de méthyle a été préparé selon le mode opératoire décrit dans l'exemple 14.

### Exemple 16

### Acide (3R,4R)-4-[3-hydroxy-3-(6-méthoxyquinolin-4-yl)propyl]-1-[3-(thién-2-yl)prop-2-ynyl]pipéridine-3-carboxylique, diastéréoisomère A et acide (3R,4R)-4-[3-hydroxy-3-(6-méthoxyquinolin-4-yl)propyl]-1-[3-(thién-2-yl)prop-2-ynyl]pipéridine-3-carboxylique, diastéréoisomère B.

3 g d'acide (3R,4R)-4-[3-(R,S)-hydroxy-3-(6-méthoxyquinolin-4-yl)propyl]-1-[3-(thién-2-yl)prop-2-ynyl]pipéridine-3-carboxylique (obtenu comme décrit à l'exemple 9) dissous dans 100 cm³ d'un mélange de dichlorométhane-méthanol-acétonitrile (85/8/7 en volumes) sont chromatographiés sur une colonne de 35 cm de long et 8 cm de diamètre, conditionnée avec 1,200 kg de silice KROMASIL^{®} (granulométrie 10µ). L'élution est effectuée à l'aide du même mélange que ci-dessus. La détection est effectuée en ultra violet à 280 nm. Cette opération conduit à l'obtention des deux diastéréoisomères. Les fractions correspondant au premier sont concentrées à sec sous pression réduite (5 kPa) à une température voisine de 40°C. On obtient un résidu solide que l'on sèche sous pression réduite (23 Pa) à une température voisine de 20°C. On obtient 0,612 g d'acide (3R,4R)-4-[3-hydroxy-3-(6-méthoxyquinolin-4-yl)propyl]-1-[3-(thién-2-yl)prop-2-ynyl]pipéridine-3-carboxylique carboxylique, diastéréoisomère A. (α_{D}²⁰= -67,5° +/-1,3 dans le dichlorométhane à 0,5%), sous forme d'une meringue de couleur blanche. Les fractions correspondant au second diastéréoisomère sont traitées comme précédemment. On obtient 0,596 g d'acide (3R,4R)-4-[3-hydroxy-3-(6-méthoxyquinolin-4-yl)propyl]-1-[3-(thién-2-yl)prop-2-ynyl]pipéridine-3-carboxylique, diastéréoisomère B. (α_{D}²⁰=+106,0° +/-1,6 dans le dichlorométhane à 0,5 %), sous forme d'une meringue de couleur blanche.
diastéréoisomère A : Spectre de R.M.N. ¹H (300 MHz, (CD₃)₂SO d6, δ en ppm) : de 1,40 à 1,95 (mt : 7H) ; 2,40 (mt : 1H) ; de 2,50 à 2,60 (mt : 1H) ; de 2,60 à 280 (mt : 2H) ; 2,90 (mf: 1H) ; 3,63 (s : 2H) ; 3,94 (s : 3H) ; 5,24 (mt : 1H) ; 5,52 (mt : 1H) ; 7,08 (dd, J = 5 et 4 Hz : 1H) ; 7,31 (dd, J = 4 et 1 Hz : 1H) ; 7,35 (d, J = 3 Hz : 1H) ; 7,40 (dd, J = 9 et 3 Hz : 1H) ; 7,56 (d, J = 4,5 Hz : 1H) ; 7,59 (dd, J = 5 et 1 Hz : 1H) ; 7,95 (d, J = 9 Hz : 1H) ; 8,71 (d, J = 4,5 Hz : 1H).
diastéréoisomère B :Spectre de R.M.N.¹H (300 MHz, (CD₃)₂SO d6, δ en ppm) : de 1,20 à 1,95 (mt : 7H) ; 2,38 (mt : 1H) ; de 2,50 à 2,75 (mt : 3H) ; 2,71 (mf : 1H) ; 3,58 (s : 2H) ; 3,90 (s : 3H) ; 5,25 (mt : 1H) ; 5,52 (mt : 1H) ; 7,07 (dd, J = 5 et 3,5 Hz : 1H) ; 7,29 (d large, J = 3,5 Hz : 1H) ; de 7,30 à 7,45 (mt : 2H) ; 7,53 (d, J = 4,5 Hz : 1H) ; 7,58 (d large, J = 5 Hz : 1H) ; 7,94 (d, J = 10 Hz : 1H) ; 8,70 (d, J = 4,5 Hz : 1H).

### Exemple 17 :

### (3R,4R)-4-[3-Oxo-3-(6-méthoxyquinolin-4-yl)propyl]pipéridine-3-acétate de méthyle

Une solution de 10,8 g d'acide (3R,4R)-4-[3-oxo-3-(6-méthoxyquinolin-4-yl)propyl]-1-(tert-butyloxycarbonyl)pipéridine-3-acétique dans 460 cm³ de méthanol anhydre additionnés de 4,3 cm³ d'acide sulfurique concentré (d=1,83) est chauffée à une température voisine de 65 °C sous agitation pendant 2 heures. Après refroidissement aux environs de 20°C, le mélange réactionnel est évaporé sous pression réduite (5 kPa) à une température voisine de 40°C, puis le résidu obtenu est repris par 200 cm³ d'eau, rendu alcalin par addition d'hydrogénocarbonate de sodium jusqu'à l'obtention d'un pH voisin de 8-9. Le mélange est extrait par 4 fois 200 cm³ d'acétate d'éthyle. La phase aqueuse est alcalinisée jusqu'à un pH voisin de 11 par addition de la quantité nécessaire de carbonate de sodium. Le mélange est extrait par 2 fois 200 cm³ d'acétate d'éthyle. Les extraits organiques sont réunis, séchés sur sulfate de magnésium, filtrés, évaporés sous pression réduite (5 kPa) à une température voisine de 40°C. On obtient 6,84 g de (3R,4R)-4-[3-oxo-3-(6-méthoxyquinolin-4-yl) propyl]pipéridine-3-acétate de méthyle, sous forme d'une huile de couleur brune.

Spectre infra rouge (CCl₄) : 2935 ; 2812 ; 1738 ; 1692 ; 1620 ; 1504 ; 1242 ; 1032 et 851 cm⁻¹

### Acide (3R,4R)-4-[3-oxo-3-(6-méthoxyquinolin-4-yl)propyl]-1-(tert-butyloxy carbonyl)pipéridine-3-acétique

A une solution de 1,2 g de (3R,4R)-4-[3-oxo-3-(6-méthoxyquinolin-4-yl)propyl]-1-(tert-butyloxycarbonyl)pipéridine-3-acétaldéhyde dans 60 cm³ d'acétone, on ajoute en 1 heure environ, sous agitation et à une température voisine de 25°C, une solution de 0,85 g de permanganate de potassium dans 25 cm³ d'eau et 120 cm³ d'acétone. Le mélange est agité pendant 3 heures à cette même température, puis refroidi à environ 10°C. Une solution de 5 g de sulfite de sodium dans 200 cm³ d'eau est ajoutée au mélange réactionnel, puis le mélange obtenu est filtré sur célite. L'acétone du filtrat est évaporée sous pression réduite (5 kPa) à une température voisine de 40°C, puis le résidu d'évaporation est repris par 200 cm³ d'eau, lavé par 200 cm³ d'éther diéthylique. La phase aqueuse est décantée, acidifiée par de l'acide citrique à l'état solide à un pH voisin de 3-4, extraite par 200 cm³ d'éther diéthylique. La solution éthérée décantée est séchée sur sulfate de magnésium, filtrée, évaporée dans les conditions de ci-dessus. On obtient 0,74 g d'acide (3R,4R)-4-[3-oxo-3-(6-méthoxyquinolin-4-yl)propyl]-1-(*tert*-butyloxycarbonyl)pipéridine-3-acétique, sous forme d'un solide jaune.

Spectre infra rouge (KBr) : 2932 ; 2588 ; 1730 ; 1690 ; 1620 ; 1431 ; 1246 ; 1165 et 857 cm⁻¹

### (3R,4R)-4-[3-Oxo-3-(6-méthoxyquinolin-4-yl)propyl]-1-(tert-butyloxycarbonyl) pipéridine-3-acétaldéhyde

A une solution de 8,3 cm³ de chlorure d'oxalyle dans 65 cm³ de dichlorométhane refroidie à une température voisine de -60°C, on ajoute lentement, sous agitation et sous atmosphère inerte, un mélange de 13,7 g de diméthylsulfoxyde dans 65 cm³ de dichlorométhane. Après 15 minutes d'agitation du mélange, on ajoute lentement 10 g de (3R,4R)-3-(2-hydroxyéthyl)-4-[3-(R,S)-hydroxy-3-(6-méthoxyquinolin-4-yl) propyl]-1-(*tert-*butyloxycarbonyl)pipéridine solubilisés dans 65 cm³ de dichloro-méthane. Après 30 minutes d'agitation du mélange, on ajoute finalement, goutte à goutte, 61,7 cm³ de triéthylamine dissous dans 65 cm³ de dichlorométhane. Le mélange est encore agité pendant 3 heures aux environs de -60°C, puis versé sur 400 cm³ d'eau glacée. Après décantation du mélange, la phase organique est lavée par 400 cm³ d'une solution aqueuse d'acide citrique à 10 % (en masse), puis séchée sur sulfate de magnésium, filtrée, évaporée sous pression réduite (5 kPa) à une température voisine de 40°C. On obtient 9,95 g de (3R,4R)-4-[3-oxo-3-(6-méthoxy-quinolin-4-yl)propyl]-1-(*tert*-butyloxycarbonyl)pipéridine-3-acétaldéhyde, sous forme d'une huile de couleur brune.

Spectre infra rouge (CCl₄) : 2932 ; 2720 ; 1729 ; 1694 ; 1430 ; 1244 ; 1164 et 850 cm⁻¹

### (3R,4R)-3-(2-Hydroxyéthyl)-4-[3-(R,S)-hydroxy-3-(6-méthoxyquinolin-4-yl) propyl]-1-(tert-butyloxycarbonyl)pipéridine

A une solution de 52,6 g de (3R,4R)-4-[3-oxo-3-(6-méthoxyquinolin-4-yl)propyl]-1-(*tert*-butyloxycarbonyl)-3-vinylpipéridine dans 500 cm³ de toluène, on ajoute 33,4 cm³ de complexe de triéthylamine borane sous agitation, à une température voisine de 20°C, puis le mélange est chauffé pendant 18 heures à une température voisine de 110°C. Après avoir concentré le mélange réactionnel sous pression réduite (5 kPa) à une température voisine de 45°C, le résidu obtenu est repris par 500 cm³ de tétrahydrofuranne. La solution qui en résulte est additionnée en 20 minutes environ de 63 cm³ d'eau, puis on ajoute en 1 heure environ et par petites fractions, 47,5 g de perborate de sodium. Le mélange est agité pendant 4 heures à une température voisine de 20°C, puis on ajoute 300 cm³ d'une solution saturée de chlorure d'ammonium. La solution organique est décantée, séchée sur sulfate de magnésium, concentrée dans les mêmes conditions que ci-dessus. Le résidu obtenu est purifié par chromatographie sur une colonne de gel de silice (granulométrie 20-45µ ; diamètre 9 cm ; volume silice 2500 cm³) en éluant d'abord par un mélange de dichlorométhane-méthanol (97,5/2,5 en volumes) et en recueillant des fractions d' 1 litre. Les fractions 1 à 17 sont séparées, puis on élue par un mélange de dichlorométhane-méthanol (95/5 en volumes) en recueillant des fractions d'1 litre. Les fractions 30 à 35 sont réunies et l'on élue finalement par un mélange de dichlorométhane-méthanol (90/10 en volumes) en recueillant des fractions d'1 litre. Les fractions 36 à 41 sont réunies, tandis que l'ensemble des fractions 30 à 41 est évaporé sous pression réduite (5 kPa) à une température voisine de 40°C. On obtient 20 g de (3R,4R)-3-(2-hydroxyéthyl)-4-[3-(R,S)-hydroxy-3-(6-méthoxyquinolin-4-yl)propyl]-1-(*tert-*butyloxycarbonyl)pipéridine, sous forme d'une huile.

Spectre infra rouge (CH₂Cl₂) 3612 ;2480 ;2937 ;1680 ;1432 ;1243 ;1163 et 859 cm⁻¹

### (3R,4R)-4-[3-Oxo-3-(6-méthoxyquinolin-4-yl)propyl]-1-(tert-butyloxycarbonyl)-3-vinylpipéridine

A une solution agitée de 126 g de (3R,4R)-4-[3-oxo-3-(6-méthoxyquinolin-4-yl) propyl]-3-vinylpipéridine dans 1700 cm³ de dichlorométhane, on ajoute en 20 minutes environ, à une température voisine de 20°C, 162 cm³ de triéthylamine, puis en 2 heures 85 g de di-tertiobutyl dicarbonate solubilisés dans 300 cm³ de dichlorométhane. Le mélange est agité pendant 16 heures à une température voisine de 20°C, puis on y ajoute 400 cm³ d'eau. La phase organique est décantée, séchée sur sulfate de magnésium, filtrée, évaporée sous pression réduite (5 kPa) à une température voisine de 40°C. On obtient un résidu huileux que l'on reprend par 1000 cm³ d'acétate d'éthyle et que l'on lave par 2 fois 200 cm³ d'eau, 1 fois par 250 cm³ d'une solution aqueuse saturée d'acide citrique, 2 fois par 200 cm³ d'eau. La solution organique est séchée sur sulfate de magnésium, filtrée, concentrée dans les conditions de ci-dessus. On obtient 148 g de (3R,4R)-4-[3-oxo-3-(6-méthoxyquinolin-4-yl)propyl]-1-(*tert*-butyloxycarbonyl)-3-vinylpipéridine, sous forme d'une huile de couleur brune.

Spectre infra rouge (CH₂Cl₂): 2979 ;1683 ;1431 ;1246 ;1164 et 853 cm⁻¹

La (3R,4R)-4-[3-oxo-3-(6-méthoxyquinolin-4-yl)propyl]-3-vinylpipéridine peut être obtenue par application de la méthode décrite dans la demande de brevet FR 2 354 771.

### Exemple 18

### Acide (3R,4R)-4-[3-(R,S)-hydroxy-3-(6-méthoxyquinolin-4-yl)propyl]-1-[3-(3,5-difluorophényl)prop-2-ynyl]pipéridine-3-carboxylique

Un mélange de 0,89 g de (3R,4R)-4-[3-(R,S)-hydroxy-3-(6-méthoxyquinolin-4-yl) propyl]-1-[3-(3,5-difluorophényl)prop-2-ynyl]pipéridine-3-carboxylate de méthyle dans 9 cm³ de dioxanne additionnés de 1,41 cm³ de soude aqueuse 5N est agité pendant 18 heures à une température voisine de 60°C. Après refroidissement aux environs de 20°C, le mélange réactionnel est évaporé sous pression réduite (5 kPa) à une température voisine de 40°C. Le résidu obtenu est purifié par chromatographie, sous une pression d'azote de 50 kPa, sur une colonne de gel de silice (granulométrie 20-45µ ; diamètre 3,3 cm ; masse 56 g) en éluant d'abord par un mélange de dichlorométhane-méthanol (95/5 en volumes). On recueille d'abord une fraction de 100 cm³, puis des fractions de 16 cm³. Les fractions 1 à 36 sont séparées. On élue ensuite par un mélange de dichlorométhane-méthanol (75/25 en volumes). On effectue une première fraction de 200 cm³, une seconde de 150 cm³, puis une troisième de 100 cm³. Ces deux dernières sont réunies, concentrées comme ci-dessus. Le résidu obtenu est repris dans le dichlorométhane, filtré. Le filtrat est évaporé comme précédemment, puis le nouveau résidu obtenu est trituré dans 25 cm³ d'un mélange d'éther diisopropylique-pentane (50/50 en volumes). Le produit qui cristallise est filtré, lavé par 2 fois 10 cm³ du même mélange, puis 3 fois 10 cm³ de pentane. On obtient 0,53 g d'acide (3R,4R)-4-[3-(R,S)-hydroxy-3-(6-méthoxyquinolin-4-yl)propyl]-1-[3-(3,5-difluorophényl)prop-2-ynyl]pipéridine-3-carboxylique, sous forme d'un solide de couleur crème fondant à 106°C.

Spectre de R.M.N. ¹H (300 MHz, (CD₃)₂SO d6, δ en ppm). On observe un mélange de deux diastéréoisomères :
de 1,40 à 1,95 (mt : 7H) ; de 2,30 à 3,00 (mt : 5H) ; 3,57 et 3,59 (2s : 2H en totalité) ; 3,90 et 3,93 (2s : 3H en totalité) ; 5,25 (mt : 1H) ; 5,55 (mf : 1H) ; 7,22 (mt : 2H) ; de 7,25 à 7,45 (mt : 3H) ; 7,56 (mt : 1H) ; 7,94 (d, J = 9 Hz : 1H) ; 8,70 (d, J = 4,5 Hz : 1H) ; de 12,10 à 12,80 (mf étalé : 1H).

### (3R,4R)-4-[3-(R,S)-Hydroxy-3-(6-méthoxyquinolin-4-yl)propyl]-1-[3-(3,5-difluorophényl)prop-2-ynyl]pipéridine-3-carboxylate de méthyle

A un mélange de 0,95 g de (3R,4R)-4-[3-(R,S)-hydroxy-3-(6-méthoxyquinolin-4-yl)propyl]-1-(prop-2-ynyl)pipéridine-3-carboxylate de méthyle dans 9,5 cm³ de triéthylamine, agité à une température voisine de 20°C sous atmosphère inerte, on ajoute 0,138 g de tétrakis triphénylphosphine palladium, 0,046 g d'iodure cuivreux et 0,42 cm³ de 1-bromo-3,5-difluorobenzène. Le mélange est chauffé à une température voisine de 80°C pendant 3 heures. Après refroidissement à environ 20°C, le mélange réactionnel est repris par 30 cm³ d'acétate d'éthyle et 30 cm³ d'eau, agité pendant 5 minutes. La phase organique est décantée tandis que la couche aqueuse est extraite par 2 fois 30 cm³ d'acétate d'éthyle. Les extraits organiques sont réunis, lavés par 3 fois 30 cm³ d'eau, séchés sur sulfate de sodium, filtrés, concentrés sous pression réduite (5 kPa) à une température voisine de 40°C. Le résidu obtenu est purifié par chromatographie sous une pression d'azote de 50 kPa, sur une colonne de gel de silice (granulométrie 20-45µ; diamètre2,3 cm ; 40 g) en éluant par de l'acétate d'éthyle pur. On recueille d'abord 2 fractions de 100 cm³, puis on effectue des fractions de 20 cm³. Les fractions 8 à 40 sont réunies, évaporées dans les conditions de ci-dessus. On obtient 0,967 g de (3R,4R)-4-[3-(R,S)-hydroxy-3-(6-méthoxyquinolin-4-yl)propyl]-1-[3-(3,5-difluorophényl)prop-2-ynyl]pipéridine-3-carboxylate de méthyle, sous forme d'une meringue de couleur orangée.

Spectre infra-rouge (KBr) : 3424 ; 2948 ; 1734 ; 1618 ; 1586 ; 1432 ; 1242 ; 1122 ; 1028 ; 990 ; 856 et 373 cm⁻¹

Le (3R,4R)-4-[3-(R,S)-hydroxy-3-(6-méthoxyquinolin-4-yl)propyl]-1-(prop-2-ynyl) pipéridine-3-carboxylate de méthyle peut être préparé comme décrit dans l'exemple 13.

### Exemple 19

### Acide (3R,4R)-4-[3-(R,S)-hydroxy-3-(6-méthoxyquinolin-4-yl)propyl]-1-[3-(2,5-difluorophényl)prop-2-ynyl]pipéridine-3-carboxylique

Un mélange de 0,874 g de (3R,4R)-4-[3-(R,S)-hydroxy-3-(6-méthoxyquinolin-4-yl) propyl]-1-[3-(2,5-difluorophényl)prop-2-ynyl]pipéridine-3-carboxylate de méthyle dans 8,8 cm³ de dioxanne additionnés de 1,37 cm³ de soude aqueuse 5 N est agité pendant 17 heures à une température voisine de 60°C. Après refroidissement à environ 20°C, le mélange réactionnel est évaporé sous pression réduite (5 kPa) à une température voisine de 40°C. On obtient une huile que l'on purifie par chromatographie sous une pression de 50 kPa d'azote, sur une colonne de gel de silice (granulométrie 20-45µ ; diamètre 3 cm ; 25 g), en éluant par un mélange de dichlorométhane-méthanol (85/15 en volumes), en recueillant d'abord une fraction de 100 cm³, puis des fractions de 50 cm³. Les fractions 3 et 4 sont réunies, concentrées comme ci-dessus. Le résidu d'évaporation est repris par du dichlorométhane, filtré. Le filtrat est concentré comme dans les conditions précédentes, puis le produit obtenu est repris sous agitation par 40 cm³ d'un mélange de pentane-éther diisoprppylique (50/50 en volumes) pendant 16 heures à une température voisine de 20°C. Les cristaux obtenus sont essorés, lavés par 2 fois 10 cm³ du même mélange que ci-dessus, puis 3 fois 20 cm³ de pentane. On obtient 0,392 g d'acide (3R,4R)-4-[3-(R,S)-hydroxy-3-(6-méthoxyquinolin-4-yl)propyl]-1-[3-(2,5-difluorophényl)prop-2-ynyl] pipéridine-3-carboxylique, sous forme d'un solide jaune pâle fondant à 109°C, mélange de deux diastéréoisomères.

Spectre de R.M.N. ¹H (300 MHz, (CD₃)₂SO d6, δ en ppm). On observe un mélange de deux diastéréoisomères :
de 1,40 à 2,00 (mts : 7H) ; de 2,30 à 3,00 (mt : 5H) ; 3,64 et 3,65 (2s : 2H en totalité) ; 3,90 et 3,92 (2s : 3H en totalité) ; 5,26 (mf: 1H) ; 5,56 (mf: 1H) ; de 7,25 à 7,50 (mt : 5H) ; 7,56 (mt : 1H) ; 7 ,94 (d, J = 9 Hz : 1H) ; 8,71 (d, J = 4,5 Hz : 1H) ; de 12,40 à 12,70 (mf : 1H).

### (3R,4R)-4-[3-(R,S)-Hydroxy-3-(6-méthoxyquinolin-4-yl)propyl]-1-[3-(2,5-difluorophényl)prop-2-ynyl]pipéridine-3-carboxylate de méthyle

Un mélange de 1,1 g de (3R,4R)-4-[3-(R,S)-hydroxy-3-(6-méthoxyquinolin-4-yl) propyl]-1-(prop-2-ynyl)pipéridine-3-carboxylate de méthyle, 11 cm³ de triéthylamine, 0,16 cm³ de tétrakis triphénylphosphine palladium, 0,053 g d'iodure cuivreux et de 0,47 cm³ de 1-bromo-2,5-difluorobenzène est agité pendant 3 heures 15 minutes sous atmosphère inerte à une température voisine de 80°C. Après refroidissement à environ 20°C, le mélange réactionnel est additionné de 30 cm³ d'acétate d'éthyle et 30 cm³ d'eau, sous agitation. La phase organique est séparée tandis que la phase aqueuse est extraite par 2 fois 30 cm³ d'acétate d'éthyle. Les extraits organiques sont réunis, lavés par 3 fois 25 cm³ d'eau, séchés sur sulfate de sodium, concentrés sous pression réduite (5 kPa) à une température voisine de 40°C. On obtient un produit que l'on purifie par chromatographie sous une pression de 50 kPa d'azote, sur une colonne de gel de silice (granulométrie 20-45µ ; diamètre 3,3 cm ; 50 g), en éluant par de l'acétate d'éthyle et en recueillant d'abord une fraction de 300 cm³, puis des fractions de 32 cm³. Les fractions 7 à 22 sont réunies, puis concentrées dans les conditions de ci-dessus. On obtient 0,91 g de (3R,4R)-4-[3-(R,S)-hydroxy-3-(6-méthoxyquinolin-4-yl)propyl]-1-[3-(2,5-difluorophényl)prop-2-ynyl]pipéridine-3-carboxylate de méthyle, sous forme d'une meringue de couleur blanc cassé.

Spectre infra rouge (CCl₄) : 3614 ; 2950 ; 1738 ; 1622 ; 1497 ; 1249 ; 1163 ; 1033 et 873 cm⁻¹

Le (3R,4R)-4-[3-(R,S)-hydroxy-3-(6-méthoxyquinolin-4-yl)propyl]-1-(prop-2-ynyl) pipéridine-3-carboxylate de méthyle a été préparé dans l'exemple 13.

### Exemple 20

### Acide (3R,4R)-4-[3-hydroxy-3-(6-méthoxyquinolin-4-yl)propyl]-1-[3-(thién-2-yl)prop-2-ynyl]pipéridine-3-acétique, diastéréoisomère A et acide (3R,4R)-4-[3-hydroxy-3-(6-méthoxyquinolin-4-yl)propyl]-1-[3-(thién-2-yl)prop-2-ynyl] pipéridine-3-acétique, diastéréoisomère B.

1,2 g d'acide (3R,4R)-4-[3-(R,S)-hydroxy-3-(6-méthoxyquinolin-4-yl)propyl]-1-[3-(thién-2-yl)prop-2-ynyl]pipéridine-3-acétique est chromatographié sur une colonne de 35 cm de long et 6 cm de diamètre, conditionnée avec 700 g de silice KROMASIL^{®} (granulométrie 10µ). L'élution est effectuée à l'aide d'un mélange de dichlorométhane-acétonitrile-méthanol-triéthylamine (70/15/15/0,05 en volumes). Le débit est de 90 cm³/mn. La détection est effectuée en ultraviolet à 265 nm. Deux injections préparatives ont conduit à la séparation des 2 diastéréoisomères. Les fractions correspondant au premier, le diastéréoisomère A, sont concentrées sous pression réduite (5 kPa) à une température voisine de 40°C. La masse cristalline obtenue est séchée à l'étuve sous pression réduite (10 Pa) à une température voisine de 20°C. On obtient 0,408 g d'acide (3R,4R)-4-[3-hydroxy-3-(6-méthoxyquinolin-4-yl)propyl]-1-[3-(thién-2-yl)prop-2-ynyl]pipéridine-3-acétique, diastéréoisomère A, sous forme d'un solide de couleur jaune. (α_{D}²⁰ = -62,9+/-1,3 dans le méthanol à 0,5 %). Les fractions correspondant au second, le diastéréoisomère B, sont concentrées sous pression réduite (5 kPa) à une température voisine de 40°C. La masse cristalline obtenue est séchée à l'étuve sous pression réduite (10 Pa) à une température voisine de 20°C. On obtient 0376 g d'acide (3R,4R)-4-[3-hydroxy-3-(6-méthoxyquinolin-4-yl)propyl]-1-[3-(thién-2-yl)prop-2-ynyl]pipéridine-3-acétique, diastéréoisomère B, sous forme d'un solide de couleur jaune. (α_{D}²⁰ = +46,9 +/-1,1 dans le méthanol à 0,5%)
diastéréoisomère A: Spectre d e R.M.N.¹H (300 M Hz, (CD₃)₂SO d 6 avec ajout de quelques gouttes de CD3COOD d4, δ en ppm) : de 1,20 à 2,00 et de 2,05 à 2,50 (2 séries de mts : 12H en totalité) ; de 2,60 à 2,85 (mt : 2H) ; 3,55 (AB limite, J = 17 Hz : 2H) ; 3,90 (s : 3H) ; 5,27 (dd, J = 8 et 3 Hz : 1H) ; 7,04 (mt : 1H) ; 7,26 (d large, J = 4 Hz : 1H) ; de 7,30 à 7,45 (mt : 2H) ; 7,52 (d, J = 5,5 Hz : 1H) ; 7,56 (d, J = 4,5 Hz : 1H) ; 7,93 (d, J = 10 Hz : 1H) ; 8,70 (d, J = 4,5 Hz : 1H).
diastéréoisomère B : Spectre de R.M.N.¹H (300 MHz, (CD₃)₂SO d6 avec ajout de quelques gouttes de CD3COOD d4, δ en ppm) : de 1,20 à 1,90 et de 2,00 à 2,45 (2 séries de mts : 12H en totalité) ; de 2,60 à 2,85 (mt : 2H) ; 3,50 (AB, J = 17 Hz : 2H) ; 3,91 (s : 3H) ; 5,27 (dd, J = 8 et 3,5 Hz : 1H) ; 7,05 (dd, J = 5,5 et 4 Hz : 1H) ; 7,26 (d large, J = 4 Hz : 1H) ; de 7,35 à 7,45 (mt : 2H) ; de 7,50 à 7,60 (mt : 2H) ; 7,95 (d, J = 10 Hz : 1H) ; 8,71 (d, J = 4,5 Hz : 1H).

### Acide (3R,4R)-4-[3-(R,S)-hydroxy-3-(6-méthoxyquinolin-4-yl)propyl]-1-[3-(thién-2-yl)prop-2-ynyl]pipéridine-3-acétique

Une solution de 3 g de (3R,4R)-4-[3-(R,S)-hydroxy-3-(6-méthoxyquinolin-4-yl) propyl]-1-[3-(thién-2-yl)prop-2-ynyl]pipéridine-3-acétate de méthyle dans 50 cm³ de dioxanne, additionnée de 5 cm³ de soude aqueuse 5N, est chauffée pendant 17 heures à une température voisine de 60°C, sous agitation et sous atmosphère inerte. Après refroidissement à environ 20°C, le mélange est concentré sous pression réduite (5 kPa) à une température voisine de 40°C, puis le résidu est repris par 100 cm³ de dichlorométhane et 5 cm³ d'eau. Le mélange est acidifié par la quantité suffisante d'acide citrique pour obtenir un pH voisin de 4-5, puis séché sur sulfate de magnésium, filtré, concentré comme ci-dessus. L'eau résiduelle est entraînée par azéotropie dans le chloroforme. Après concentration comme précédemment, le résidu obtenu est purifié par chromatographie à pression atmosphérique sur une colonne de gel de silice (granulométrie 20-45µ ; diamètre 3,5 cm ; volume de silice 200 cm³), en éluant par un mélange de chloroforme-méthanol-ammoniaque à 28% (12/3/0,5 en volumes) et en recueillant des fractions d'environ 60 cm³. Les fractions contenant le produit attendu sont réunies, puis concentrées sous pression réduite (5 kPa) à une température voisine de 40°C. Le résidu obtenu est repris par 40 cm³ d'éther diéthylique, essoré, lavé par 2 fois 5 cm³ d'éther, séché sous pression réduite (5 kPa) à une température voisine de 20°C, puis à l'étuve sous pression réduite (10 Pa) à une température voisine de 40°C. On obtient 1,9 g d'acide (3R,4R)-4-[3-(R,S)-hydroxy-3-(6-méthoxyquinolin-4-yl)propyl]-1-[3-(thién-2-yl)prop-2-ynyl]pipéridine-3-acétique, sous forme d'un solide blanc.

Spectre de R.M.N. ¹H (300 MHz, (CD₃)₂SO d6, δ en ppm). On observe un mélange de deux diastéréoisomères :
de 1,20 à 2,45 (mts : 12H) ; de 2,60 à 2,85 (mt : 2H) ; 3,48 (mt : 2H) ; 3,92 (s : 3H) ; 5,28 (mt : 1H) ; 7,06 (dd, J = 5,5 et 3,5 Hz : 1H) ; 7,27 (dd, J = 3,5 et 1 Hz : 1H) ; de 7,30 à 7,45 (mt : 2H) ; de 7,50 à 7,60 (mt : 2H) ; 7,94 (d, J = 9 Hz : 1H) ; 8,70 (d, J = 4,5 Hz : 1H).

### Exemple 21

### (3R,4R)-4-[3-(R,S)-Hydroxy-3-(6-méthoxyquinolin-4-yl)propyl]-1-[3-(thién-2-yl)prop-2-ynyl]pipéridine-3-acétate de méthyle

A une solution de 4,0 g de (3R,4R)-4-[3-oxo-3-(6-méthoxyquinolin-4-yl)propyl]-1-[3-(thién-2-yl)prop-2-ynyl]pipéridine-3-acétate de méthyle dans 65 cm³ de méthanol, additionnée d'une goutte de soude aqueuse 5N, on ajoute en 40 minutes environ, à une température inférieure à 30°C, sous agitation et sous atmosphère inerte, 0,317 g de borohydrure de sodium. Après 3 heures d'agitation à une température voisine de 20°C, le mélange est additionné de 100 cm³ d'eau, puis extrait par 4 fois 50 cm³ de dichlorométhane. Les extraits sont lavés par 3 fois 50 cm³ d'eau, séchés sur sulfate de magnésium, filtrés, concentrés sous pression réduite (5 kPa) à une température voisine de 40°C. On obtient une huile que l'on purifie par chromatographie à pression atmosphérique, sur une colonne de gel de silice (granulométrie 20-45µ ; diamètre 3,2 cm ; volume de silice 300 cm³), en éluant par l'acétate d'éthyle et en recueillant des fractions d'environ 100 cm³. On réunit les fractions correspondant au produit attendu. Celles-ci sont concentrées comme ci-dessus. On obtient 3,5 g de (3R,4R)-4-[3-(R,S)-hydroxy-3-(6-méthoxyquinolin-4-yl)propyl]-1-[3-(thién-2-yl)prop-2-ynyl] pipéridine-3-acétate de méthyle, sous forme d'une huile de couleur jaune.

Spectre infra rouge (CCl₄) : 2939;1739;1622;1509;1434;1241;850 et 696 cm⁻¹

On a préparé le chlorhydrate de la façon suivante : Une solution de 0,5 g de (3R,4R)-4-[3-(R,S)-hydroxy-3-(6-méthoxyquinolin-4-yl)propyl]-1-[3-(thién-2-yl)prop-2-ynyl] pipéridine-3-acétate de méthyle dans 20 cm³ de dichlorométhane est ajoutée sous agitation à 30 cm³ d'éther diéthylique additionnés de 4 cm³ d'éther chlorhydrique 1N. Au bout de 2 heures, le solide blanc formé est filtré, lavé par 2 fois 5 cm³ d'éther diéthylique, séché sous pression partielle (5 kPa), puis à poids constant, à l'étuve, sous pression réduite (10 Pa) à une température voisine de 40°C. On obtient 0,35 g de dichlorhydrate de (3R,4R)-4-[3-(R,S)-hydroxy-3-(6-méthoxyquinolin-4-yl)propyl]-1-[3-(thién-2-yl)prop-2-ynyl]pipéridine-3-acétate de méthyle, sous forme d'un solide blanc.

Spectre infra rouge (KBr) : 3278 ; 2932 ; 2524 ; 1730 ; 1619 ; 1601 ; 1427 ; 1248 ; 1021 ; 849 et 714 cm⁻¹

### (3R,4R)-4-[3-Oxo-3-(6-méthoxyquinolin-4-yl)propyl]-1-[3-(thién-2-yl)prop-2-ynyl]pipéridine-3-acétate de méthyle

A une solution de 4,3 g de (3R,4R)-4-[3-oxo-3-(6-méthoxyquinolin-4-yl)propyl]-1-(prop-2-ynyl]pipéridine-3-acétate de méthyle dans 100 cm³ d'acétonitrile , on ajoute sous agitation à une température voisine de 20°C et sous atmosphère inerte, 0,263 g de triphénylphosphine, puis 0,85 g de tétrakis triphénylphosphine palladium, 0,4 g d'iodure cuivreux, 1,75 cm³ de 2-iodothiophène. On agite pendant 10 minutes le mélange, puis on ajoute 2,95 cm³ de triéthylamine. Après 48 heures d'agitation à une température voisine de 20°C, le mélange est filtré sur célite, l'insoluble lavé par 2 fois 50 cm³ d'acétonitrile. Le filtrat est concentré sous pression réduite (5 kPa) à une température voisine de 40°C. Le résidu d'évaporation est purifié par chromatographie à pression atmosphérique sur une colonne de gel de silice (granulométrie 20-45µ ; diamètre 4,5 cm ; volume de silice 500 cm³), en éluant par de l'acétate d'éthyle pur et en recueillant des fractions d'environ 60 cm³. On réunit les fractions correspondant au produit attendu. Celles-ci sont concentrées dans les conditions de ci-dessus. On obtient 4 g de (3R,4R)-4-[3-oxo-3-(6-méthoxyquinolin-4-yl)propyl]-1-[3-(thién-2-yl)prop-2-ynyl]pipéridine-3-acétate de méthyle, sous forme d'une huile de couleur jaune.

Spectre infra rouge (CCl₄) : 2935 ; 1740 ; 1692 ; 1620 ; 1431 ; 1242 ; 1032 ; 849 et 698 cm⁻¹

### (3R,4R)-4-[3-Oxo-3-(6-méthoxyquinolin-4-yl)propyl]-1-(prop-2-ynyl]pipéridine-3-acétate de méthyle

A une solution de 5 g de (3R,4R)-4-[3-oxo-3-(6-méthoxyquinolin-4-yl)propyl] pipéridine-3-acétate de méthyle dans 60 cm³ de diméthylformamide, on ajoute sous agitation à une température voisine de 20°C et sous atmosphère inerte, 4,8 cm³ de triéthylamine. Après 15 minutes d'agitation à cette température, on ajoute en 15 minutes environ 1,5 cm³ de bromure de propargyle, puis au bout de 15 minutes le mélange est chauffé à une température voisine de 45°C pendant 4 heures. Après refroidissement à environ 20°C, le mélange réactionnel est versé sur environ 700 cm³ d'eau, puis le mélange est extrait par 4 fois 80 cm³ d'éther diéthylique. Les extraits réunis sont lavés par 3 fois 80 cm³ d'eau, séchés, filtrés, concentrés sous pression réduite (5 kPa) à une température voisine de 40°C. Le résidu d'évaporation est purifié par chromatographie à pression atmosphérique sur une colonne de gel de silice (granulométrie 20-45µ ; diamètre 4 cm ; volume de silice 500 cm³), en éluant par de l'acétate d'éthyle pur et en recueillant des fractions de 60 cm³. Les fractions 9 à 20 sont réunies puis concentrées comme dans les conditions précédentes. On obtient 4,3 g de (3R,4R)-4-[3-oxo-3-(6-méthoxyquinolin-4-yl)propyl]-1-(prop-2-ynyl] pipéridine-3-acétate de méthyle, sous forme d'une huile de couleur jaune.

Spectre infra rouge (CCl₄): 3311 ; 2936 ; 1739 ; 1692 ; 1620 ; 1431 ; 1242 ; 1032 ; 849 ; 654 et 627 cm⁻¹

Le (3R,4R)-4-[3-oxo-3-(6-méthoxyquinolin-4-yl)propyl]pipéridine-3-acétate de méthyle a été préparé dans l'exemple 17.

### Exemple 22

### Acide (3R,4R)-4-[3-(R,S)-hydroxy-3-(6-méthoxyquinolin-4-yl)propyl]-1-[3-(2,6-difluorophényl)prop-2-ynyl]pipéridine-3-carboxylique

Une solution de 0,531 g de (3R,4R)-4-[3-(R,S)-hydroxy-3-(6-méthoxyquinolin-4-yl) propyl]-1-[3-(2,6-difluorophényl)prop-2-ynyl]pipéridine-3-carboxylate de méthyle dans 5,3 cm³ de dioxanne additionnés de 0,84 cm³ de soude aqueuse 5N est agitée pendant 15 heures à une température voisine de 60°C. Après refroidissement à une température voisine de 20°C, le mélange réactionnel est concentré sous pression réduite (5 kPa) à une température voisine de 40°C. Le résidu obtenu est purifié par chromatographie sous une pression de 50 kPa d'azote, sur une colonne de gel de silice (granulométrie 20-45µ ; diamètre 3 cm ; 20 g), en éluant par un mélange de dichlorométhane-méthanol (90/10 en volumes) et en recueillant d'abord une fraction de 100 cm³, puis des fractions de 25 cm³. Les fractions 1 à 12 sont réunies, puis concentrées comme précédemment. On obtient une meringue que l'on reprend sous agitation dans 15 cm³ d'éther diisopropylique pendant 15 minutes. Après addition de 15 cm³ de pentane et une agitation supplémentaire de 10 minutes, le produit cristallisé formé est filtré, lavé par 2 fois 10 cm³ d'un mélange éther diisopropylique-pentane (50/50 en volumes), puis 3 fois 20 cm³ de pentane, séché à l'air. On obtient 0,293 g d'acide (3R,4R)-4-[3-(R,S)-hydroxy-3-(6-méthoxyquinolin-4-yl)propyl]-1-[3-(2,6-difluorophényl)prop-2-ynyl]pipéridine-3-carboxylique, sous forme d'un solide de couleur crème fondant à 107°C.

Spectre de R.M.N.¹H (300 MHz, (CD₃)₂SO d6, δ en ppm). On observe un mélange de deux diastéréoisomères :
de 1,40 à 1,95 et de 2,30 à 3,00 (mts : 12H e n totalité); 3,67 et 3 ,68 (2s : 2 H en totalité) ; 3,88 et 3,92 (2s : 3H en totalité) ; 5,24 (mt : 1H) ; 5,55 (mf : 1H) ; 7,22 (mt : 2H) ; de 7,30 à 7,60 (mt : 4H) ; 7,93 (d, J = 9 Hz : 1H) ; 8,70 (mt : 1H) ; de 12,20 à 12,80 (mf étalé: 1H).

### (3R,4R)-4-[3-(R,S)-Hydroxy-3-(6-méthoxyquinolin-4-yl)propyl]-1-[3-(2,6-difluorophényl)prop-2-ynyl]pipéridine-3-carboxylate de méthyle

Un mélange de 1,07 g de (3R,4R)-4-[3-(R,S)-hydroxy-3-(6-méthoxyquinolin-4-yl)propyl]-1-(prop-2-ynyl)pipéridine-3-carboxylate de méthyle dans 10 cm³ de triéthylamine est agité pendant 5 minutes sous atmosphère inerte à une température voisine de 20°C. On ajoute 0,156 g de tétrakis triphénylphosphine palladium, 0,051 g d'iodure cuivreux et 0,78 g de 1-bromo-2,6-difluorobenzène. Le mélange est agité pendant 3 heures 30 minutes à une température voisine de 80°C. Après refroidissement à environ 20°C, le mélange réactionnel est additionné de 30 cm³ d'acétate d'éthyle et 30 cm³ d'eau. Après 10 minutes d'agitation, le mélange est décanté. Après séparation de la phase organique, la couche aqueuse est extraite par 2 fois 30 cm³ d'acétate d'éthyle. Les extraits organiques sont réunis, lavés par 3 fois 30 cm³ d'eau, séchés sur sulfate de sodium, filtrés, concentrés sous pression réduite (5 kPa) à une température voisine de 40°C. Le résidu obtenu est purifié par chromatographie sous une pression d'azote de 50 kPa, sur une colonne de gel de silice (granulométrie 20-45µ ; diamètre 3 cm ; 50 g), en éluant par de l'acétate d'éthyle pur et en recueillant d'abord une fraction de 300 cm³, puis des fractions de 38 cm³. Les fractions 6 à 16 sont réunies, concentrées comme ci-dessus. On obtient 0,55 g de (3R,4R)-4-[3-(R,S)-hydroxy-3-(6-méthoxyquinolin-4-yl)propyl]-1-[3-(2,6-difluorophényl)prop-2-ynyl]pipéridine-3-carboxylate de méthyle, sous forme d'une meringue de couleur jaune pâle.

Spectre infra rouge (CCl₄) :3615 ; 2950 ; 1738 ; 1622 ; 1470 ; 1241 ; 1007 ; 854 et 719 cm⁻¹

Le (3R,4R)-4-[3-(R,S)-hydroxy-3-(6-méthoxyquinolin-4-yl)propyl]-1-(prop-2-ynyl) pipéridine-3-carboxylate de méthyle peut être préparé comme décrit dans l'exemple 13.

### Exemple 23

### Acide (3R,4R)-4-[3-(6-méthlxyquinolin-4-yl)propyl]-1-[3-(3,5-difluorophényl) prop-2-ynyl]pipéridine-3-acétique

A une solution de 0,525 g de (3R,4R)-4-[3-(6-méthoxyquinolin-4-yl)propyl]-1-[3-(3,5-difluorophényl)prop-2-ynyl]pipéridine-3-acétate de méthyle dans 5 cm³ de dioxanne, on introduit sous agitation, à une température voisine de 20°C, 0,83 cm³ de soude aqueuse 5N, puis le mélange est chauffé pendant 3 heures à une température voisine de 60°C. Après refroidissement à environ 20°C, le mélange est agité pendant 3 jours, puis concentré sous pression réduite (5 kPa) à une température voisine de 40°C. Le résidu obtenu est repris par 15 cm³ d'eau, puis la solution est extraite par 5 cm³ d'acétate d'éthyle. La phase aqueuse est amenée à pH voisin de 5-6 par addition de 4,1 cm³ d'acide chlorhydrique aqueux 1N, puis extraite d'abord par 30 cm³ de dichlorométhane, puis 10 cm³ du même solvant. Les extraits organiques sont réunis, lavés par 2 fois 5 cm³ d'eau, séchés sur sulfate de magnésium, filtrés, concentrés sous pression réduite (5 kPa) à une température voisine de 40°C. Le résidu obtenu est repris dans de l'éther diéthylique, puis concentré dans les mêmes conditions que précédemment jusqu'à obtention d'un poids constant. On obtient 0,45 g d'acide (3R,4R)-4-[3-(6-méthoxyquinolin-4-yl)propyl]-1-[3-(3,5-difluoro-phényl)prop-2-ynyl]pipéridine-3-acétique, sous forme d'un solide de couleur beige clair.

Spectre de R.M.N.¹H (300 MHz, (CD₃)₂SO d6, δ en ppm) : de 1,20 à 1,80 (mt : 7H) ; de 2,10 à 2,35 (mt : 4H) ; 2,43 (dd, J = 16,5 et 10,5 Hz : 1H) ; 2,75 (mt : 2H) ; 3,05 (mt : 2H) ; 3,47 (s : 2H) ; 3,93 (s : 3H) ; 7,18 (mt : 2H) ; de 7,25 à 7,35 (mt : 1H) ; 7,33 (d, J = 4 Hz : 1H) ; de 7,35 à 7,45 (mt : 2H) ; 7,92 (d, J = 9 Hz : 1H) ; 8,62 (d, J = 4 Hz : 1H).

### (3R,4R)-4-[3-(6-Méthoxyquinolin-4-yl)propyl]-1-[3-(3,5-difluorophényl)prop-2-ynyl]pipéridine-3-acétate de méthyle

A un mélange agité de 0,77 g de (3R,4R)-4-[3-(6-méthoxyquinolin-4-yl)propyl]-1-(prop-2-ynyl)pipéridine-3-acétate de méthyle dans 7,7 cm³ de triéthylamine, on ajoute à une température voisine de 20°C, sous atmosphère inerte, 0,35 cm³ de 1-bromo-3,5-difluorobenzène, puis 0,112 g de tétrakis triphénylphosphine palladium et 0,037 g d'iodure cuivreux. La suspension obtenue est chauffée à une température voisine de 80°C pendant 3 heures, puis concentrée sous pression réduite (5 kPa) à une température voisine de 40°C. Le résidu obtenu est repris par 50 cm³ d'acétate d'éthyle et 20 cm³ d'eau. Après agitation du mélange pendant 15 minutes, l'insoluble persistant est filtré sur clarcel, puis le filtrat est décanté : la phase organique est séparée, puis lavée par 3 fois 3 cm³ d'eau, une solution aqueuse saturée de chlorure de sodium, séchée sur sulfate de magnésium, filtrée, concentrée sous pression réduite (5 kPa) à une température voisine de 40°C. Le résidu huileux obtenu est purifié par chromatographie sous une pression d'azote de 50 kPa, sur une colonne de gel de silice (granulométrie 40-63µ ; diamètre 3 cm ; 45 g) en éluant par un mélange de dichlorométhane-méthanol (97/3 en volumes). On recueille les fractions contenant le produit attendu. Celles-ci sont concentrées sous pression réduite (5 kPa) à une température voisine de 40°C. On obtient 0,345 g de (3R,4R)-4-[3-(6-méthoxyquinolin-4-yl)propyl]-1-[3-(3,5-difluorophényl)prop-2-ynyl]pipéridine-3-acétate de méthyle, sous forme d'une huile de couleur jaune pâle.

Spectre de masse (EI) m/z=506 M⁺

Le (3R,4R)-4-[3-(6-méthoxyquinolin-4-yl)propyl]-1-(prop-2-ynyl)pipéridine-3-acétate de méthyle a été préparé selon le mode opératoire décrit dans l'exemple 15.

### Exemple 24

### Acide (3R,4R)-4-[3-(R,S)-hydroxy-3-(6-méthoxyquinolin-4-yl)propyl]-1-[3-(2,3-difluorophényl)prop-2-ynyl]pipéridine-3-carboxylique

Un mélange de 0,810 g de (3R,4R)-4-[3-(R,S)-hydroxy-3-(6-méthoxyquinolin-4-yl) propyl]-1-[3-(2,3-difluorophényl)prop-2-ynyl]pipéridine-3-carboxylate de méthyle dans 8 cm³ de dioxanne additionnés de 1,3 cm³ de soude aqueuse 5N est agité pendant 17 heures à une température voisine de 60°C. Après refroidissement aux environs de 20°C, le mélange réactionnel est évaporé sous pression réduite (5 kPa), à une température voisine de 50°C. Le résidu obtenu est purifié par chromatographie, sous une pression de 50 kPa d'azote, sur une colonne de gel de silice (granulométrie 20-45µ ; diamètre 3 cm ; masse 45 g) en éluant par un mélange de dichlorométhane-méthanol (92/8 en volumes). On recueille d'abord une fraction de 275 cm³, puis des fractions de 20 cm³. On recueille les fractions 12 à 46. Celles-ci sont réunies, concentrées sous pression réduite (5 kPa) à une température voisine de 35°C. Le résidu obtenu est repris dans le dichlorométhane, filtré. Le filtrat est évaporé comme précédemment, puis le nouveau résidu obtenu est trituré dans 10 cm³ d'éther diisopropylique. Le mélange est agité pendant 1 heure à une température voisine de 20°C, puis abandonné pendant 16 heures. Le précipité solide est séparé par filtration, lavé par 2 fois 10 cm³ du même solvant, puis par 2 fois 10 cm³ de pentane. On obtient 0,47 g d'acide (3R,4R)-4-[3-(R,S)-hydroxy-3-(6-méthoxyquinolin-4-yl)propyl]-1-[3-(2,3-difluorophényl)prop-2-ynyl]pipéridine-3-carboxylique, sous forme d'un solide de couleur blanche, fondant à 92°C.

Spectre de R.M.N.¹H (300 MHz, (CD₃)₂SO d6, δ en ppm) : on observe un mélange de diastéréoisomères :
de 1,40 à 1,95 et de 2,30 à 3,00 (2 séries de mts : 12H en totalité) ; 3,64 et 3,65 (2s : 2H en totalité) ; 3,90 et 3,92 (2s : 3H en totalité) ; 5,25 (mt : 1H) ; 5,52 (mf : 1H) ; de 7,15 à 7,30 (mt : 1H) ; de 7,30 à 7,60 (mt : 5H) ; 7,93 (d, J = 10 Hz : 1H) ; 8,70 (d, J = 4,5 Hz : 1H) ; de 11,90 à 12,80 (mf très étalé : 1H).

### (3R,4R)-4-[3-(R,S)-Hydroxy-3-(6-méthoxyquinolin-4-yl)propyl]-1-[3-(2,3-difluorophényl)prop-2-ynyl]pipéridine-3-carboxylate de méthyle

A un mélange de 1,1 g de (3R,4R)-4-[3-(R,S)-hydroxy-3-(6-méthoxyquinolin-4-yl) propyl]-1-(prop-2-ynyl)pipéridine-3-carboxylate de méthyle dans 11 cm³ de triéthylamine, agité à une température voisine de 20°C sous atmosphère inerte, on ajoute 0,16 g de tétrakis triphénylphosphine palladium, 0,053 g d'iodure cuivreux et 0,47 cm³ de 1-bromo-2,3-difluorobenzène. Le mélange est chauffé à une température voisine de 80°C pendant 3 heures 30 minutes. Après refroidissement à environ 20°C, le mélange réactionnel est repris par 30 cm³ d'acétate d'éthyle et 30 cm³ d'eau, agité pendant 15 minutes. La phase organique est décantée tandis que la couche aqueuse est extraite par 3 fois 30 cm³ d'acétate d'éthyle. Les extraits organiques sont réunis, lavés par 3 fois 30 cm³ d'eau, séchés sur sulfate de sodium, filtrés, concentrés sous pression réduite (5 kPa) à une température voisine de 40°C. Le résidu obtenu est purifié par chromatographie sous une pression d'azote de 50 kPa, sur une colonne de gel de silice (granulométrie 20-45µ ; diamètre 3 cm ; 50 g) en éluant par de l'acétate d'éthyle pur. On recueille d'abord 1 fraction de 300 cm³, puis on effectue des fractions de 30 cm³. Les fractions 10 à 30 sont réunies, évaporées dans les conditions de ci-dessus. On obtient 0,94 g de (3R,4R)-4-[3-(R,S)-hydroxy-3-(6-méthoxy-quinolin-4-yl)propyl]-1-[3-(2,3-difluorophényl)prop-2-ynyl]pipéridine-3-carboxylate de méthyle, sous forme d'une meringue de couleur orangée.

Spectre infra rouge (CH₂Cl₂) : 3598 ; 2951 ; 1733 ; 1622 ; 1489 ; 1475 ; 1243 ; 1227 ; 1031 ; 856 et 831 cm⁻¹.

Le (3R,4R)-4-[3-(R,S)-hydroxy-3-(6-méthoxyquinolin-4-yl)propyl]-1-(prop-2-ynyl) pipéridine-3-carboxylate de méthyle peut être préparé comme décrit dans l'exemple 13.

### Exemple 25

### Acide (3R,4R)-4-[3-(R,S)-hydroxy-3-(6-méthoxyquinolin-4-yl)propyl]-1-[3-(2,3,5-trifluorophényl)prop-2-ynyl]pipéridine-3-carboxylique

Une solution de 1,7 g de (3R,4R)-4-[3-(R,S)-hydroxy-3-(6-méthoxyquinolin-4-yl) propyl]-1-[3-(2,3,5-trifluorophényl)prop-2-ynyl]pipéridine-3-carboxylate de méthyle dans 17 cm³ de dioxanne additionnés de 2,58 cm³ de soude aqueuse 5N est agitée pendant 15 heures à une température voisine de 60°C. Après refroidissement à une température voisine de 20°C, le mélange réactionnel est concentré sous pression réduite (5 kPa) à une température voisine de 40°C. Le résidu obtenu est purifié par chromatographie sous une pression de 50 kPa d'azote, sur une colonne de gel de silice (granulométrie 20-45µ ; diamètre 3 cm ; 50 g), en éluant par un mélange de dichlorométhane-méthanol (90/10 en volumes) et en recueillant d'abord une fraction de 200 cm³, puis des fractions de 23 cm³. Les fractions 3 à 21 sont réunies, puis concentrées comme précédemment. On obtient une meringue que l'on soumet à une deuxième purification par chromatographie sous une pression de 50 kPa d'azote, sur une colonne de gel de silice (granulométrie 20-45 µ ; diamètre 3 cm ; 70 g), en éluant par un mélange de dichlorométhane-méthanol (95/5 en volumes) et en recueillant d'abord une fraction de 250 cm³, puis une fraction de 100 cm³, puis des fractions de 20 cm³. Les fractions 1 à 17 sont réunies, puis concentrées comme précédemment. On obtient une meringue que reprend par du dichlorométhane, puis par 20 cm³ d'un mélange 50/50 de diisopropyloxyde et de pentane. Le produit cristallisé formé est filtré, lavé par 2 fois 10 cm³ d'un mélange diisopropyloxyde-pentane (50/50 en volumes), puis 2 fois 10 cm³ de pentane, séché à l'air. On obtient 0,524 g d'acide (3R,4R)-4-[3-(R,S)-hydroxy-3-(6-méthoxyquinolin-4-yl)propyl]-1-[3-(2,3,5-trifluorophényl)prop-2-ynyl]pipéridine-3-carboxylique, sous forme d'un solide de couleur crème fondant vers 97°C.

Spectre de R.M.N. ¹H (300 MHz, (CD₃)₂SO d6, δ en ppm) : on observe un mélange de diastéréoisomères dans les proportions 50/50.
de 1,40 à 2,00 (mt : 7H) ; de 2,35 à 3,00 (mt : 5H) ; 3,65 et 3,66 (2s : 2H en totalité) ; 3,90 et 3,92 (2s : 3H en totalité) ; 5,24 (mt : 1H) ; 5,54 (mf : 1H) ; de 7,25 à 7,45 (mt: 3H) ; de 7,50 à 7,70 (mt : 2H) ; 7,94 (d, J=9,5 Hz : 1 H) ; 8,71(d, J = 4,5 Hz : 1H).

### (3R,4R)-4-[3-(R,S)-hydroxy-3-(6-méthoxyquinolin-4-yl)propyl]-1-[3-(2,3,5-trifluorophényl)prop-2-ynyl] pipéridine-3-carboxylate de méthyle

Un mélange de 1,95 g de (3R,4R)-4-[3-(R,S)-hydroxy-3-(6-méthoxyquinolin-4-yl) propyl]-1-(prop-2-ynyl)pipéridine-3-carboxylate de méthyle dans 20 cm³ de triéthylamine est agité pendant 5 minutes sous atmosphère inerte à une température voisine de 20°C. On ajoute 0,284 g de tétrakis triphénylphosphine palladium, 0,094 g d'iodure cuivreux et 1,56 g de 1-bromo-2,3,5-trifluorobenzène. Le mélange est agité pendant 2 heures 30 minutes à une température voisine de 80°C. Après refroidissement à environ 20°C, le mélange réactionnel est additionné de 60 cm³ d'acétate d'éthyle et 60 cm³ d'eau. Après 30 minutes d'agitation, le mélange est décanté. Après séparation de la phase organique, la couche aqueuse est extraite par 3 fois 30 cm³ d'acétate d'éthyle. Les extraits organiques sont réunis, lavés par 3 fois 30 cm³ d'eau, séchés sur sulfate de sodium, filtrés, concentrés sous pression réduite (5 kPa) à une température voisine de 40°C. Le résidu obtenu est purifié par chromatographie sous une pression d'azote de 50 kPa, sur une colonne de gel de silice (granulométrie 20-45µ ; diamètre 4 cm ; 80 g), en éluant par de l'acétate d'éthyle pur et en recueillant d'abord une fraction de 100 cm³, puis des fractions de 20 cm³. Les fractions 23 à 27 sont réunies, concentrées comme ci-dessus. On obtient 1,3 g de (3R,4R)-4-[3-(R,S)-hydroxy-3-(6-méthoxyquinolin-4-yl)propyl]-1-[3-(2,3,5-trifluorophényl)prop-2-ynyl]pipéridine-3-carboxylate de méthyle, sous forme d'une meringue.

Spectre infrarouge dans C Cl₄ : 2950 ; 1740 ; 1624 ; 1496 ; 1231 ; 1133 ; 861 et 845 cm⁻¹

Le (3R,4R)-4-[3-(R,S)-hydroxy-3-(6-méthoxyquinolin-4-yl)propyl]-1-(prop-2-ynyl) pipéridine-3-carboxylate de méthyle peut être préparé comme décrit dans l'exemple 13.

### Exemple 26

### Acide (3R,4R)-4-[3-hydroxy-3-(6-méthoxyquinolin-4-yl)propyl]-1-[3-(2,3,5-trifluoro-phényl)prop-2-ynyl]pipéridine-3-carboxylique, diastéréoisomère A et Acide (3R,4R)-4-[3-hydroxy-3-(6-méthoxyquinolin-4-yl)propyl]-1-[3-(2,3,5-trifluoro-phényl)prop-2-ynyl]pipéridine-3-carboxylique, diastéréoisomère B

1,50 g d'acide (3R,4R)-4-[3-(R,S)-hydroxy-3-(6-méthoxyquinolin-4-yl)propyl]-1-[3-(2,3,5-trifluorophényl)prop-2-ynyl]pipéridine-3-carboxylique dissous dans 200 cm³ de dichloro-méthane est chromatographié sur une colonne de 30 cm et de 6 cm de diamètre conditionnée avec 700 g de silice Kromasil® (granulométrie 10 µ). L'élution est effectuée à l'aide d'un mélange dichlorométhane acétonitrile- -méthanol dans des proportions 90/5/5 en volumes. Le débit est de 130 cm³ par minute et la détection effectuée en ultra violet à 265 nm. Cette opération conduit après quatre injections préparatives à l'obtention des deux diastéréoisomères. Les fractions correspondant au premier sont concentrées à sec sous pression réduite (5 kPa) à une température voisine de 40°C, puis le résidu obtenu est séché à l'étuve sous pression réduite (13 Pa) à une température voisine de 40°C. On obtient 1,9 g d'acide (3R,4R)-4-[3-hydroxy-3-(6-méthoxyquinolin-4-yl)propyl]-1-[3-(2,3,5-trifluorophényl) prop-2-ynyl]pipéridine-3-carboxylique, diastéréoisomère A. (α_{D}²⁰= -50.9°+/-1,4, dans le méthanol à 0,5 %), sous forme d'une meringue.

Spectre de R.M.N. ¹H (300 MHz, (CD₃)₂SO d6, δ en ppm) :
de 1,40 à 2,00 (mt : 7H) ; de 2,35 à 3,00 (mt : 5H) ; 3,66 (s : 2H) ; 3,92 (s : 3H en totalité) ; 5,24 (mt : 1H) ; 5,54 (mf: 1H) ; de 7,25 à 7,45 (mt: 3H) ; de 7,50 à 7,70 (mt : 2H) ; 7,94 (d, J=9,5 Hz : 1H) ; 8,71(d, J = 4,5 Hz : 1H).

Les fractions correspondant au second diastéréoisomère sont traitées comme précédemment. On obtient 2,17 g d'acide (3R,4R)-4-[3-hydroxy-3-(6-méthoxyquinolin-4-yl)propyl]-1-[3-(2,3,5-trifluorophényl)prop-2-ynyl]pipéridine-3-carboxylique, diastéréoisomère B. (α_{D}²⁰=+67.8°+/-1,2, dans le méthanol à 0,5 %), sous forme d'une meringue.

Spectre de R.M.N. ¹H (300 MHz, (CD₃)₂SO d6, δ en ppm) :
de 1,40 à 2,00 (mt : 7H) ; de 2,35 à 3,00 (mt : 5H) ; 3,65 (s : 2H) ; 3,90 (s : 3H en totalité) ; 5,24 (mt : 1H) ; 5,54 (mf: 1H) ; de 7,25 à 7,45 (mt: 3H) ; de 7,50 à 7,70 (mt : 2H) ; 7,94 (d, J=9,5 Hz : 1H) ; 8,7 1 (d, J = 4,5 Hz : 1H).

L'acide (3R,4R)-4-[3-(R,S)-hydroxy-3-(6-méthoxyquinolin-4-yl)propyl]-1-[3-(2,3,5-trifluoro-phényl)prop-2-ynyl]pipéridine-3-carboxylique peut être préparé comme décrit à l'exemple 25.

La présente invention concerne également les compositions pharmaceutiques contenant au moins un dérivé de quinolyl propyl pipéridine selon l'invention, le cas échéant sous forme de sel, à l'état pur ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de quinolyl propyl pipéridine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 750 mg et 3 g de produit actif en 2 ou 3 prises par jour par voie orale ou entre 400 mg et 1,2 g par voie intraveineuse pour un adulte.

## Revendications

1. Un dérivé de quinolyl propyl pipéridine de formule générale : 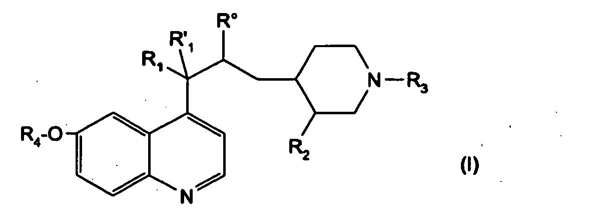 dans laquelle :
R₁ est un atome d'hydrogène ou d'halogène, ou un radical hydroxy,
R'₁ est un atome d'hydrogène, ou peut représenter halogène lorsque R₁ est également un atome d'halogène, et
R° est un atome d'hydrogène, ou bien
R₁ et R° forment ensemble une liaison et
R'₁ est un atome d'hydrogène, et
R₂ représente un radical carboxy, carboxyméthyle, carboxy-2 éthyle, hydroxyméthyle, alcoyloxycarbonyle, alcoyloxycarbonylméthyle ou alcoyloxycarbonyl-2-éthyle (dont les parties alcoyle contiennent de 1 à 6 C),
R₃ représente un radical propargyle substitué par un radical phényle pouvant lui même porter 1 à 4 s ubstituants [ choisis parmi halogène, hydroxy, alcoyle, alcoyloxy, trifluorométhyle, trifluorométhoxy, carboxy, alcoyloxycarbonyle, cyano ou amino], ou substitué par un radical cycloalcoyle contenant 3 à 7 chaînons ou substitué par un radical hétérocyclyle aromatique de 5 à 6 chaînons comprenant 1 à 4 hétéroatomes choisis parmi l'azote, l'oxygène et le soufre et éventuellement lui-même substitué [par halogène, hydroxy, alcoyle, alcoyloxy, trifluorométhyle, trifluorométhoxy, oxo, carboxy, alcoyloxycarbonyle, cyano ou amino],
et R₄ représente un radical alcoyle (contenant de 1 à 6 atomes de carbone), alcényl-CH₂- ou alcynyl-CH₂- dont les parties alcényle ou alcynyle contiennent 2 à 6 atomes de carbone,
étant entendu que les radicaux et portions alcoyle sont en en chaîne droite ou ramifiée et contiennent, sauf mention spéciale, 1 à 4 atomes de carbone,
sous ses formes diastéréoisomères ou leurs mélanges, ainsi que ses sels.

2. Un dérivé de quinolyl propyl pipéridine selon la revendication 1, **caractérisé en ce que**
R₁ est un atome d'hydrogène ou d'halogène, ou un radical hydroxy,
R'₁ est un atome d'hydrogène, et
R° est un atome d'hydrogène, ou bien
R₁ et R° forment ensemble une liaison et
R'₁ est un atome d'hydrogène,
R₂ représente un radical carboxy ou carboxyméthyle, et
R₃ représente un radical propargyle substitué par un radical phényle pouvant lui même porter 1 à 3 substituants halogène, ou propargyle substitué par un radical hétérocyclyle aromatique de 5 à 6 chaînons comprenant 1 à 4 hétéroatomes choisis parmi l'azote, l'oxygène et le soufre, ou bien
R₂ représente un radical hydroxyméthyle, alcoyloxycarbonyle ou alcoyloxycarbonyl-méthyle (dont les portions alcoyle contiennent 1 à 6 atomes de carbone) et
R₃ représente un radical propargyle substitué par un radical hétérocyclyle aromatique de 5 à 6 chaînons comprenant 1 à 4 hétéroatomes choisis parmi l'azote, l'oxygène et le soufre,et
R₄ représente un radical alcoyle (contenant 1 à 6 atomes de carbone),
les radicaux et portions alcoyle étant en en chaîne droite ou ramifiée, sous ses formes diastéréoisomères ou leurs mélanges, ainsi que ses sels.

3. Un dérivé de quinolyl propyl pipéridine selon l'une des revendications 1 ou 2, **caractérisé en ce qu'**il s'agit de l'acide (3R,4R)-4-[3-hydroxy-3-(6-méthoxyquinolin-4-yl)propyl]-1-[3-(2,3,5-trifluorophényl)prop-2-ynyl]pipéridine-3-carboxylique, sous ses formes diastéréoisomères ou leurs mélanges, ainsi que ses sels.

4. Un procédé de préparation de dérivé de quinolyl propyl pipéridine selon la revendication 1, **caractérisé en ce que** l'on condense la chaîne R₃ définie dans la revendication 1, sur le dérivé de quinolyl propyl pipéridine de formule générale : 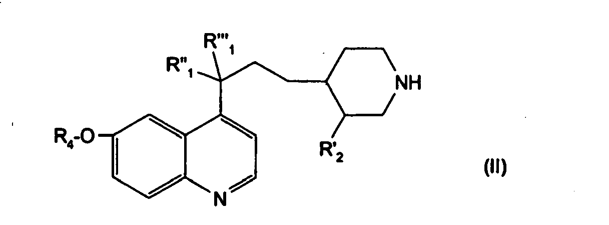 dans laquelle R₄ est défini commedans la revendication 1, R"₁ et R"'₁ représentent des atomes d'hydrogène ou forment ensemble un radical oxo et R'₂ représente un radical carboxy, carboxyméthyle ou carboxy-2 éthyle protégés, ou un radical alcoyloxycarbonyle, alcoyloxycarbonylméthyle ou alcoyloxycarbonyl-2-éthyle, pour obtenir un dérivé de quinolyl propyl pipéridine de formule générale : 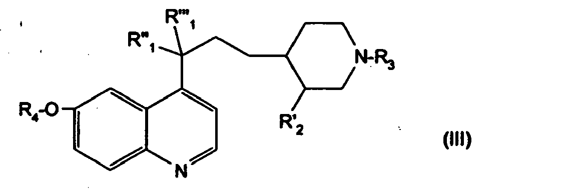 pour lequel R"₁, R"'₁, R'₂ et R₄ sont définis comme ci-dessus et R₃ est défini comme dans la revendication 1,
puis élimine le cas échéant le radical protecteur d'acide,
ou bien, le cas échéant réduit le radical oxo représenté par R"₁ et R"'₁ en un alcool pour lequel R₁ représente hydroxy puis éventuellement effectue l'halogénation si l'on veut obtenir un dérivé de quinolyl propyl pipéridine pour lequel R₁ est un atome d'halogène, et éventuellement effectue la déhydrohalogénation du dérivé halogéné correspondant, pour obtenir un dérivé de quinolyl propyl pipéridine pour lequel R₁ et R° forment ensemble une liaison, ou bien m et en oeuvre la dihalogénation du produit de formule générale (III) pour lequel R"₁ et R"'₁ forment ensemble un radical oxo pour obtenir un dérivé de quinolyl propyl pipéridine pour lequel R₁ et R'₁ sont des atomes d'halogène,
et/ou le cas échéant réduit l'acide protégé sous forme d'un radical R'₂ en position -3 de la pipéridine, en un radical hydroxyméthyle et éventuellement transforme en un radical carboxyméthyle ou carboxy-2 éthyle selon les méthodes habituelles,
puis éventuellement élimine le radical protecteur d'acide et/ou sépare, le cas échéant, les diastéréoisomères et éventuellement transforme le produit obtenu en un sel.

5. Un procédé selon la revendication 4, **caractérisé en ce que** la condensation de la chaîne R₃ sur la pipéridine s'effectue par action d'un dérivé de formule générale :
R₃-X
dans laquelle R₃ est défini comme précédemment et X représente un atome d'halogène, un radical méthylsulfonyle, un radical trifluorométhylsulfonyle ou p.toluènesulfonyle.

6. Un procédé selon l'une des revendications 4 ou 5, **caractérisé en ce que** la réaction s'effectue par condensation d'un halogénure de propargyle, puis substitution de la chaîne par un radical phényle, cycloalcoyle ou hétérocyclyle.

7. Composition pharmaceutique **caractérisée en ce qu'**elle contient au moins un dérivé selon la revendication 1, à l'état pur ou en association avec un ou plusieurs diluants ou adjuvants compatibles et pharmaceutiquement acceptables.

## Claims

1. Quinolylpropylpiperidine derivative of general formula: 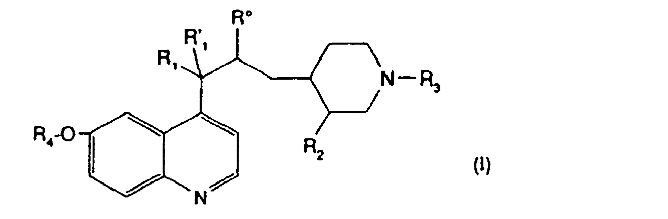 in which:
R₁ is a hydrogen or halogen atom or a hydroxyl radical,
R'₁ is a hydrogen atom or can represent a halogen atom when R₁ is also a halogen atom, and
R° is a hydrogen atom, or else
R₁ and R° together form a bond and
R'₁ is a hydrogen atom,
R₂ represents a carboxyl, carboxymethyl, 2-carboxyethyl, hydroxymethyl, alkyloxycarbonyl, alkyloxycarbonylmethyl or 2-(alkyloxycarbonyl)ethyl radical (the alkyl parts of which comprise from 1 to 6 C),
R₃ represents a propargyl radical substituted by a phenyl radical which can itself carry 1 to 4 substituents [chosen from halogen, hydroxyl, alkyl, alkyloxy, trifluoromethyl, trifluoromethoxy, carboxyl, alkyloxycarbonyl, cyano and amino], or substituted by a 3- to 7-membered cycloalkyl radical or substituted by a 5- to 6-membered aromatic heterocyclyl substituent comprising I to 4 heteroatoms chosen from nitrogen, oxygen and sulphur and being itself optionally substituted [by halogen, hydroxyl, alkyl, alkyloxy, trifluoromethyl, trifluoromethoxy, oxo, carboxyl, alkyloxycarbonyl, cyano or amino],
and R₄ represents an alkyl radical (comprising 1 to 6 carbon atoms), an alkenyl-CH₂- radical or an alkynyl-CH₂- radical, the alkenyl or alkynyl parts of which comprise 2 to 6 carbon atoms,
it being understood that the alkyl radical and portions are straight- or branched-chain radicals, and comprise, unless otherwise specified, 1 to 4 carbon atoms, in its diastereoisomeric forms or their mixtures, as well as its salts.

2. Quinolylpropylpiperidine derivative according to Claim 1, **characterized in that**
R₁ is a hydrogen or halogen atom or a hydroxyl radical,
R'₁ is a hydrogen atom, and
R° is a hydrogen atom, or else
R₁ and R° together form a bond and
R'₁ is a hydrogen atom,
R₂ represents a carboxyl or carboxymethyl radical, and
R₃ represents a propargyl radical substituted by a phenyl substituent which can itself carry 1 to 3 halogens, or a propargyl substituted by an 5- to 6-membered aromatic heterocyclyl substituent comprising 1 to 4 heteroatoms chosen from nitrogen, oxygen and sulphur, or else
when R² represents a hydroxymethyl, alkyloxycarbonyl or alkyloxycarbonylmethyl radical (the alkyl portions of which comprise 1 to 6 carbon atoms), then
R₃ represents a propargyl radical substituted by a 5- to 6-membered aromatic heterocyclyl radical comprising 1 to 4 heteroatoms chosen from nitrogen, oxygen or sulphur,
and R₄ represents an alkyl radical (comprising 1 to 6 carbon atoms),
the alkyl radicals and portions being straight- or branched-chain radicals and portions,
in its diastereoisomeric forms or their mixtures, as well as its salts.

3. Quinolylpropylpiperidine derivative according to either of Claims 1 and 2, **characterized in that** it is (3R,4R)-4-[3-hydroxy-3-(6-methoxyquinoline-4-yl)-propyl]-1-[3-(2,3,5-trifluorophényl)prop-2-ynyl]-piperidine-3-carboxylic acid, in its diastereoisomeric forms or their mixtures, as well as its salts.

4. Process for the preparation of quinolylpropylpiperidine derivative according to Claim 1, **characterized in that** the R₃ chain defined in Claim 1 is condensed onto the quinolylpropylpiperidine derivative of general formula: 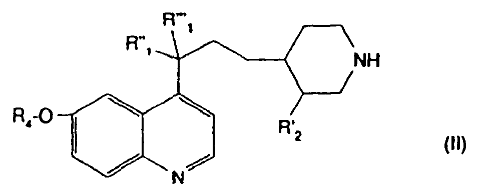 in which R₄ is defined as in Claim 1, R"₁ and R"'₁ represent hydrogen atoms or together form an oxo radical and R'₂ represents a protected carboxyl, carboxymethyl or 2-carboxyethyl radical or an alkyloxycarbonyl, alkyloxycarbonylmethyl or 2-(alkyloxycarbonyl)ethyl radical, in order to obtain a quinolylpropylpiperidine derivative of general formula: 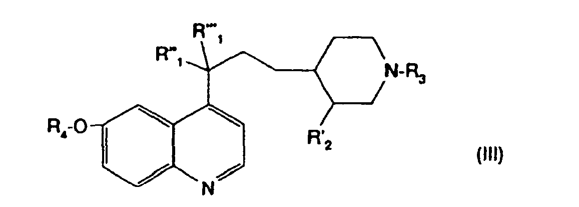 in which R"₁, R"'₁, R'₂ and R₄ are defined as above and R₃ is defined as in Claim 1,
then, if appropriate, the acid-protecting radical is removed,
or else, if appropriate, the oxo radical represented by R"₁ and R'''₁ is reduced to an alcohol in which R₁ represents hydroxyl, then, optionally, halogenation is carried out, if it is desired to obtain a quinolylpropylpiperidine derivative in which R₁ is a halogen atom, and, optionally, dehydrohalogenation of the corresponding halogenated derivative is carried out, in order to obtain a quinolylpropylpiperidine derivative in which R₁ and R° together form a bond, or else dihalogenation of the product of general formula (III) in which R"₁ and R"'₁ together form an oxo radical is carried out, in order to obtain a quinolylpropylpiperidine derivative in which R₁ and R'₁ are halogen atoms,
and/or, if appropriate, the acid, protected in the form of an R'₂ radical, in the 3-position of the piperidine is reduced to a hydroxymethyl radical and optionally converted to a carboxymethyl or 2-carboxyethyl radical according to the usual methods,
then, optionally, the acid-protecting radical is removed and/or, if appropriate, the diastereoisomers are separated and the product obtained is optionally converted to a salt.

5. Process according to Claim 4, **characterized in that** the condensation of the R₃ chain onto the piperidine is carried out by the action of a derivative of general formula:
R₃-X
in which R₃ is defined as above and X represents a halogen atom, a methylsulphonyl radical, a trifluoromethylsulphonyl radical or a p-toluenesulphonyl radical.

6. Process according to claim 4 or 5, **characterized in that** the reaction is carried out by condensation of a propargyl halide, then by substitution of the chain by a phenyl, cycloalkyl or heterocyclyl radical.

7. Pharmaceutical composition, **characterized in that** it comprises at least one derivative according to Claim 1 in the pure state or in combination with one or more compatible and pharmaceutically acceptable diluents or adjuvants.

## Patentansprüche

1. Chinolyl-propyl-piperidin-derivat der allgemeinen Formel: 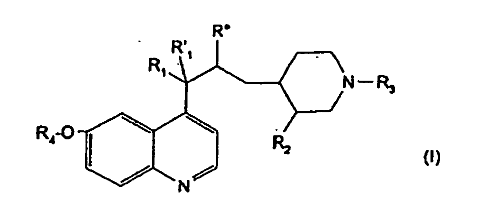 worin:
R₁ ein Wasserstoff- oder Halogenatom oder ein Hydroxyrest ist,
R'₁ ein Wasserstoffatom ist, oder ein Halogenatom bedeuten kann, wenn R₁ ebenfalls ein Halogenatom ist und
R⁰ ein Wasserstoffatom ist, oder
R₁ und R⁰ gemeinsam eine Bindung bilden und
R'₁ ein Wasserstoffatom ist,
R₂ einen Carboxy-, Carboxymethyl-, 2-Carboxyethyl-, Hydroxymethyl-, Alkyloxycarbonyl-, Alkyloxycarbonylmethyl- oder 2-Alkyloxycarbonylethylrest bedeutet (deren Alkylteile 1 bis 6 C enthalten),
R₃ einen Propargylrest bedeutet, der substituiert ist durch, einen Substituenten Phenyl, der seinerseits 1 bis 4 Substituenten aufweisen kann [ausgewählt unter Halogen, Hydroxy, Alkyl, Alkoxy, Trifluormethyl, Trifluormethoxy, Carboxy, Alkyloxycarbonyl, Cyano und Amino], oder der substituiert ist durch einen Cycloalkylrest, mit 3 bis 7 Gliedern oder einen aromatischen heterocyclischen Substituenten mit 5 bis 6 Gliedern trägt, der 1 bis 4 Heteroatome, ausgewählt unter Stickstoff, Sauerstoff und Schwefel, umfaßt, und seinerseits gegebenenfalls substituiert ist [durch Halogen, Hydroxy, Alkyl, Alkoxy, Trifluormethyl, Trifluormethoxy, Oxo, Carboxy, Alkyloxycarbonyl, Cyano oder Amino] und
R₄ einen Alkylrest (mit 1 bis 6 Kohlenstoffatomen), Alkenyl-CH₂- oder Alkinyl-CH₂-Rest, deren Alkenyl- oder Alkinylteile 2 bis 6 Kohlenstoffatome aufweisen, bedeutet,
mit der Maßgabe, dass die Alkylreste und -teile in gerader oder verzweigter Kette vorliegen, und, wenn nicht anders angegeben ist, 1 bis 4 Carbon atomen enthalten in seinen diastereomeren Formen oder deren Gemische, sowie seine Salze.

2. Chinolylpropylpiperidinderivat gemäß Anspruch 1, **dadurch gekennzeichnet, dass**
R₁ ein Wasserstoff- oder Halogenatom oder ein Hydroxyrest ist,
R'₁ ein Wasserstoffatom ist und
R⁰ ein Wasserstoffatom ist, oder
R₁ und R⁰ gemeinsam eine Bindung bilden und
R'₁ ein Wasserstoffatom ist,
R₂ einen Carboxy- oder Carboxymethylrest bedeutet, und
R₃ einen Propargylrest bedeutet, der substituiert ist durch einen Substituenten Phenyl, der seinerseits 1 bis 3 Halogenatome aufweisen kann, oder einen Propargylrest der substituiert ist durch einen aromatischen heterocyclischen Substituenten mit 5 bis 6 Gliedern, trägt, der 1 bis 4 Heteroatome, ausgewählt unter Stickstoff, Sauerstoff und Schwefel, umfaßt,
oder wenn R₂ einen Hydroxymethyl-, Alkoxycarbonyl- oder Alkoxycarbonylmethylrest (deren Alkylteile 1 bis 6 Kohlenstoffatome enthalten) bedeutet, dann
R₃ einen Alkylenrest (1 bis 6 Kohlenstoffatome) bedeutet, der substituiert ist durch einen aromatischen heterocyclischen Substituenten mit 5 bis 6 Gliedern trägt, der 1 bis 4 Heteroatome, ausgewählt unter Stickstoff, Sauerstoff oder Schwefel, umfasst,
und R₄ einen Alkylrest (mit 1 bis 6 Kohlenstoffatomen) bedeutet,
wobei die Alkylreste und -teile in gerader oder verzweigter Kette vorliegen, in seinen diastereomeren Formen oder deren Gemische, sowie seine Salze.

3. Chinolylpropylpiperidinderivat gemäß einem der Ansprüche 1 oder 2, **dadurch gekennzeichnet, dass** es sich um die (3R,4R)-4-[3-Hydroxy-3-(6-methoxychinolin-4-yl)-propyl]-1-[3-(2,3,5-trifluorophenyl)-prop-2-ynyl]-piperidin-3-carbonsäure in ihren diastereomeren Formen oder deren Gemische, sowie ihre Salze, handelt.
Formen oder ihre Gemische, sowie ihre Salze, handelt.

4. Verfahren zur Herstellung des Chinolylpropylpiperidinderivats gemäß Anspruch 1, **dadurch gekennzeichnet, dass** man die Kette R₃, wie in Anspruch 1 definiert, mit dem Chinolylpropylpiperidinderivat der allgemeinen Formel 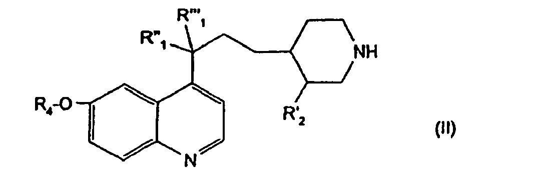 kondensiert, worin R₄ wie in Anspruch 1 definiert ist, R"₁ und R"'₁ Wasserstoffatome bedeuten oder gemeinsam einen Oxorest bilden und R'₂ einen Carboxy-, Carboxymethyl- oder 2-Carboxyethylrest in geschützter Form oder einen Alkoxycarbonyl-, Alkoxycarbonylmethyl- oder 2-Alkoxycarbonylethylrest bedeutet, um zu einem Chinolylpropylpiperidinderivat der allgemeinen Formel 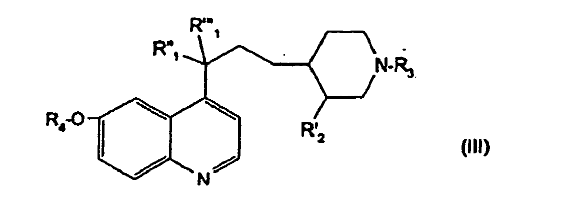 zu gelangen, worin R"₁, R"'₁, R'₂ und R₄ wie vorstehend definiert sind und R₃ die in Anspruch 1 angegebene Bedeutung besitzt,
anschließend gegebenenfalls die Schutzgruppe der Säure entfernt,
oder gegebenenfalls den durch R"₁ und R"'₁ wiedergegebenen Oxorest in einen Alkohol überführt, worin R₁ Hydroxy bedeutet, danach gegebenenfalls die Halogenierung durchführt, wenn man ein Chinolylpropylpiperidinderivat erhalten möchte, bei dem R₁ ein Halogenatom bedeutet und gegebenenfalls die Halogenwasserstoffabspaltung von dem entsprechenden Halogenderivat durchführt, um zu einem Chinolylpropylpiperidinderivat zu gelangen, bei dem R₁ und R⁰ gemeinsam eine Bindung bilden, oder die Dihalogenierung des Produkts der allgemeinen Formel (III), worin R"₁ und R"'₁ gemeinsam einen Oxorest bilden, durchführt, um zu einem Chinolylpropylpiperidinderivat zu gelagen, bei dem R₁ und R'₁ Halogenatome sind,
und/oder gegebenenfalls die in Form eines Restes R'₂ in 3-Stellung des Piperidins geschützte Säure in einen Hydroxymethylrest reduziert, und gegebenenfalls in einen Carboxymethyl- oder 2-Carboxyethylrest nach üblichen Methoden überführt,
anschließend gegebenenfalls die Schutzgruppe der Säure entfernt und/oder gegebenenfalls die Diastereomeren trennt und gegebenenfalls das erhaltene Produkt in ein Salz überführt.

5. Verfahren gemäß Anspruch 4, **dadurch gekennzeichnet, dass** die Kondensation der Kette R₃ mit dem Piperidin durch Einwirken eines Derivats der allgemeinen Formel:
R₃-X
erfolgt, worin R₃ wie in Anspruch 1 definiert ist, und X ein Halogenatom, einen Methylsulfonylrest, einen Trifluormethylsulfonyl- oder p-Toluolsulfonylrest bedeutet.

6. Verfahren gemäß Anspruch 4 oder 5, **dadurch gekennzeichnet, dass** die Reaktion durch Kondensation mit einen Propargyl Halide und dann durch Substituieren der Kette mit einem Phenyl, Cycloalkyl- oder Heterocyclylrest erfolgt.

7. Pharmazeutische Zusammensetzung, **dadurch gekennzeichnet, dass** sie zumindest ein Derivat gemäß Anspruch 1 in reiner Form oder in Assoziation mit ein oder mehreren kompatiblen und pharmazeutisch verträglichen Verdünnungsmitteln oder Adjuvantien enthält.
